# EUROPEAN PATENT APPLICATION

(11) **EP 3 357 926 A1**
(43) Date of publication of application: **08.08.2018**
(21) Application number: 17177947.3
(22) Date of filing: 10.12.2006
(51) Int. Cl.: C07D 499/00, A61P 31/04, A61K 9/00

(54) **TRANSDERMAL DELIVERY SYSTEMS OF BETA-LACTAM ANTIBIOTICS**

(62) Divisional of application: 13161970.2
(71) Applicant: Yu, Chongxi, Kensington, MD 20895 (US)
(72) Inventor: Yu, Chongxi, Kensington, MD 20895 (US)
(74) Representative: Finnegan Europe LLP

(57) **Abstract**

The novel positively charged pro-drugs of beta-lactam antibiotics in the general 'Structure 4' were designed. The positively charged amino group of the pro-drug not only makes the drugs soluble in water, but also bonds to the negative charge on the phosphate head group of membranes. This bonding will disturb the membrane a little bit and may make some room for the lipophilic portion of the prodrug. The prodrugs can be used medicinally in treating beta-lactam antibiotics-treatable conditions in humans or animals. The prodrugs can be administered transdermally for any kind of medical treatments. Controlled transdermal administration systems of the prodrug enables beta-lactam antibiotics to reach constantly optimal therapeutic blood levels to increase effectiveness and reduce the side effects of beta-lactam antibiotics.

## Description

### Technical Field

The present invention relates to the preparations of positively charged and watersoluble pro-drugs of beta-lactam antibiotics and related compounds and their medicinal use in treating any beta-lactam antibiotics and related compounds-treatable conditions in humans or animals. More specifically, the present invention is to enable quick bacterial cell wall and human skin penetration of beta-lactam antibiotics and related compounds.

### Background Art

A beta-lactam is a cyclic amide with four atoms in its ring and the chemical name for this ring system is azetidinone. Since French medical student Ernest Duchesne initially discovered penicillin in 1896 and Alexander Fleming rediscovered it in 1929 along with the subsequent purification of penicillin from it in the late 1930s and early 1940s by Florey, Chain, Abraham, and Heatley, over a hundred thousand of beta-lactam antibiotics have been prepared by partial or total chemical synthesis (L.A. Mitscher, et al., Antibiotic and Antimicrobial Drugs, in D.F. Smith, Ed., Handbook of Stereoisomers: Therapeutic Drugs, Boca Raton, FL, CRC Press, 1989; R.B. Morin and M. Gorman Eds., Chemistry and Biology of Beta-lactam Antibiotics, Volumes 1-3, New York, Academic Press, 1982; and A.L.Demain and N.A. Solomon, Eds., Antibiotics Containing the Beta-lactam Structure, Vols, 1 and 2, Handbook of experimental Phgarmacology, vol. 67, New York, Springer, 1983).

When penicillin became widely available during the second world war, it was a medical miracle, rapidly vanquishing the biggest wartime killer-infected wounds. Penicillin killed many types of disease-causing bacteria. But just four years after drug companies began mass-producing penicillin in 1943, microbes began appearing that could resist it. Antibiotic resistance spreads fast. Between 1979 and 1987, for example, only 0.02 % of pneumococcus strains infecting a large number of patients surveyed by the national Centers for Disease Control and Prevention were penicillin-resistant. Only 7 years late , 6.6 percent of pneumococcus strains are resistant, according to a report in the June 15, 1994, Journal of the American Medical Association by Robert F. Breiman, M.D., and colleagues at CDC (Ricki Lewis, U.S. Food and Drug Administration Home Page). Antimicrobial resistance is driving up health care costs, increasing the severity of disease, and increasing the death rates from certain infections. According to CDC statistics, nearly 2 million patients in the United States get an infection in the hospital each year and about 90,000 of those patients die each year as a result of their infection, up from 13,300 patient deaths in 1992. More than 70 percent of the bacteria that cause hospital-acquired infections are resistant to at least one of the antibiotics most commonly used to treat them. The increased prevalence of antibiotic resistance is an outcome of evolution, but overuse of antibiotics accelerates antibiotic resistance. To develop new antibiotics is a very urgent and challenging task.

Intravenous infusion, intramuscular injection, subcutaneous, buccal, oral, and rectal routes are the methods for administration of a wide variety of antimicrobial agents for the treatment of systemic bacterial diseases. Oral administration has disadvantage of poor absorption of the antibiotics from GI tract. The intravenous, subcutaneous and intramuscular routes are not only painful, but also must be performed by trained individuals and there is the risk of needle injury, infection, and other trauma. One alternative method of administering drugs is topical delivery. Topical drug delivery has several advantages. This method helps avoid inactivation of a drug caused by first pass metabolism in the liver and gastro-intestinal tract. It can provide local delivery of appropriate concentrations of a drug to the intended site of action without systemic exposure. Fishman (Fishman; Robert, U.S. Pat. No. 7,052,715) indicated that an additional problem associated with oral medications, is that the concentration levels which must be achieved in the bloodstream must be significant in order to effectively treat distal areas of pain, inflammation, or infection. These levels are often much higher than would be necessary if it were possible to accurately target the particular site of pain or injury. Watts, et al. (U.S. Pat. Nos. 6,191,143 and 5,929,086) have described topical administration of some antimicrobial agents that have special structure characteristics (we noticed that all these chemicals have one positive charge and a lipophilic portion). For most of antibiotics, topical administration cannot deliver an effective therapeutic level. Susan Milosovich, et al. designed and prepared testosteronyl-4-dimethylaminobutyrate.HCl (TSBH), which has a lipophilic portion and a tertiary amine group that exists in the protonated form at physiological pH. They found that the prodrug (TSBH) diffuses through human skin ~60 times faster than does the drug (TS) itself [Susan Milosovich, et al., J. Pharm. Sci., 82, 227(1993).

### Disclosure of Invention

### Technical Problem

The continued efficacy is threatened by microbial resistance caused by evolutionary pressures and overuse. Antibiotic resistance spreads fast. More people are contracting infections. People on chemotherapy and transplant recipients taking drugs to suppress their immune function are at greater risk of infection. The general aging of patients who live longer, get sicker, and die slower contributes to the problem. Developing new antibiotics is a very urgent and challenging task.

Insufficient contact of the antibiotics with pathogenic bacteria at the site of infection is a major cause of meningitis, encephslitis, myelitis, abscess, mastitis, and prostatitis treatment failure. The reason of the treatment failure is that antibiotics cannot penetrate the blood-brain barrier, blood-milk barrier, and other biological barriers efficiently,

Most antibiotics are rapidly metabolized and inactivated by various pathways. When antibiotics are taken orally, the first pass metabolism, which refers to the chemical breakdown of compounds in the liver and gastro-intestinal tract, can destroy and inactivate a large portion of them. In the case of injection, administration of antibiotics is painful and in many cases requires frequent and costly office visits to treat chronic conditions. The blood and liver can also destroy and inactivate most antibiotics before they reach the intended site of action.

### Technical Solution

Topical drug delivery for the treatment of local infections helps to avoid inactivation of a drug caused by first pass metabolism in the liver and gastro-intestinal tract and can provide local delivery of appropriate concentrations of a drug to the intended site of action without systemic exposure.

This invention relates to the designing and the preparation of novel positively charged pro-drugs of beta-lactam antibiotics and their medicinal use. The principles for designing these prodrugs of beta-lactam antibiotics are: 1. The prodrug must have a lipophilic portion and a primary, secondary, or tertiary amine group that exists in the protonated form (hydrophilic portion) at physiological pH. 2. Every prodrug of beta-lactam antibiotics should have only one or two (preferably one) primary, secondary, tertiary amine, or monoprotected quanidino groups that exists in the protonated form (hydrophilic portion) at physiological pH. 3. The primary, secondary, tertiary amine group, a quanidino, or monoprotected quanidino groups can be on any part of the compound. The design in which one end of the compound is the positive charge and other end is the lipophilic portion is preferable. 4. Carboxyl groups, amino groups, hydroxyl groups, guanidino groups and other hydrophilic groups can be protected with an alkyl, aryl, or heteroaryl ester or amide group to make the beta-lactam antibiotics more lipophilic.

One of beta-lactam antibiotics, Penethamate Hydriodide (Jensen et al., Ugeskrift for Laeger 112, 1043, 1075, 1950; Span. Pat. 206,653-5; Brit. Pat. 759,603), has the exact same structure characteristics of the novel prodrugs and show high penetration rate of milk-blood barrier. Surprisingly, nobody has tried to administer it transdermally .

The novel pro-drugs of beta-lactam antibiotics in this invention have the general formula (1) 'Structure 1'. 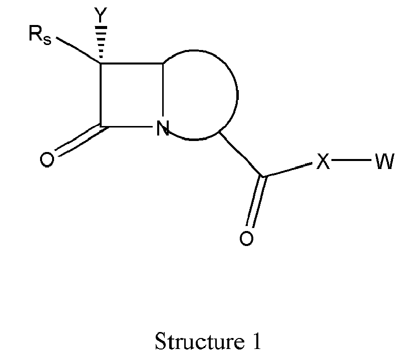 wherein, X represents O, S, or NH; Y represents H, OH, OR₅, NHCHO, CH₂CH₂ NHC(=NR₇)NHR₇, OCOCH₃, OCH₃, OC₂H₅, CH₃SO₃, NO₂, CN, CF₃, OCF₃, F, Br, I, or Cl; 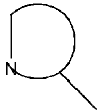 represents 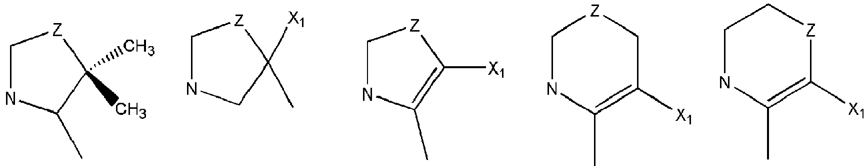 wherein, Z represents CH₂, S, SO, SO₂, NH, CHCH₃, CHCH₂CH₃, or O; -X₁ represents: -H, -OH, -OCH₃, -OC₂H₅, OC₃H₇, -CONH₂, -CH₂OCONH₂, -CH₂OCOCH₃, -OCOCH₃, -CH₂OCOCH₃, -CH₂OCH₃, -CH₃, -C₂H₅, -C₃H_{7,} -C₄H₉, C₅H₁₁, -C₆H₁₃, -Cl, -F, -Br, -I, - HC=CHCH₃, -HC=CH₂, -CH₂OCH₃, -S(CH₂)ₙ-NHR₇, 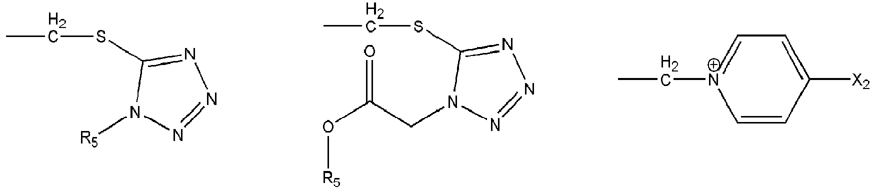 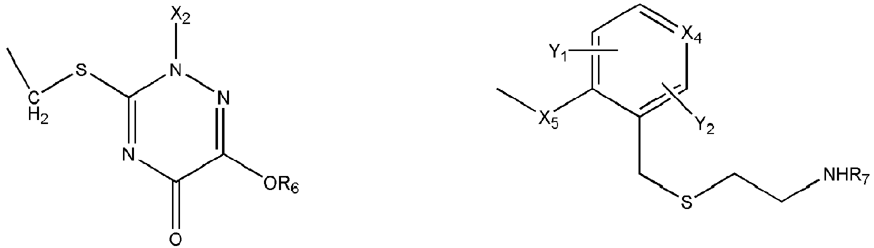 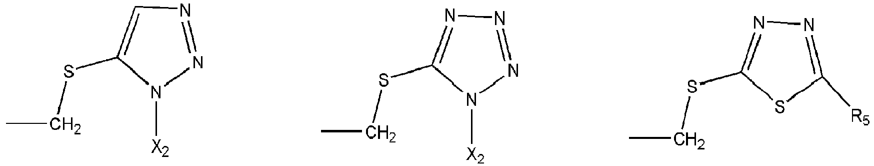 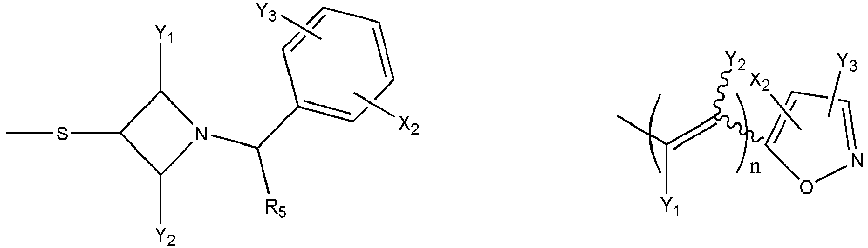 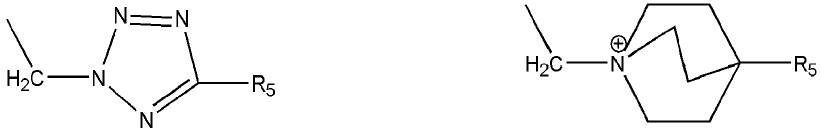 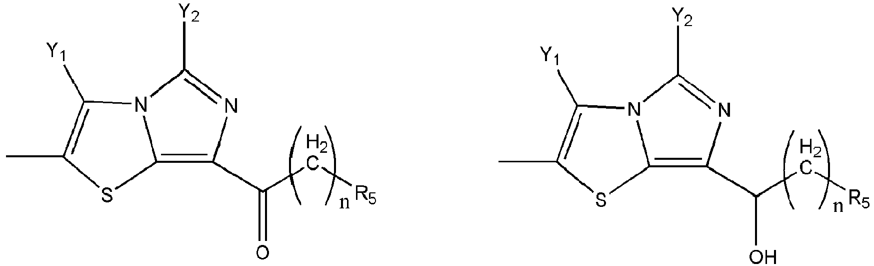 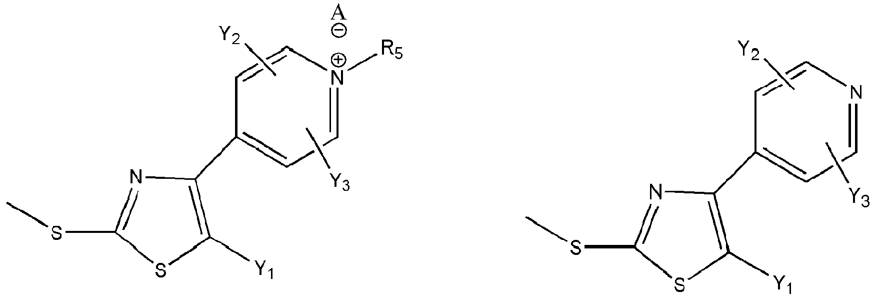 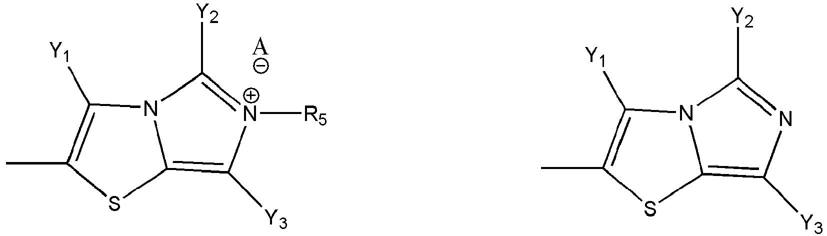 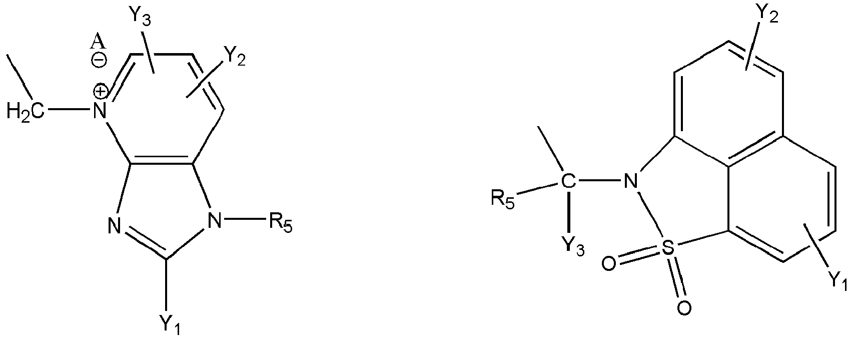 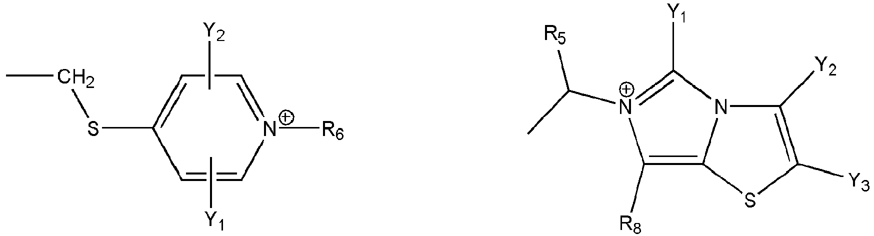 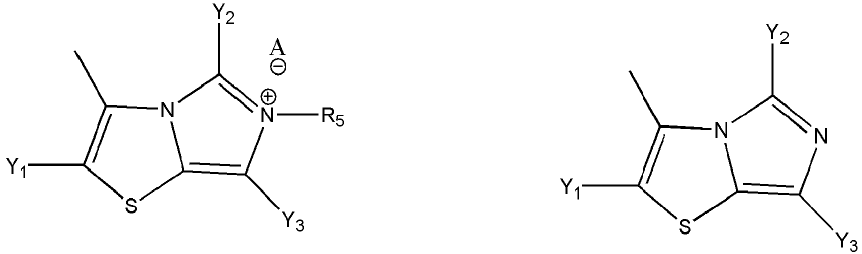 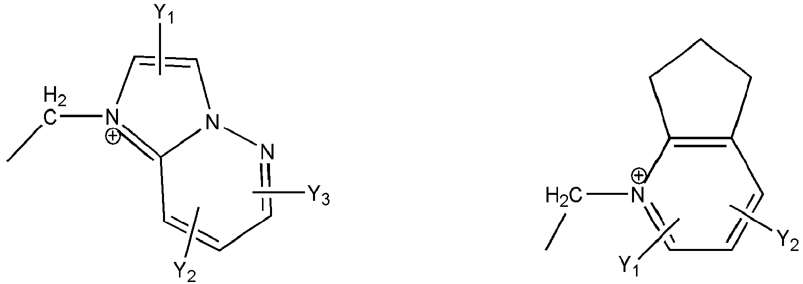 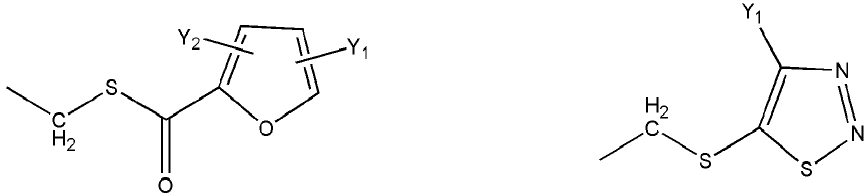 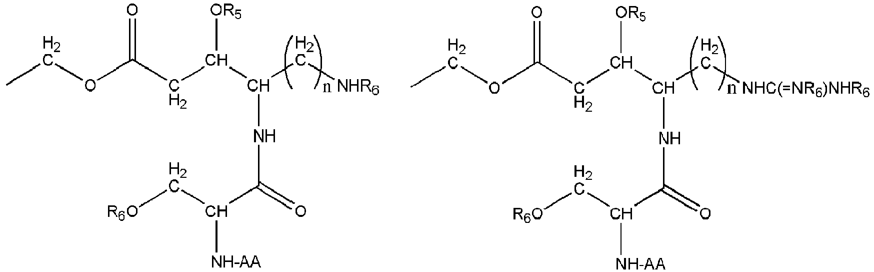 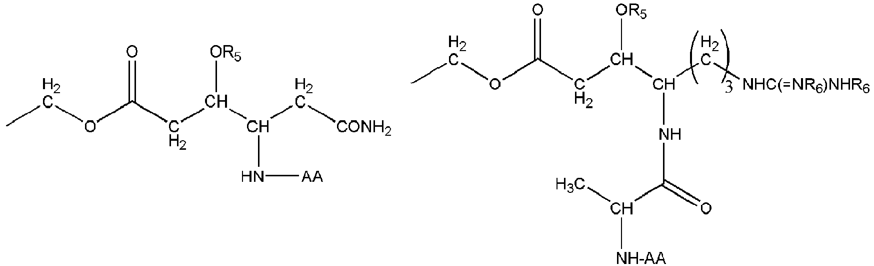 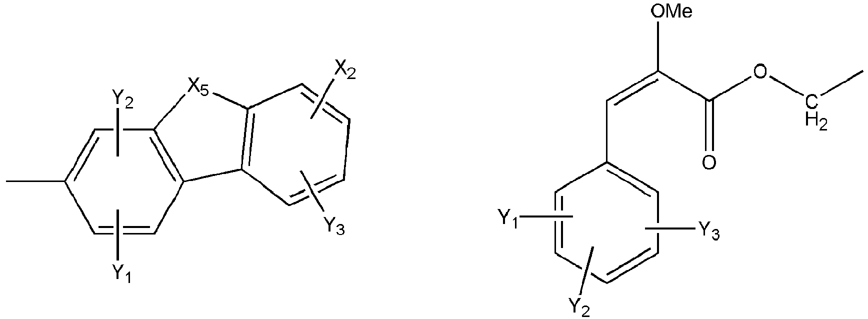 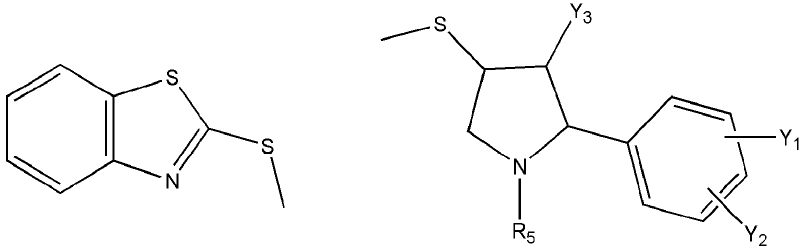 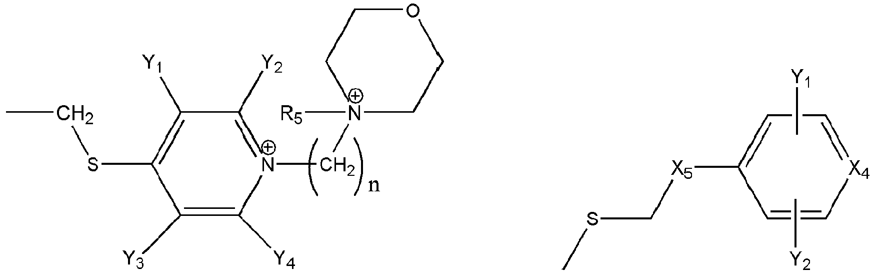 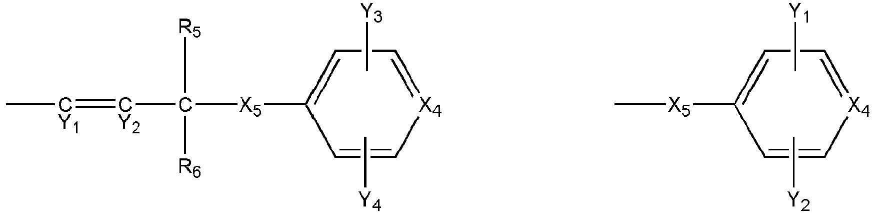 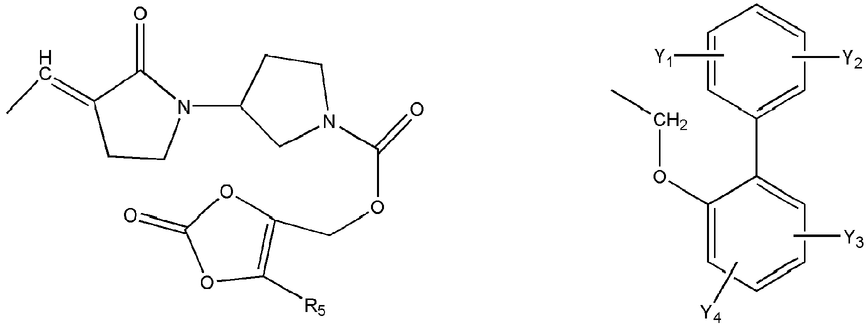 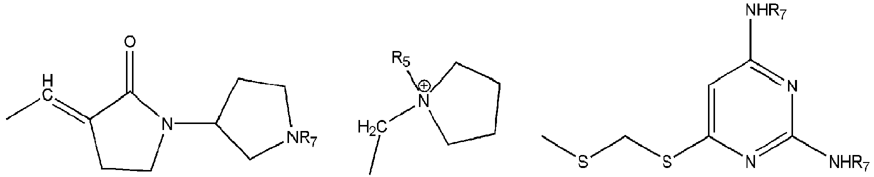 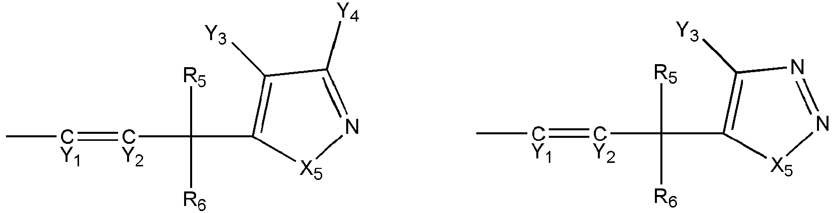 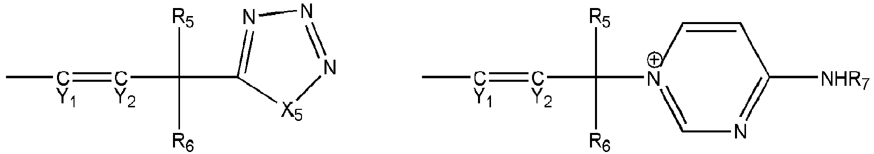 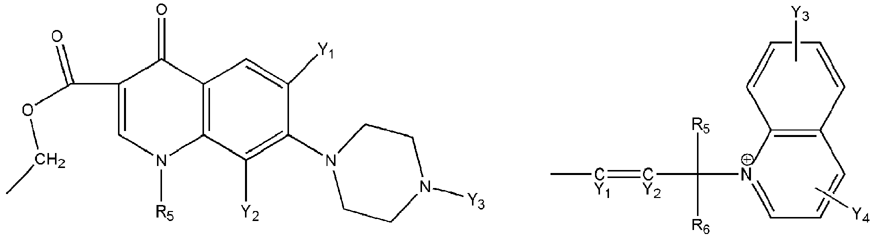 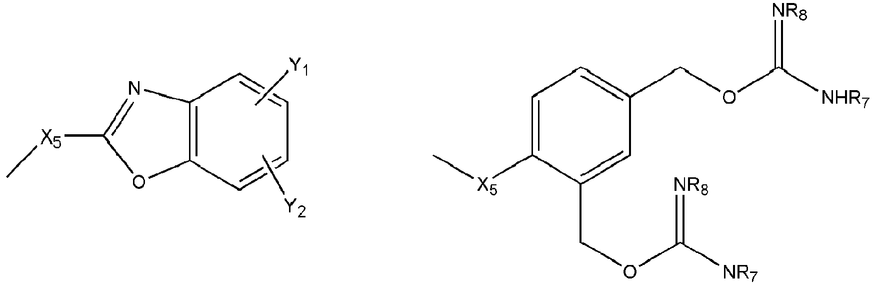 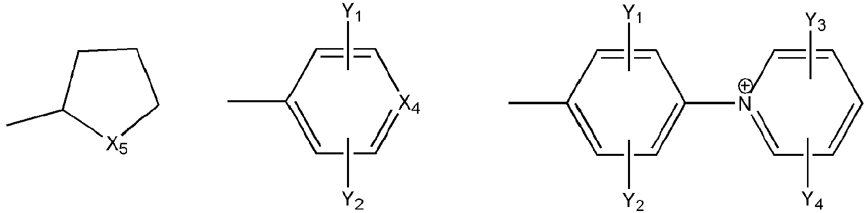 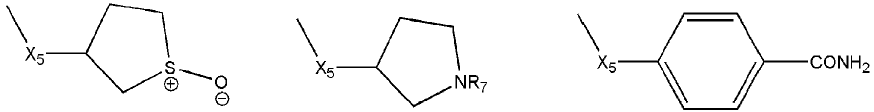 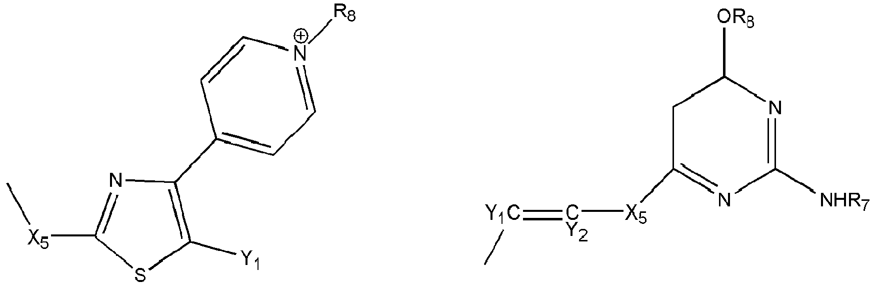 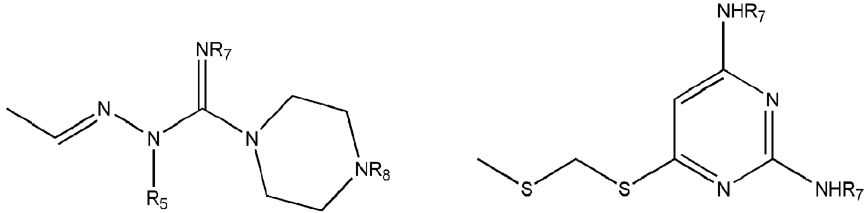 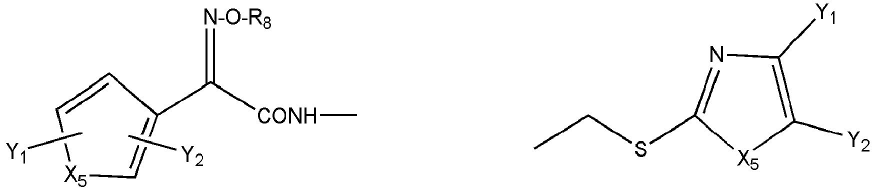 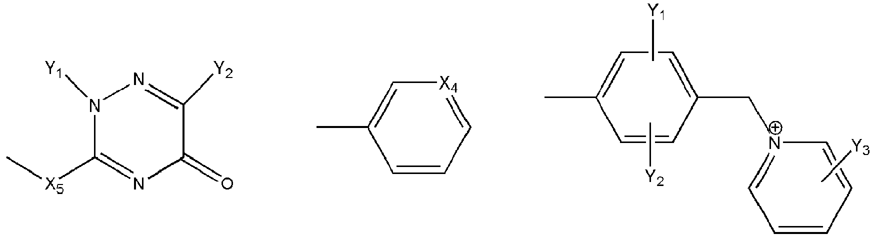 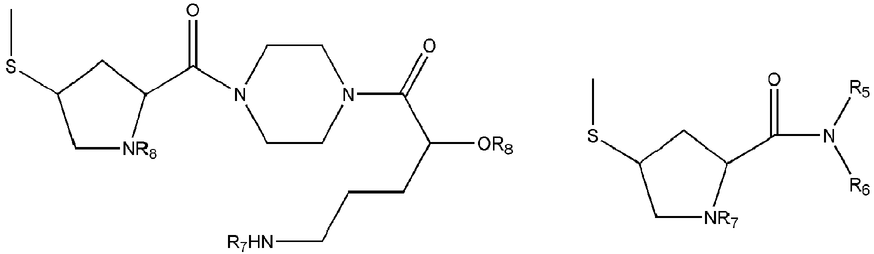 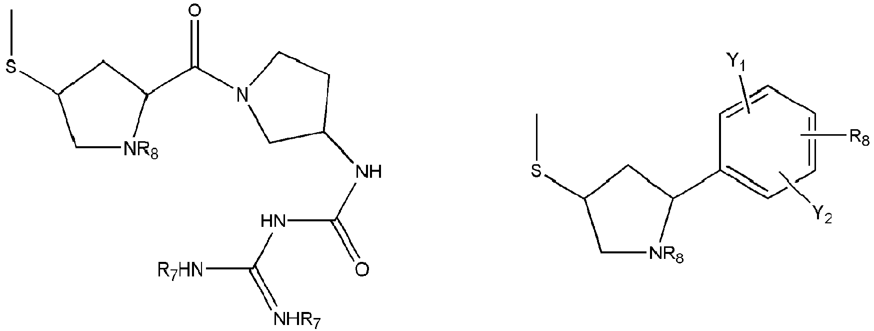 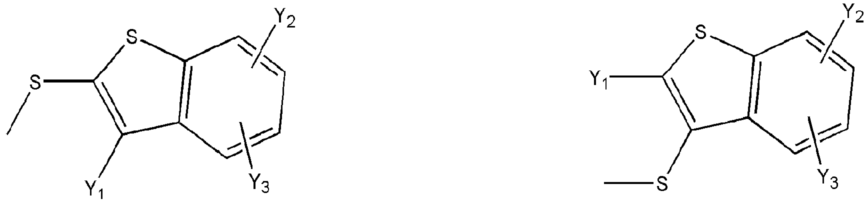 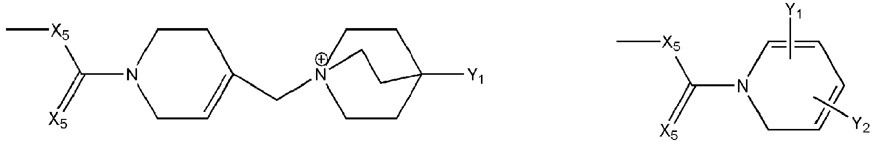 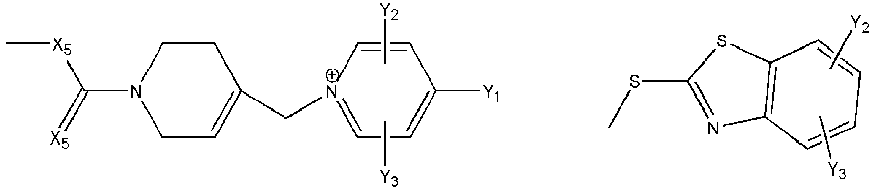 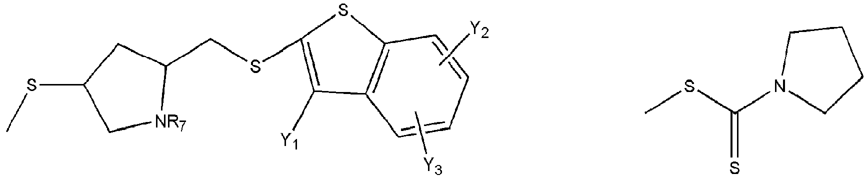 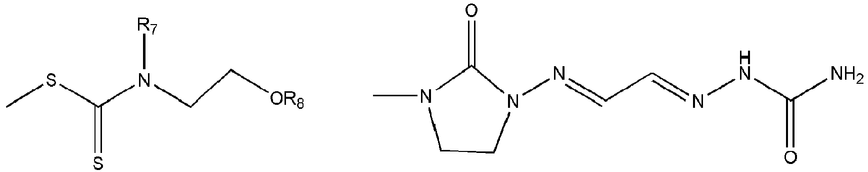 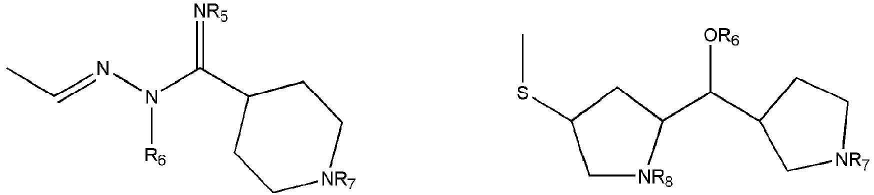 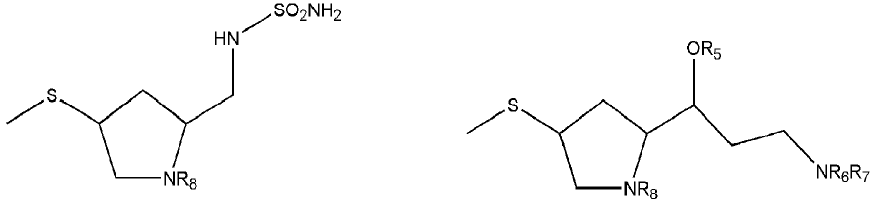 wherein , Z represents O, S, SO, SO₂, NH, CH₂, CHCH₃, or CHCH₂CH₃; R₅ represents H, CONH₂, CH₂CH₂OR₆, CH₂CH₂N(CH₃)₂, CH₂CH₂N(CH₂CH₃)₂, Cl, F, Br, I, one of any alkyl, alkyloxyl, alkenyl or alkynyl residues having 1 to 12 carbon atoms, aryl or heteroaryl residues , CO-W, or W; R₆ represents H, F, Cl, Br, I, Na⁺, K⁺, COR₅, one of any alkyl, alkyloxyl, alkenyl or alkynyl residues having 1 to 12 carbon atoms, aryl or heteroaryl residues , CO-W, or W; R₇ represents H, CH₃, C₂H₅, CF₃, COCH₃, C₃H₇, C₄ H₉, C₅H₁₁, CONH₂, COR₆, COR₅, CO-W, or W; R₈ represents H, CH₃, C₂H₅, CF₃, CH₂ CH₂F, CH₂CH₂Cl, CH₂CH₂Br, CH₂CH₂I, CH₂CHF₂, CH₂CF₃, CH₂F, CH₂Cl, CH₂Br, CH₂I, CH₂NR₆R₇, CH(NHR₇)CH₂CONH₂, C₃H₇, C₄H₉, C₅H₁₁, CONH₂, CH₂ CONH₂, CH₂OCONH₂, C(CH₃)₂COOR₆, CH(CH₃)COOR₆, CH₂COOR₆, COR₆, COR₅, CO-W, or W; X represents CH₂, S, NH, or O; X₂ represents H, CH₃, CH₂(CH₂)ₙOR₈, Cl, F, Br, I, NO₂, CN, CF₃, OCF₃, NH₂, CH₃, C₂H₅, CONH₂, CH₂OCONH₂, CH₂COOR₅, CH₂(CH₂)ₙ N(CH₃)₂, CH₂(CH₂)ₙSO₃R₅, C₁₋₈ alkyl, C₁₋₈ alkylthiol, C₁₋₈ alkylamine, or C₁₋₈ alkyloxy; X represents H, CH₃, CH₂(CH₂)ₙOR₈, Cl, F, Br, I, NO₂, CN, CF₃, OCF₃, NH₂, CH₂, C₂ H₅ CONH₂, CH₂OCONH₂, CH₂COOR₅, CH₂(CH₂)ₙN(CH₃)₂, CH₂(CH₂)ₙSO₃R₅, C₁₋₈ alkyl, C₁₋₈ alkylthiol, C₁₋₈ alkylamine, or C₁₋₈ alkyloxy; X₄ represents N, CH , or CY₁; X₅ represents CH₂, S, O or NH; Y represents H, OH, OW, OCOW, OCOCH₃, CH₃, C₂ H₅, C₃H₇, C₄H₉, CH₂COOR₈, OCH₃, OC₂H₅, CH₃SO₃, NO₂, CN, CF₃, OCF₃, CH₂(CH₂)ₙ NR₅R₆, CH₂(CH₂)ₙOR₆, CH(CONH₂)NHR₆, CH₂CONH₂, F, Br, I, or Cl; Y₂ represents H, OH, OW, OCOW, CH₃, C₂H₅, C₃H₇, C₄H₉, CH₂COOR₈, OCOCH₃, OCH₃, OC₂H₅, CH SO₃, NO₂, CN, CF₃, OCF₃, CH₂(CH₂)ₙNR₅R₆, CH₂(CH₂)ₙOR₆, CH(CONH₂)NHR₆, CH₂CONH₂, F, Br, I, or Cl; Y₃ represents H, OH, OW, OCOW, OCOCH₃, CH₃, C₂H₅, C₃H₇, C₄H₉, CH₂COOR₈, OCH₃, OC₂H₅, CH₃SO₃, NO₂, CN, CF₃, OCF₃, CH₂(CH₂)ₙNR₅ R₆, CH₂(CH₂)ₙOR₆, CH(CONH₂)NHR₆, CH₂CONH₂, F, Br, I, or Cl; Y₄ represents H, OH, OW, OCOW, OCOCH₃, CH₃, C₂H₅, C₃H₇, C₄H₉, CH₂COOR₈, OCH₃, OC₂H₅, CH₃ SO₃, NO₂, CN, CF₃, OCF₃, CH₂(CH₂)ₙNR₅R₆, CH₂(CH₂)ₙOR₆, CH(CONH₂)NHR₆, CH₂ CONH₂, F, Br, I, or Cl; AA represents amino acids; n=0, 1, 2, 3, 4, 5, 6,...; Rₛ-represents: 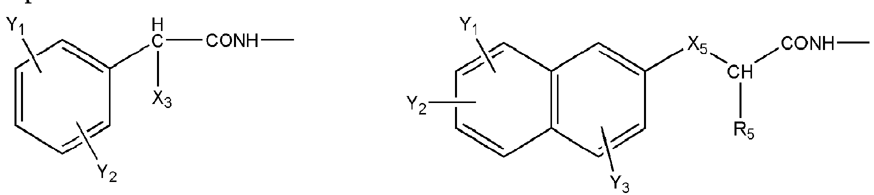 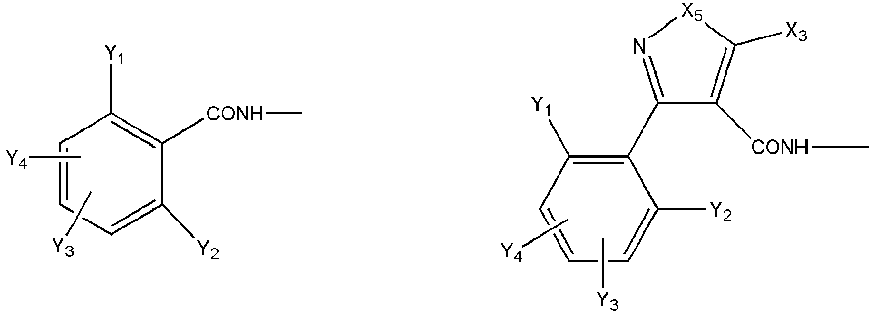 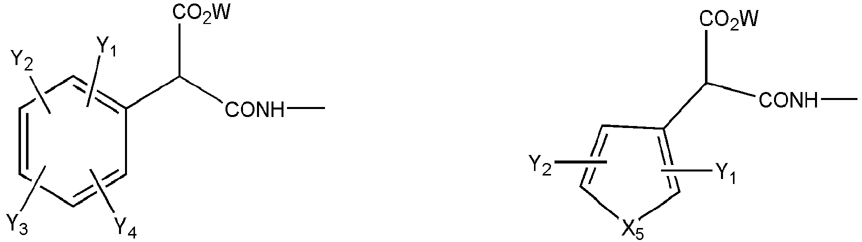 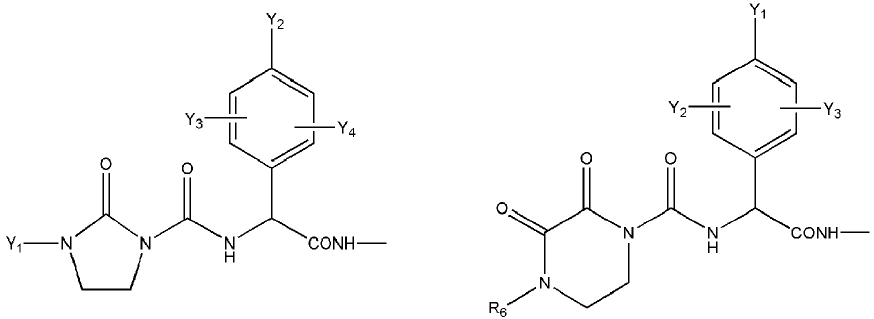 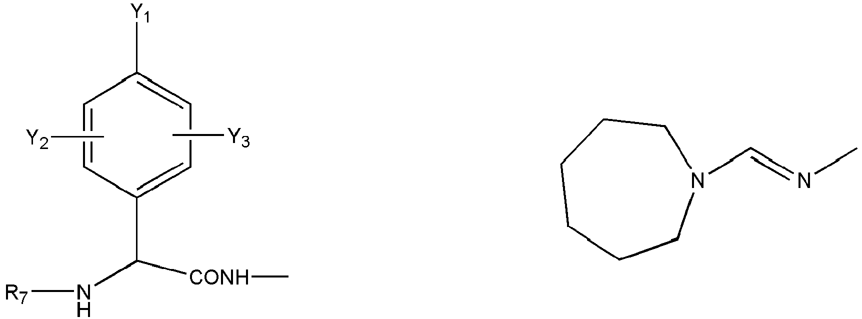 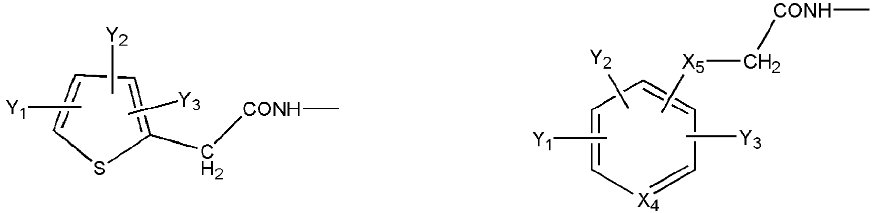 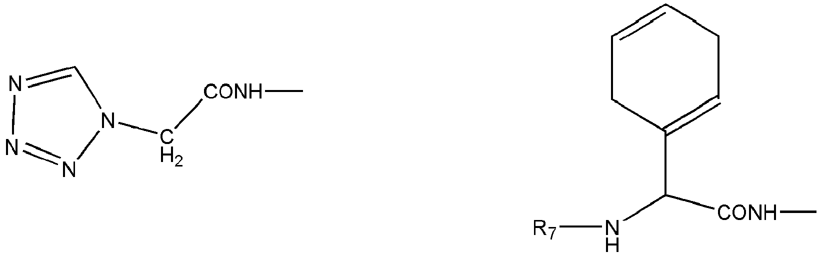 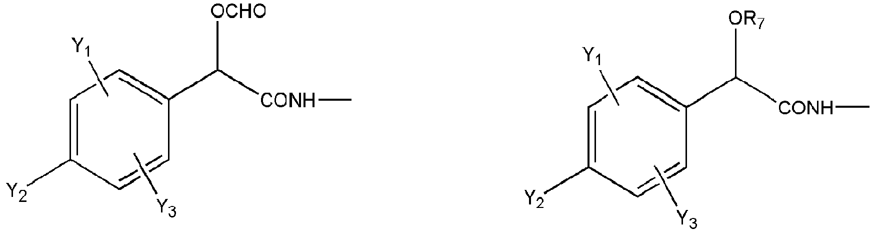 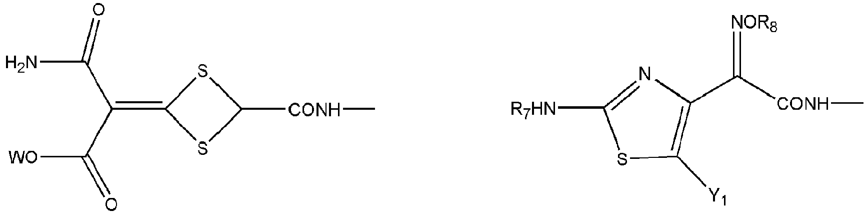 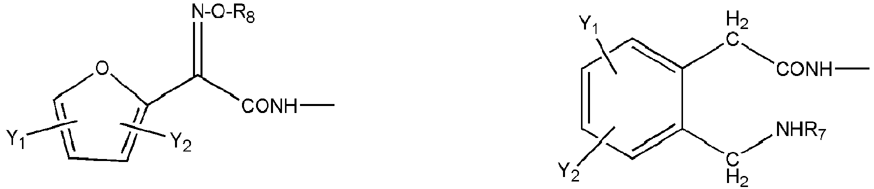 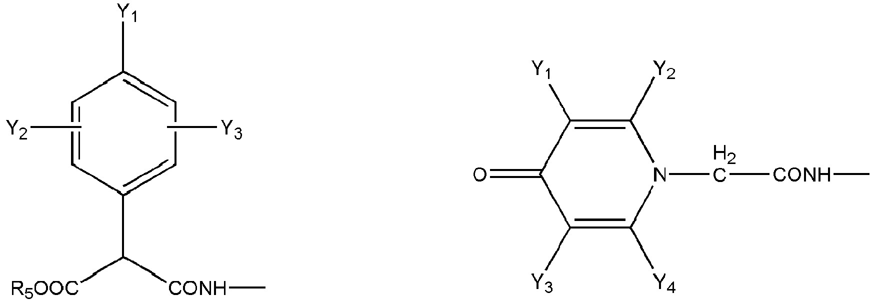 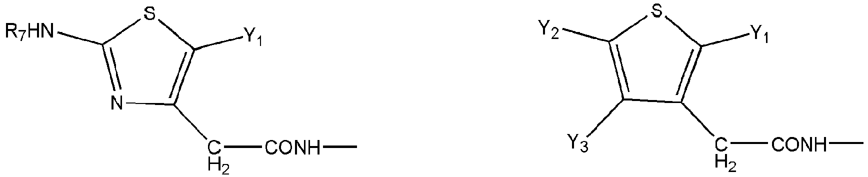 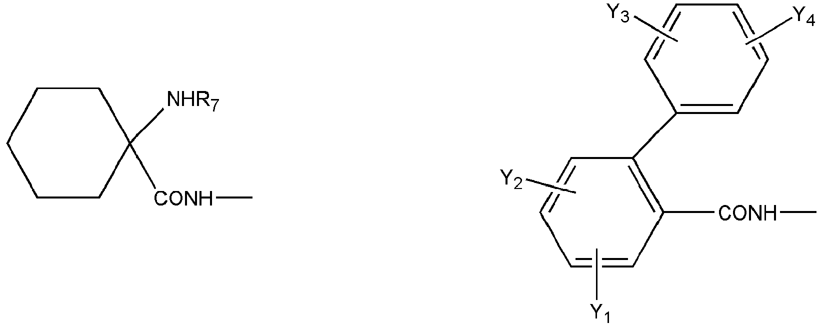 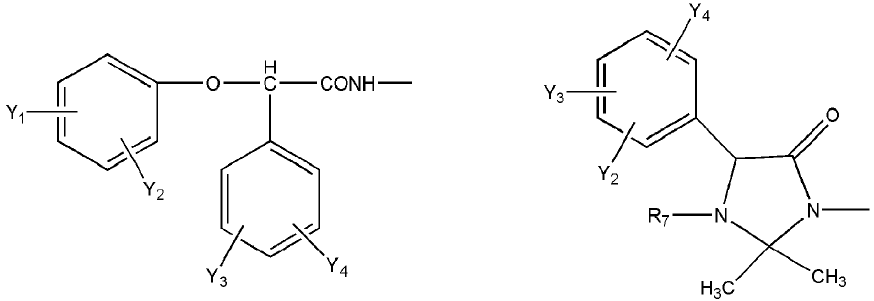 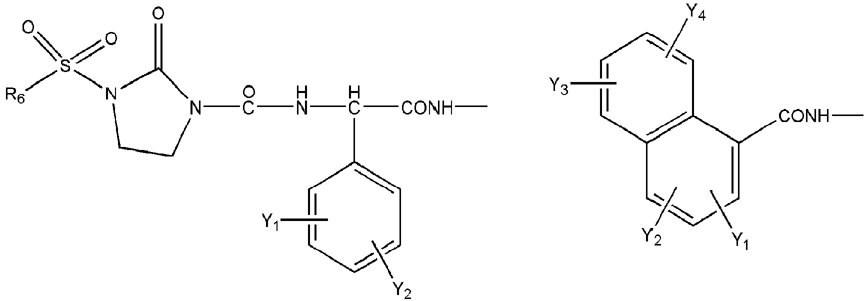 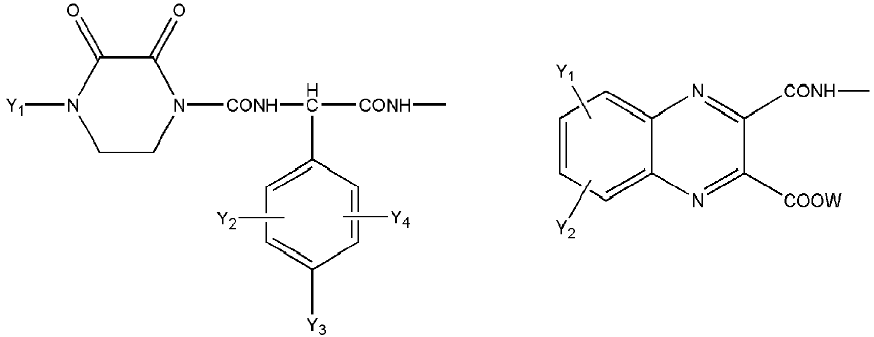 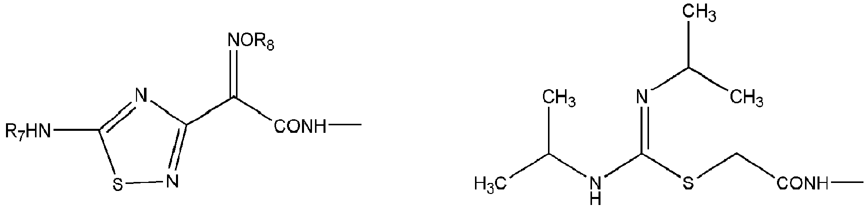 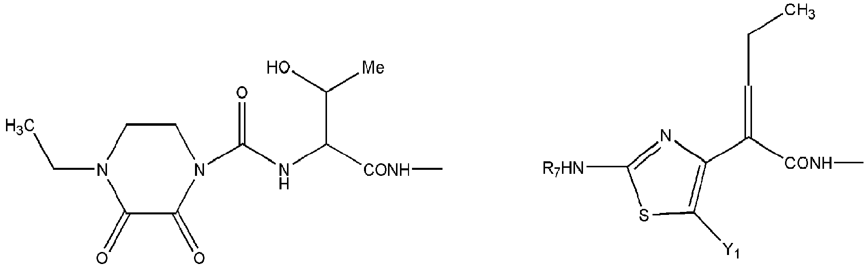 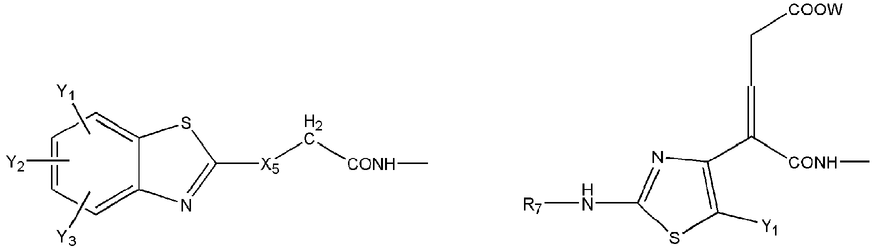 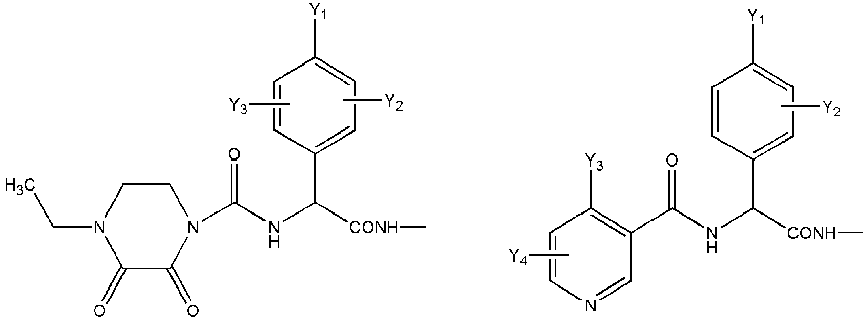 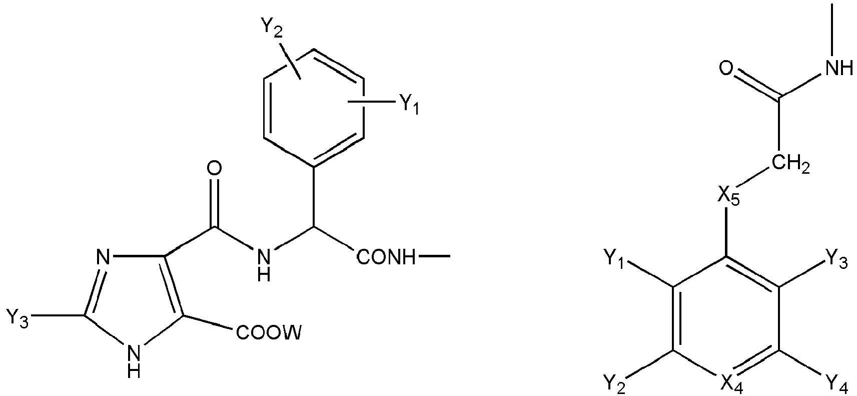 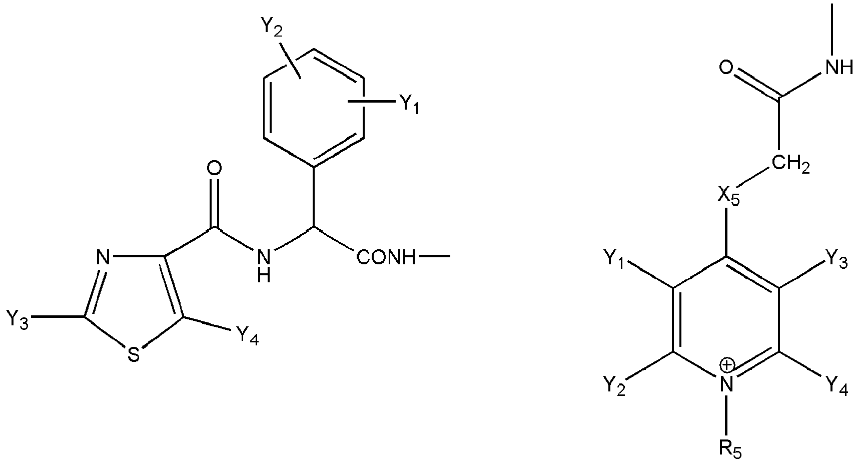 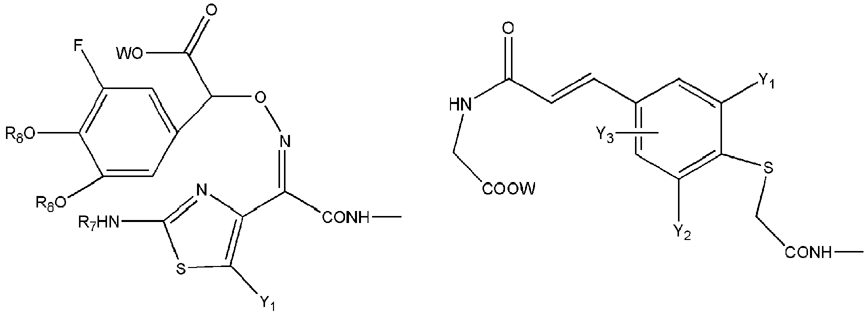 R₆OOCCH(NHR₇)(CH₂)ₙCONH-, R₆OOCCH(NHR₇)(CH₂)ₙSCONH- , CF₃SCH₂ CONH-, CF₃CH₂CONH-, CHF₂SCH₂CONH- , CH₂FSCH₂CONH- , NH₂COCHFSCH₂ CONH-, R₇NHCH(COOW)CH₂SCH₂CONH-, CNCH₂SCH₂CONH-, CH₃(CH₂)ₙ CONH-, R₇N=C(NHR₇)CH₂CH₂S-, R₇N=C(NHR₇)NHCO-, CH₃C(Cl)=CHCH₂SCH₂ CONH-, (CH₃)₂C(OR₆)-, CNCH₂CONH-, CNCH₂CH₂S-, R₇N=C(NHR₇)CH₂CH₂S-, CH₂=CHCH₂SCH₂CONH-, CH₃CH(OH)-, CH₃CH(OR₈)-, CH₃CH(Y₁)-, (CH₃)₂CH-, CH₃CH₂-, or CH₃(CH₂)ₙCH=CH(CH₂)ₘCONH-. Wherein, n or m= 0, 1, 2, 3, 4, 5, 6, ...; X₃ represents H, N₃, SO₃W, F, Cl, Br, I, OH, OCH₃, OC₂H₅, CH₃, C₂H₅, COOW, OW, or W; X₄ represents N, CH , or CY₁; X₅ represents CH₂, S, O or NH; R₅ represents H, -CONH₂, CH₂CH₂N(CH₃)₂, CH₂CH₂N(CH₂CH₃)₂, CH₂CH₂OR₆, Cl, F, Br, I, one of any alkyl, alkyloxyl, alkenyl or alkynyl residues having 1 to 12 carbon atoms, aryl or heteroaryl residue, CO-W, or W; R₆ represents H, F, Cl, Br, I, 2-oxo-1-imidazolidinyl, one of any alkyl, alkyloxyl, alkenyl or alkynyl residues having 1 to 12 carbon atoms, aryl (phenyl, 5-indanyl, 2,3-dihydro-1H-inden-5-yl, et al.) or heteroaryl (4-hydroxy-1,5-naphthyridin-3-yl, et al.) residues; R₇ represents H, F, Cl, Br, I, CH₃NHCOCH₂CH(NHR₈)CO, R₅N=C(NHR₆)NHCO-, COCH₃, COR₆, PO(OR₅ )OR₆, one of any alkyl, alkyloxyl, alkenyl or alkynyl residues having 1 to 12 carbon atoms, aryl or heteroaryl residues , CO-W , or W; R₈ represents H, F, Cl, Br, I, CH₃, C ₂H₅, CF₃, CH₂CH₂F, CH₂CH₂Cl, CH₂CH₂Br, CH₂CH₂I, CH₂CHF₂, CH₂CF₃, CH₂F, CH₂ Cl, CH₂Br, CH₂I, C₃H₇, C₄H₉, C₅H₁₁, COR₆, CONH₂, CH₂OCONH₂, PO(OR₅)OR₆, C(CH₃)₂COOR₆, CH(CH₃)COOR₆, CH₂COOR₆, CO-W, or W; Y represents H, OH, OW, OCOW, OCOCH₃, CH₃, C₂H₅, C₃H₇, C₄H₉, SO₃R₆, CH₂COOR₈, OCH₃, OC₂H₅, CH₃SO₃, NO₂, CN, CF₃, OCF₃, CH=CHCONHCH₂COOW, CH₂(CH₂)ₙNR₅R₆, CH₂(CH ₂)ₙOR₆, CH(CONH₂)NHR₆, COOR₅, F, Br, I, or Cl; Y₂ represents H, OH, OW, OCOW, CH₃, C₂H₅, C₃H₇, C₄H₉, CH₂COOR₈, OCOCH₃, OCH₃, OC₂H₅, CH₃SO₃, NO₂, CN, CF₃, OCF₃, CH=CHCONHCH₂COOW, CH₂(CH)₂ₙNR₅R₆, CH₂(CH₂)ₙOR₆, CH(CONH₂ )NHR₆, SO₃R₆, COOR₅, F, Br, I, or Cl; Y represents H, OH, OW, OCOW, OCOCH₃, CH₃, C₂H₅, C₃H₇, C₄H₉, CH₂COOR₈, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR₆, CH(CONH₂)NHR₆, SO₃ R₆, COOR₅, F, Br, I, or Cl; Y₄ represents H, OH, OW, OCOW, OCOCH₃, CH₃, C₂H₅, C ₃H₇, C₄H₉, CH₂COOR₈, OCH₃, OC₂H₅, CH₃SO₃, NO₂, NR₈COR₅, SO₂NR₅R₈, COR₅,SR ₅, CN, CF₃, OCF₃, CH=CHCONHCH₂COOW, CH₂(CH₂)ₙNR₅R₆, CH₂(CH₂)ₙOR₆, CH(CONH₂)NHR₆,SO₃R₆, COOR₅, F, Br, I, or Cl; -W represents -H, one of any alkyl, 2 6 3 6 5 alkyloxy, alkenyl or alkynyl residues having 1 to 12 carbon atoms, aryl or heteroaryl residues, or one of the following residues:

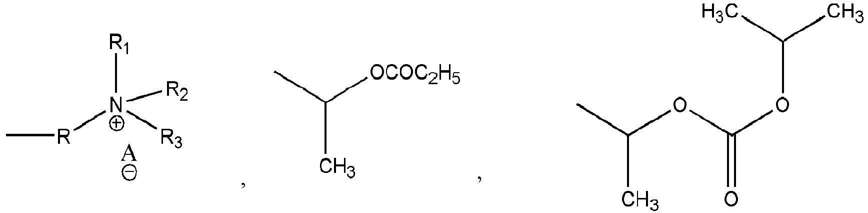

Wherein, R represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, or aryl and heteroaryl residues; R represents a branched or straight chain, -(CH₂)ₙ- , aryl residues or heteroaryl residues; n = 0, 1, 2, 3, 4, 5, 6, 7, 8, ...; Rₛ and Y taken together are R₆OCH₂C(R₅)=; A⁻ represents Cl⁻, Br⁻, F⁻, I ⁻, AcO⁻, citrate, or any negative ions. All double bond can be cis or trans. All R, R₁, R₂, R₃, R₄, R₅, R₆, R₇, R₈, -(CH₂)ₙ- or -(CH₂)ₘ- groups are branched or straight chains and may include C, H, O, S, or N atoms and may have single, double, and triple bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of a membrane and very hydrophobic drugs will stay in the hydrophobic layer as part of the membrane due to their similarities and cannot efficiently enter the cytosol on the inside.

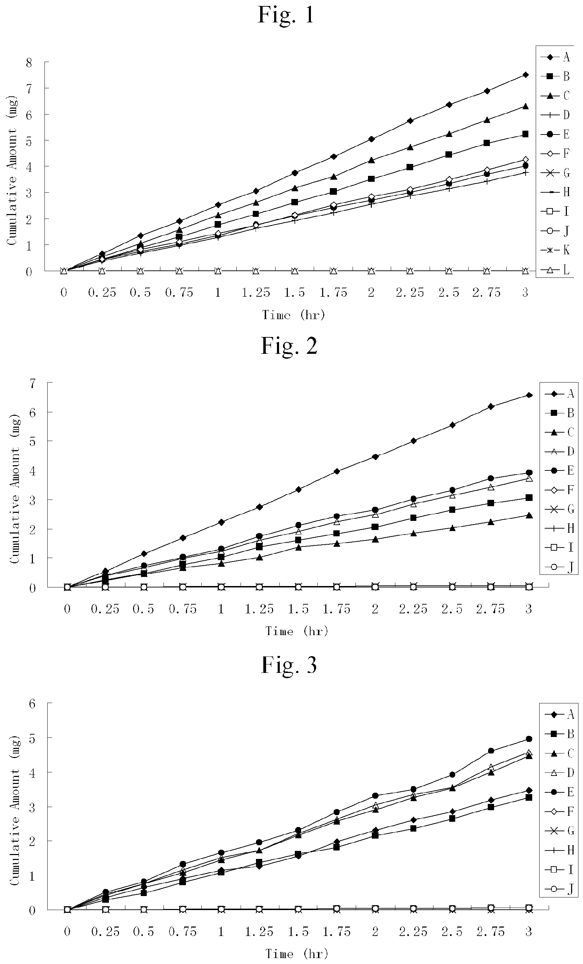

The goal of this invention is to make beta-lactam antibiotics administrable transdermally (topical application) by increasing their penetration rate through the skin barrier. These novel pro-drugs of beta-lactam antibiotics have two structural features in common: they have a lipophilic portion (which can be formed by using lipophilic alcohols to protect the carboxyl groups and lipophilic acids to protect amino, hydroxyl, or guanidino groups or other hydrophilic groups of beta-lactam antibiotics) and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water and the lipophilic portion will help the prodrugs enter the lipophilic membrane and skin barrier. When these new pro-drugs are administered transdermally in a dosage form such as solution, spray, lotion, ointment, emulsion or gel, they will dissolve in moisture of the skin surface immediately. The positive charge on the amino groups of these pro-drugs will bond to the negative charge on the phosphate head group of membrane. Thus, the local concentration of the outside of the membrane will be very high and will facilitate the passage of these prodrugs from a region of high concentration to a region of low concentration. When these pro-drugs enter the membrane, the hydrophilic part will push the pro-drug into the cytosol, a semi-liquid concentrated aqueous solution or suspension. The penetration rates of some prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of a 10% solution or suspention of some of the prodrugs and beta-lactam antibiotics in 0.2mL of pH 7.4 phosphate buffer (0.2M) are shown in Figure 1, Figure 2, Figure 3, and Figure 4. Apparent flux values of 2.52 mg, 1.75 mg, 2.12 mg, 1.27 mg, 1.35 mg, 1.43 mg, 2.22 mg, 1.02 mg, 0.82 mg, 1.24 mg, 1.32 mg, 1.15 mg, 1.07 mg, 1.45 mg, 1.52 mg, 1.65 mg, 0.93 mg, 1.02 mg, 0.53 mg, 0.81 mg, 0.85 mg, 0.001 mg, 0.001 mg, 0.001 mg, 0.001 mg, 0.001 mg, 0.001 mg, 0.015 mg, 0.001 mg, mg, 0.001 mg, 0.001 mg, 0.001 mg, 0.001 mg, 0.001 mg, 0.001 mg, 0.016 mg, 0.018 mg, 0.010 mg, 0.015 mg, 0.001 mg, 0.010 mg, and 0.25 mg/cm²/h were calculated for 6-phenoxyacetacetamidopenicillanic acid 2-diethylaminoethyl ester hydrochloride, allylmercaptomethylpenicillinic acid 2-diethylaminoethyl ester hydrochloride, 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride, 6-[3-(o-chlorophenyl)-5-methyl-4-isoxazolecarboxamido]penicillinic acid 2-diethylaminoethyl ester hydrochloride, 6-[3-(2,6-dichlorophenyl)-5-methyl-4-isoxazolecarboxamido]penicillinic acid 2-diethylaminoethyl ester hydrochloride, 6-[D(-)-α-acetamidophenylacetamidopenicillinic acid 2-diethylaminoethyl ester hydrochloride, D-α-[(imidazolidin-2-on-1-yl)carbonylamino]benzylpenicillin 2-diethylaminoethyl ester hydrochloride, 6R-[2-[3-(methylsulfonyl)-2-oxo-1-imidazolidinecarboxamido]-2-phenylacetamido]pe nicillinic acid 2-diethylaminoethyl ester hydrochloride, 6-D(-)-α-(4-ethyl-2,3-dioxo-1-piperazinylcarbonylamino)-α-phenylacetamidopenicilli nic acid 2-diethylaminoethyl ester hydrochloride, 7-(2-thienylacetamido)cephalosporanic acid 2-diethylaminoethyl ester hydrochloride, 7-[(hydroxyphenylacetyl)aminol-3-[[(1-methyl-1H-tetrazol-5-yl)thio]methyl]-8-oxo-5-thia-1-azabicyclo[4.2.0]oct-2-ene-2-carboxylic acid 2-diethylaminoethyl ester hydrochloride, 3-[[(aminocarbonyl)oxy]methyl]-7-[[2-furanyl(methoxyimino)acetyl] amino]-8-oxo-5-thia-1-azabicyclo[4.2.0]oct-2-ene-2-carboxylic acid 2-diethylaminoethyl ester hydrochloride, 3-[[(aminocarbonyl)oxy]methyl] - 7-methoxy-8-oxo-7-[(2-thienylacetyl)amino]-5-thia-1-azabicyclo[4.2.0]oct-2-ene-2-car boxylic acid 2-diethylaminoethyl ester hydrochloride, 7-[[[2-(acetylaminomethyl)phenyl]acetyl]amino]-3-[[[1-(ethoxylcarbonylmethyl)-1H-t etrazol-5-yl]thio]methyl]-8-oxo-5-thia-1-azabicyclo[4.2.0]oct-2-ene-2-carboxylic acid 2-diethylaminoethyl ester hydrochloride, 7-[(acetylaminophenylacetyl)amino] - 3-chloro-8-oxo-5-thia-1-azabicyclo[4.2.0]oct-2-ene-2-carboxylic acid 2-diethylaminoethyl ester hydrochloride, 3-[(acetyloxy)methyl] - 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7- [[[[(4-ethyl-2,3-dioxo-1-piperazinyl)carbonyl]amino](4-acetoxyphenyl)acetyl]amino ]-3-[[(1-methyl-1H-tetrazol-5-yl)thio]methyl]-8-oxo-5-thia-1-azabicyclo[4.2.0]oct-2-e ne-2-carboxylic acid 2-diethylaminoethyl ester hydrochloride, 7-[2-(2-acetylamino-4-thiazolyl)-2-((Z)-methoxyimino)acetamido]-3-(methoxymethyl) -8-oxo-5-thia-1-azabicyclo[4.2.0]oct-2-ene-2-carboxylic acid 2-diethylaminoethyl ester hydrochloride, 7-[2-(2-acetylamino-4-thiazolyl)-2-((Z)-ethoxycarbonylmethoxy)imino]acetamido]-3-( vinyl)-8-oxo-5-thia-1-azabicyclo[4.2.0]oct-2-ene-2-carboxylic acid 2-diethylaminoethyl ester hydrochloride, penicillin V, penicillin O, methicillin, oxacillin, cloxacillin, dicloxacillin, ampicillin, azlocillin, mezlocillin, piperacillion, cephalothin, cefamandole, cefuroxime, cefoxitin, ceforanide, cefaclor, cefotaxime, ceftizoxime, cefoperazone, cefpodoxime proxetil, or cefixime diffuse through human skin. The results suggest that most of the pro-drugs of antibiotics diffuse through human skin hundred times faster than do the parent antibiotics. Only cefpodoxime proxetil has very high skin penetration rate. The reason is that cefprodoxime has a positive charged amino group and a lipophilic portion. Watts, et al. (U.S. Pat. Nos. 6,191,143 and 5,929,086) have demonstrated that topical administration of cefpodoxime proxetil is effective for the treatment of a systemic bacterial disease, but they did not know the reason at that time. The results suggest that the positive charge on the dialkyaminoethyl group has a very important role in the passage of the drug across the membrane and skin barrier. The positive charge on the amino groups of these pro-drugs will bond to the negative charge on the phosphate head group of membrane. This bonding will disturb the membrane a little bit and may make some room for the lipophilic portion of the prodrug. When the molecules of membrane move, the membrane may 'crack' a little bit due to the bonding of the prodrug. This will let the prodrug insert into the membrane. At pH 7.4, only about 99% of amino group is protonated. When the amino group is not protonated, the bonding between the amino group of the prodrug and the phosphate head group of membrane will disassociate, and the prodrug will enter the membrane completely. When the amino group of the prodrug flips to the other side of the membrane and thus become protonated, then the prodrug is pulled into the cytosol, a semi-liquid concentrated aqueous solution or suspension.

The in vivo rates of penetration of through the skin and blood-brain barrier of intact hairless mice were studied. The donor consisted of a 20% solution of 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride and 6-[3-(o-chlorophenyl)-5-methyl-4-isoxazolecarboxamido]penicillinic acid 2-diethylaminoethyl ester hydrochloride in 1 mL of isopropanol applied to a 10 cm² on the backs of the hairless mice. After 2 hours, the mice were killed. 5 ml of methanol was added 1 g of homogenized blood, liver, kidney, muscle, or brain. The samples were centrifuged for 5 min and analyzed by HPLC. The amount of the parent drugs is 50 +/-7 µg/g in blood, 40 +/-5 µg/g in liver, 35 +/-5 µg/g in kidney, 40 +/-6 µg/g in muscle, 20 +/-5 µg/g in brain for methicillin, 55 +/-7 µg/g in blood, or 38 +/-5 µg/g in liver, 36 +/-5 µg/g in kidney, 32 +/-4 µg/g in muscle, 18 +/-5 µg/g in brain for oxacillin, and 45 +/-7 µg/g in blood, or 34 +/-5 µg/g in liver, 32 +/-5 µg/g in kidney, 30 +/-4 µg/g in muscle, 16 +/-5 µg/g in brain for cloxacillin. The results show these prodrugs have very high pentration rate of blood-brain barrier.

In vitro plasma hydrolysis studies, 10 mg of the prodrug was dissolved in 0.1 ml of 0.2M pH 7.4 phosphate buffer and 1 ml of human plasma, preheated to 37C, was added into the mixture. The mixture was kept in a water bath at 37C, and at every 2 min intervals. 0.2 ml of samples were withdrawn and added to 0.4 ml of methanol to precipitate the plasma protein. The samples were centrifuged for 5 min and analyzed by HPLC. The half life of the prodrugs are 8+/- 1 min., 8+/- 1 min., 10+/- 1 min., 12+/- 1 min., 8+/- 1 min., 12+/- 1 min., 10+/- 1 min., 9+/- 1 min., 13+/- 1 min., 15+/- 1 min., 9+/- 1 min., 10+/- 1 min., 8+/- 1 min., 7+/- 1 min., 9+/- 1 min., 8+/- 1 min., 10+/- 1 min., 11+/- 1 min., 12+/- 1 min., 8+/- 1 min., and 9+/- 1 min. for 6-phenoxyacetacetamidopenicillanic acid 2-diethylaminoethyl ester hydrochloride, allylmercaptomethylpenicillinic acid 2-diethylaminoethyl ester hydrochloride, 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride, 6-[3-(o-chlorophenyl)-5-methyl-4-isoxazolecarboxamido]penicillinic acid 2-diethylaminoethyl ester hydrochloride, 6-[3-(2,6-dichlorophenyl)-5-methyl-4-isoxazolecarboxamido]penicillinic acid 2-diethylaminoethyl ester hydrochloride, 6-[D(-)-α-acetamidophenylacetamidopenicillinic acid 2-diethylaminoethyl ester hydrochloride, D-α-[(imidazolidin-2-on-1-yl)carbonylamino]benzylpenicillin 2-diethylaminoethyl ester hydrochloride, 6R-[2-[3-(methylsulfonyl)-2-oxo-1-imidazolidinecarboxamido]-2-phenylacetamido]pe nicillinic acid 2-diethylaminoethyl ester hydrochloride, 6-D(-)-α-(4-ethyl-2,3-dioxo-1-piperazinylcarbonylamino)-α-phenylacetamidopenicilli nic acid 2-diethylaminoethyl ester hydrochloride, 7-(2-thienylacetamido)cephalosporanic acid 2-diethylaminoethyl ester hydrochloride, 7-[(hydroxyphenylacetyl)amino]-3-[[(1-methyl-1H-tetrazol-5-yl)thio]methyl]-8-oxo-5-thia-1-azabicyclo[4.2.0]oct-2-ene-2-carboxylic acid 2-diethylaminoethyl ester hydrochloride, 3-[[(aminocarbonyl)oxy]methyl]-7-[[2-furanyl(methoxyimino)acetyl] amino]-8-oxo-5-thia-1-azabicyclo[4.2.0]oct-2-ene-2-carboxylic acid 2-diethylaminoethyl ester hydrochloride, 3-[[(aminocarbonyl)oxy]methyl] - 7-methoxy-8-oxo-7-[(2-thienylacetyl)amino]-5-thia-1-azabicyclo[4.2.0]oct-2-ene-2-car boxylic acid 2-diethylaminoethyl ester hydrochloride, 7-[[[2-(acetylaminomethyl)phenyl]acetyl]amino]-3-[[[1-(ethoxylcarbonylmethyl)-1H-t etrazol-5-yl]thio]methyl]-8-oxo-5-thia-1-azabicyclo[4.2.0]oct-2-ene-2-carboxylic acid 2-diethylaminoethyl ester hydrochloride, 7-[(acetylaminophenylacetyl)amino] - 3-chloro-8-oxo-5-thia-1-azabicyclo[4.2.0]oct-2-ene-2-carboxylic acid 2-diethylaminoethyl ester hydrochloride, 3-[(acetyloxy)methyl] - 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7- [[[[(4-ethyl-2,3-dioxo-1-piperazinyl)carbonyl]amino](4-acetoxyphenyl)acetyl]amino ]-3-[[(1-methyl-1H-tetrazol-5-yl)thio]methyl]-8-oxo-5-thia-1-azabicyclo[4.2.0]oct-2-e ne-2-carboxylic acid 2-diethylaminoethyl ester hydrochloride, 7-[2-(2-acetylamino-4-thiazolyl)-2-((Z)-methoxyimino)acetamido]-3-(methoxymethyl) -8-oxo-5-thia-1-azabicyclo[4.2.0]oct-2-ene-2-carboxylic acid 2-diethylaminoethyl ester hydrochloride, 7-[2-(2-acetylamino-4-thiazolyl)-2-((Z)-ethoxycarbonylmethoxy)imino]acetamido]-3-( vinyl)-8-oxo-5-thia-1-azabicyclo[4.2.0]oct-2-ene-2-carboxylic acid 2-diethylaminoethyl ester hydrochloride.

To study efficacy of topical antibiotic treatment in lactating cows with clinical mastitis, 90 lactating dairy cows were recruited. Bacteriological care was considered to have been achieved if the samples taken from the affected quarter on day 17 and day 22 were free of the bacterial species isolated in the pretreatment sample. Clinical cure was defined as the disappearance of clinical signs of disease which were observed on day 1 before treatment, in other words by the return to normal feed intake, rectal temperature<39.0°C, good general condition, absence of udder edema, normal milk appearance, and normal milk yield. 500 mg of 6-phenoxyacetacetamidopenicillanic acid 2-diethylaminoethyl ester hydrochloride (penicillin V-DAEE), 6-(2,6-dimethoxybenzamido)penicillinic acid 2-diethylaminoethyl ester hydrochloride (methicillin-DAEE), or 7-[[(2-acetylamino-4-thiazolyl)(methoxyimino)acetyl]amino] - 8-oxo-5-thia-1-azabicyclo[4.2.0]oct-2-ene-2-carboxylic acid 2-diethylaminoethyl ester hydrochloride (ceftizoxime-DAEE) in 10 ml of pH 7.4 phosphate buffer (0.2 M) was sprayed on the skin of udder twice per day. The results are shown in table 1 and 2.

**Table 1. Clinical cure rates of the topical treatment of cow mastitis with the novel prodrugs of antibiotics.**

| Prodrugs | Number of Cows | Cure Rate (%) | | | |
|---|---|---|---|---|---|
| | | Day 3 | Day 8 | Day 17 | Day 22 |
| Penicillin V-DAEE | 30 | 50 | 90 | 93 | 97 |
| Methicillin-DA EE | 30 | 43 | 90 | 97 | 100 |
| Ceftizoxime-D AEE | 30 | 53 | 93 | 99 | 100 |

**Table 2. Bacteriological cure rates (day 22) of the topical treatment of cow mastitis with the novel prodrugs of antibiotics.**

| Pathogens | | Prodrugs | | |
|---|---|---|---|---|
| | | Penicillin V-DAEE | Methicillin-DA EE | Ceftizoxime-DA EE |
| Staphylococcus aureus | No of cows | 6 | 5 | 6 |
| | No. cured (%) | 4 (67%) | 4 (80%) | 5 (83%) |
| Streptococcus uris | No of cows | 10 | 10 | 11 |
| | No. cured (%) | 8 (80%) | 7 (70%) | 9 (82%) |
| E. coli | No of cows | 8 | 10 | 8 |
| | No. cured (%) | 7 (87.5%) | 8 (80%) | 7 (87.5%) |
| Coagulase-negativ e staphylococci | No of cows | 9 | 7 | 8 |
| | No. cured (%) | 7 (78%) | 6 (85.7%) | 7 (87.5%) |
| Enterobacteriacea e | No of cows | 7 | 8 | 6 |
| | No. cured (%) | 6 (85.7) | 6 (75%) | 5 (83.3%) |

The prodrugs have demonstrated very high Clinical and Bacteriological cure rates.

After 1 hours of topical application, the milk samples were taken and analysized. The amount of the parent drugs is 80 +/-7 µg/g in milk for penicillin V, 75 +/-6 µg/g methicillin, and 70 +/-7 µg/g for ceftizoxime. The results have shown that these prodrugs have very high penetration rate of blood-milk barrier. Their very high penetration rates of blood-milk or blood-brain barrier make them very valuable for treatment of brain, breast, prostate gland and other infections.

The cell wall of gram-negative bacteria contains an outer membrane (lipophilic) and periplasm (hydrophilic) that make many antibiotics unpassible. These novel prodrugs of beta-lactam antibiotics have a lipophilic portion and a hydrophilic portion that make them highly passible to both of outer membrane and periplasm, so they can easily reach the penicillin binding proteins. On the very outside of the cell wall of gram-positive bacteria is a set of characteristic carbohydrates and proteins that are hydrophilic. The hydrophilic portion of the prodrugs will facilitate the passage of these pro-drugs through the carbohydrates and proteins barrier to reach the penicillin binding proteins. These prodrugs should have very broad antibiotics spectrum to both of gram-negative bacteria and gram-positive bacteria and have much less antibiotic-resistance problem.

The compounds of the general formula (1) 'Structure 1' indicated above can be prepared from beta-lactam antibiotics (free acids), by reaction with compounds of the general formula (2) 'Structure 2' by using coupling reagents, such as N,N'-Dicyclohex ylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al. 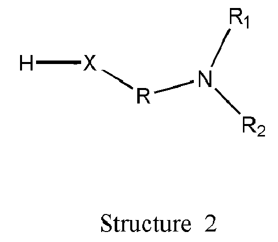 wherein, R represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R represents a branched or straight chain, -(CH₂)ₙ-, aryl residues or heteroaryl residues; n = 0, 1, 2, 3, 4, 5, 6, 7, 8, ....; X represents O, S, or NH.

When X represents O, the compounds of the general formula (1) 'Structure 1' ind icated above can be prepared from metal salts, organic base salts, or immobilized base salts of beta-lactam antibiotics, by reaction with compounds of the general formula (3) 'Structure 3': 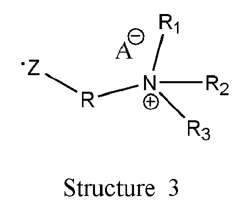 wherein, R represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; Z represents F, Cl, Br, I, or p-toluenesulphonyl; A⁻ represents Cl⁻, Br⁻, F⁻, I⁻, AcO⁻, citrate, or any negative ions; and n=0,1,2,3,4,5...; R represents a branched or straight chain, -(CH₂)ₙ-, aryl residues or heteroaryl residues; n = 0, 1, 2, 3, 4, 5, 6, 7, 8, ....; X represents O, S, or NH.

### Advantageous Effects

These pro-drugs of beta-lactam antibiotics have a lipophilic portion and a hydrophilic portion (the amine groups that exist in the protonated form at physiological pH). The positively charged amino groups of these pro-drugs have two major advantages. First, it makes prodrugs soluble in water; when these new pro-drugs are administered transdermally in a dosage form such as solution, spray, lotion, ointment, emulsion or gel, they will mix with moisture on the skin, eye, genital area, mouth, nose, or other part of the body immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. This bonding will also disturb the membrane a little bit and may make some room for the lipophilic portion (by modification of the polar groups with lipophilic alkyl groups) of the prodrug. When the molecules of membrane move, the membrane may 'crack' a little bit due to the bonding of the prodrug. This will let the prodrug insert into the membrane. At pH 7.4, only about 99% of amino group is protonated. When the amino group is not protonated, the bonding between the amino group of the prodrug and the phosphate head group of membrane will disassociate, and the prodrug will enter the membrane completely. When the amino group of the prodrug flips to the other side of the membrane and thus become protonated, then the prodrug is pulled into the cytosol, a semi-liquid concentrated aqueous solution or suspension. The high penetration rates of the skin, membrance, blood-brain and blood-milk barriers make them very valuable for treatment of brain, breast, prostate gland and other infections. These prodrugs have very broad antibiotics spectrum to both of gram-negative bacteria and gram-positive bacteria and have much less antibiotic-resistance problem.

### Description of Drawings

Figure 1: Cumulative amounts of 6-phenoxyacetacetamidopenicillanic acid 2-diethylaminoethyl ester hydrochloride, allylmercaptomethylpenicillinic acid 2-diethylaminoethyl ester hydrochloride, 6-(2,6-dimethoxybenzamido)penicillinic acid 2-diethylaminoethyl ester hydrochloride, 6-(5-methyl-3-phenyl-2-isoxazoline-4-carboxamido)penicillinic acid 2-diethylaminoethyl ester hydrochloride, 6-[3-(o-chlorophenyl)-5-methyl-4-isoxazolecarboxamido]penicillinic acid 2-diethylaminoethyl ester hydrochloride, 6-[3-(2,6-dichlorophenyl)-5-methyl-4-isoxazolecarboxamido]penicillinic acid 2-diethylaminoethyl ester hydrochloride, penicillin V, penicillin O, methicillin, oxacillin, cloxacillin, dicloxacillin, crossing isolated human skin tissue in Franz cells (n=5). In each case, the vehicle was pH 7.4 phosphate buffer (0.2 M).
Figure 2: Cumulative amounts of 6-[D(-)-α-acetamidophenylacetamidopenicillinic acid 2-diethylaminoethyl ester hydrochloride, D-α-[(imidazolidin-2-on-1-yl)carbonylamino]benzylpenicillin 2-diethylaminoethyl ester hydrochloride, 6R-[2-[3-(methylsulfonyl)-2-oxo-1-imidazolidinecarboxamido] - 2-phenylacetamido]penicillinic acid 2-diethylaminoethyl ester hydrochloride, 6-D(-)-α-(4-ethyl-2,3-dioxo-1-piperazinylcarbonylamino)-α-phenylacetamidopenicilli nic acid 2-diethylaminoethyl ester hydrochloride, 7-(2-thienylacetamido)cephalosporanic acid 2-diethylaminoethyl ester hydrochloride, ampicillin, azlocillin, mezlocillin, piperacillion, cephalothin, crossing isolated human skin tissue in Franz cells (n=5). In each case, the vehicle was pH 7.4 phosphate buffer (0.2 M).
Figure 3: Cumulative amounts of 7-[(hydroxyphenylacetyl)amino] - 3-[[(1-methyl-1H-tetrazol-5-yl)thio]methyl]-8-oxo-5-thia-1-azabicyclo[4.2.0]oct-2-ene -2-carboxylic acid 2-diethylaminoethyl ester hydrochloride, 3-[[(aminocarbonyl)oxy] methyl]-7-[[2-furanyl(methoxyimino)acetyl]amino]-8-oxo-5-thia-1-azabicyclo[4.2.0] oct-2-ene-2-carboxylic acid 2-diethylaminoethyl ester hydrochloride, 3-[[(aminocarbonyl)oxy]methyl]-7-methoxy-8-oxo-7-[(2-thienylacetyl)amino]-5-thia-1 -azabicyclo[4.2.0]oct-2-ene-2-carboxylic acid 2-diethylaminoethyl ester hydrochloride, 7-[[[2-(acetylaminomethyl)phenyl]acetyl]amino]-3-[[[1-(ethoxylcarbonylmethyl)-1H-t etrazol-5-yl]thio]methyl]-8-oxo-5-thia-1-azabicyclo[4.2.0]oct-2-ene-2-carboxylic acid 2-diethylaminoethyl ester hydrochloride, 7-[(acetylaminophenylacetyl)amino] - 3-chloro-8-oxo-5-thia-1-azabicyclo[4.2.0]oct-2-ene-2-carboxylic acid 2-diethylaminoethyl ester hydrochloride, cefamandole, cefuroxime, cefoxitin, ceforanide, cefaclor, crossing isolated human skin tissue in Franz cells (n=5). In each case, the vehicle was pH 7.4 phosphate buffer (0.2 M).

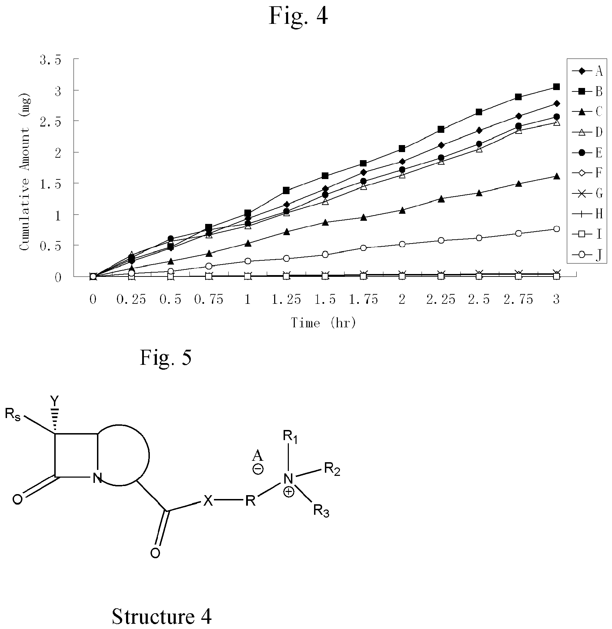

Figure 4: Cumulative amounts of 3-[(acetyloxy)methyl] - 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[(2-acetylamino-4-thiazolyl)(methoxyimino)acetyl]amino]-8-oxo-5-thia-1-azabicycl o[4.2.0]oct-2-ene-2-carboxylic acid 2-diethylaminoethyl ester hydrochloride, 7-[[[[(4-ethyl-2,3-dioxo-1-piperazinyl)carbonyl]amino](4-acetoxyphenyl)acetyl]amino ]-3-[[(1-methyl-1H-tetrazol-5-yl)thio]methyl]-8-oxo-5-thia-1-azabicyclo[4.2.0]oct-2-e ne-2-carboxylic acid 2-diethylaminoethyl ester hydrochloride, 7-[2-(2-acetylamino-4-thiazolyl)-2-((Z)-methoxyimino)acetamido]-3-(methoxymethyl) -8-oxo-5-thia-1-azabicyclo[4.2.0]oct-2-ene-2-carboxylic acid 2-diethylaminoethyl ester hydrochloride, 7-[2-(2-acetylamino-4-thiazolyl)-2-((Z)-ethoxycarbonylmethoxy)imino]acetamido]-3-( vinyl)-8-oxo-5-thia-1-azabicyclo[4.2.0]oct-2-ene-2-carboxylic acid 2-diethylaminoethyl ester hydrochloride, cefotaxime, ceftizoxime, cefoperazone, cefpodoxime proxetil, or cefixime, crossing isolated human skin tissue in Franz cells (n=5). In each case, the vehicle was pH 7.4 phosphate buffer (0.2 M).
Figure 5. Wherein, X represents O, S, or NH; Y represents H, OH, NHCHO, CH₂ CH₂NHC(=NR₇)NHR₇, OCOCH₃, OCH₃, OC₂H₅, CH₃SO₃, NO₂, CN, CF₃, OCF₃, F, Br, I, or Cl; R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; R represents a branched or straight chain, -(CH₂)ₙ-, aryl residues or heteroaryl residues; n = 0, 1, 2, 3, 4, 5, 6, 7, 8, ...; Rₛ represents the side chains of beta-lactam antibiotics; A⁻ represents Cl⁻, Br⁻, F⁻, I⁻, AcO⁻, citrate, or any negative ions. All R, -(CH₂)ₙ - or -(CH₂)ₘ- groups are branched or straight chains and may include C, H, O, S, or N atoms and may have single, double, and triple bonds. Any CH₂ groups may be replaced with O, S, or NH.

### Best Mode

### Preparation of 6-phenoxyacetacetamidopenicillanic acid 2-diethylaminoethyl ester hydrochloride

39 g (0.1 mol) of Penicillin V potassium was dissolved in 100 ml of acetonitrile. 39 g (0.15 mol) of 2-Bromo-N,N-diethylethylamine.HBr in ethyl acetate was added into the reaction mixture. The mixture was stirred for 3 h at RT. Then 8 g of sodium bicarbonate was added into the reaction mixture. The mixture is stirred for another 2 h at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 3.5 g of HCl in 50 ml of ether was added into the reaction mixture with stirring. The solid product was collected by filtration. After drying, it yielded 38 g of the desired hygroscopic product (78.2%). Solubility in water: 50 mg/ml; Elementary analysis: C₂₂H₃₂ClN₃O₅S; MW: 486.0. Calculated % C: 54.37; H: 6.64; N: 8.65; Cl: 7.29; O: 16.46; S: 6.60; Found % C: 54.32; H: 6.68; N: 8.61; Cl: 7.32; O: 16.51; S: 6.56. ¹H-NMR (400 MHz, D₂O): δ: 1.28 (s, 6H), 1.35 (t, 6H), 3.21 (m, 4H), 3.43 (m, 2H), 4.51 (m, 2H), 4.68 (s, 1 H), 4.79 (s, 2 H), 4.86 (m, 1 H), 5.19 (m, 1H), 6.68 (m, 2H), 6.80 (m, 1H), 7.11 (m, 2H), 7.21 (b, 1H).

The other compounds of the general formula (1) 'Structure 1' can be synthesized by the same processure.

### Mode for Invention

### Preparation of 6-(2,6-dimethoxybenzamido)penicillinic acid 2-diethylaminoethyl ester hydrochloride

38 g (0.1 mol) of 6-(2,6-dimethoxybenzamido)penicillinic acid was dissolved in 300 ml of chloroform. 20.6 g of N, N'-Dicyclohexylcarbodiimide was added into the reaction mixture. 11.7 g of N,N-dimethylaminoethanol and 2g of 4-dimethylaminopyridine were added into the reaction mixture. The mixture was stirred for 10 hours at RT. The solid was removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 3.5 g of HCl in 50 ml of ether was added into the reaction mixture with stirring. The solid product was collected by filtration. After drying, it yielded 40 g of the desired hygroscopic product (77.5%). Solubility in water: 50 mg/ ml; Elementary analysis: C₂₃H₃₄ClN₃O₆S; MW: 516.05. Calculated % C: 53.53; H: 6.64; N: 8.14; Cl: 6.87; O: 18.60; S: 6.21; Found % C: 53.49; H: 6.68; N: 8.11; Cl: 6.90; O: 18.64; S:6.18. ¹H-NMR (400 MHz, D₂O): δ: 1.28 (s, 6H), 1.35 (t, 6H), 3.21 (m, 4H), 3.75 (s, 6H), 3.43 (m, 2H), 4.51 (m, 2H), 4.68 (s, 1 H), 4.84 (m, 1 H), 5.17 (m, 1H), 6.50 (m, 2H), 7.21 (m, 1H), 7.28 (b, 1H).

The other compounds of the general formula (1) 'Structure 1' can be synthesized by the same processure.

### Industrial Applicability

The compounds of the general formula (1) 'Structure 1' can penetrate the skin, blood-brain, and blood-milk barriers easily. They are very valuable for treatment of skin, brain, breast, prostate gland and other infections due to their high penetration rates of the skin, blood-brain, and blood-milk barriers. These prodrugs have very broad antibiotics spectrum to both of gram-negative bacteria and gram-positive bacteria and have much less antibiotic-resistance problem.

The present invention provides:
[1] The compounds of the general formula (1) 'Structure 1' except penethamate hydriodide (3,3-dimethyl-7-oxo-6-[(phenylacetyl)amino] - 4-thia-1-azabicyclo[3.2.0]heptane-2-carboxylic acid 2-(diethylamino)ethyl ester monohydriodide) which is a known compound (the high penetration rate through skin, blood-brain, and blood-milk barriers of this compound have never been mentioned). 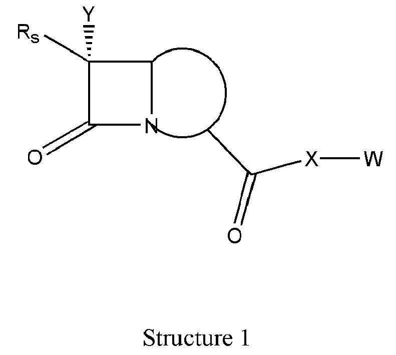 Wherein, X represents O, S, or NH; Y represents H, OH, OR₅, NHCHO, CH₂CH₂ NHC(=NR₇)NHR₇, OCOCH₃, OCH₃, OC₂H₅, CH₃SO₃, NO₂, CN, CF₃, OCF₃, F, Br, I, or Cl; 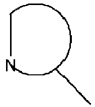 represents 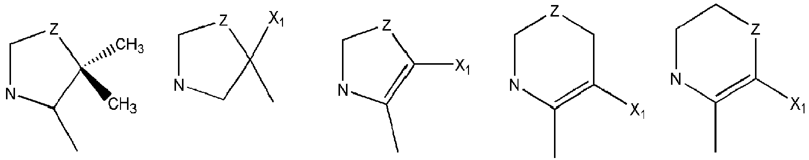 wherein, Z represents S, O, SO, SO₂, NH, CHCH₃, CHCH₂CH₃, or CH₂; -X₁ represents: -H, -OH, -OCH₃, -OC₂H₅, OC₃H₇, -CONH₂, -CH₂OCONH₂, -CH₂ OCOCH₃, -OCOCH₃, -CH₂OCOCH₃, -CH₂OCH₃, -CH₃, -C₂H₅, -C₃H₇, -C₄H₉, C₅H ₁₁, C₆H₁₃, -Cl, -F, -Br, -I, -HC=CHCH₃, -HC=CH₂, -CH₂OCH₃, -S(CH₂)ₙ-NHR₇, 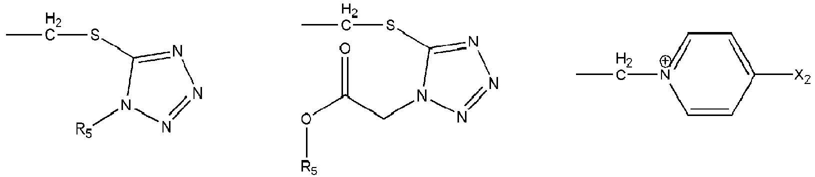 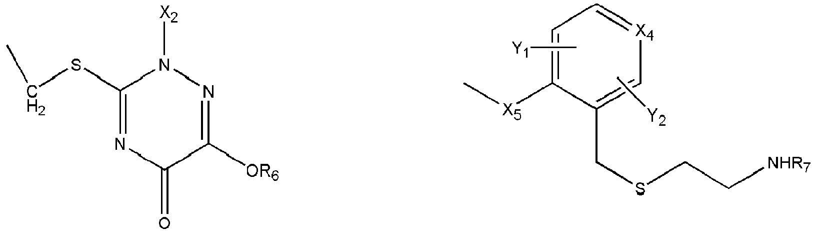 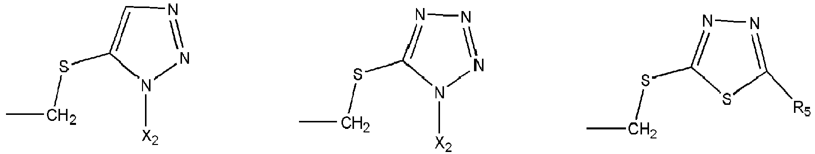 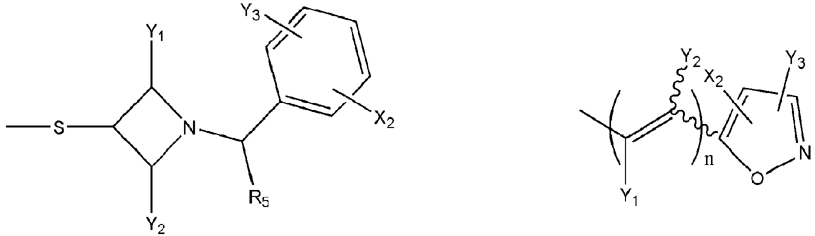 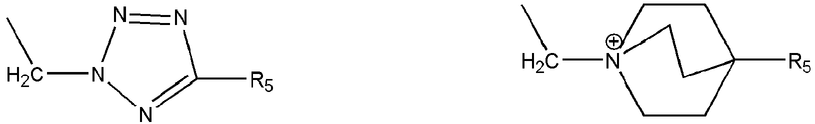 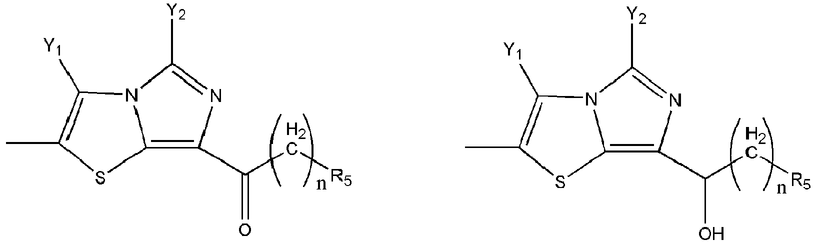 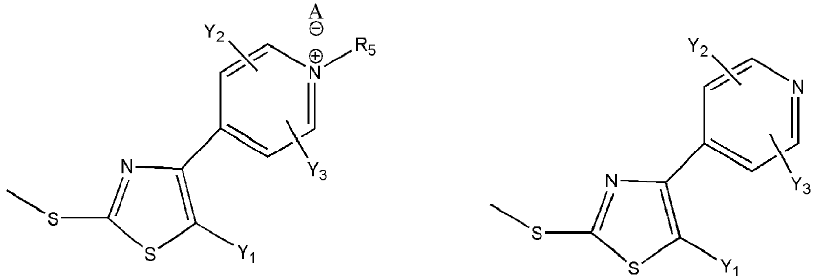 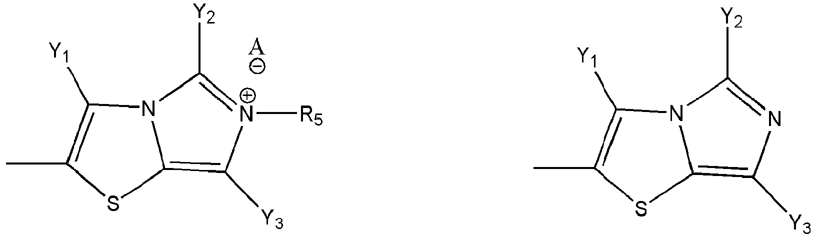 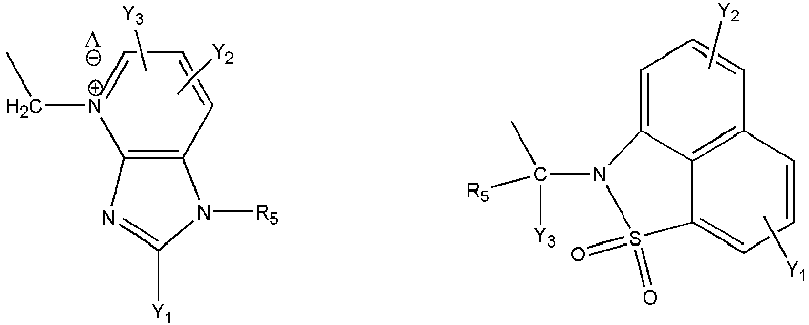 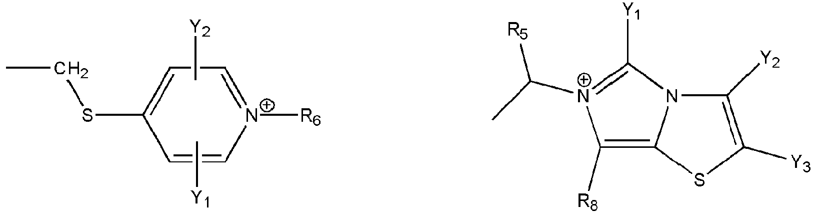 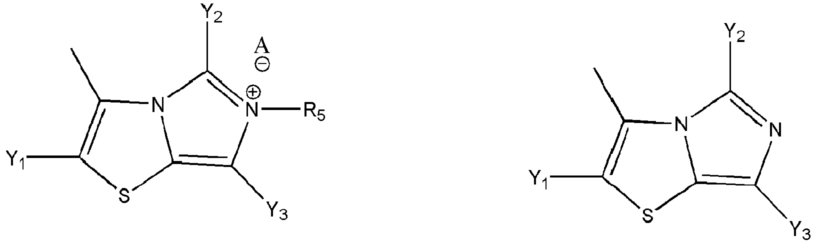 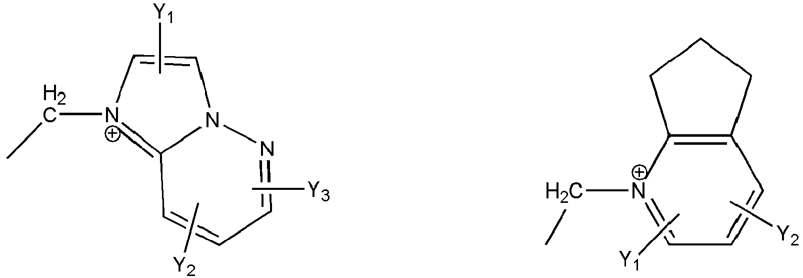 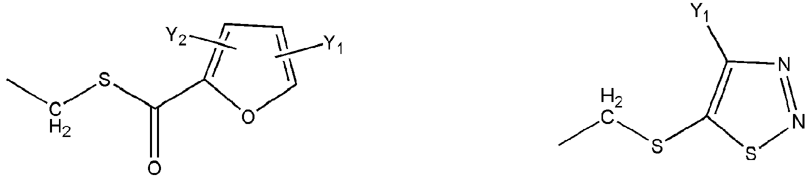 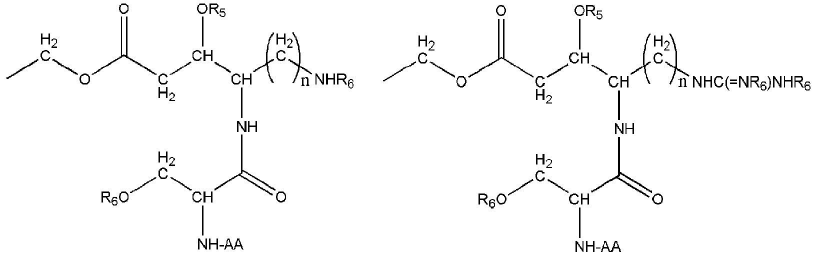 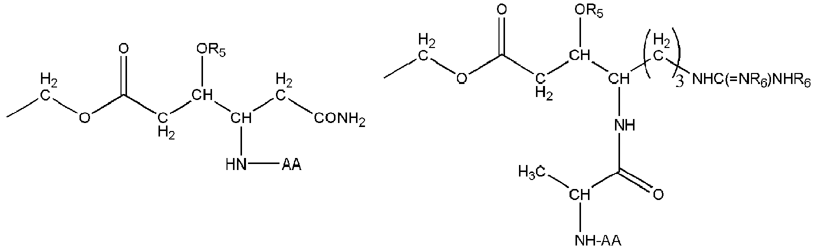 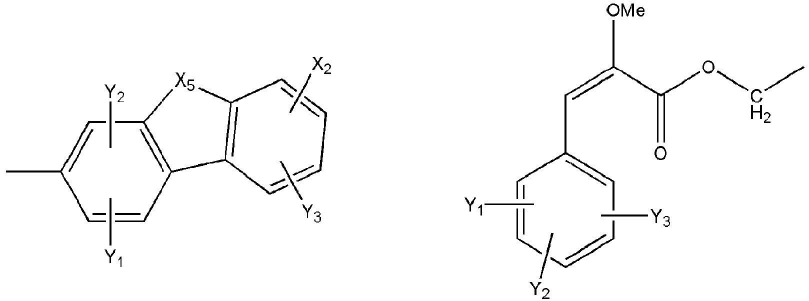 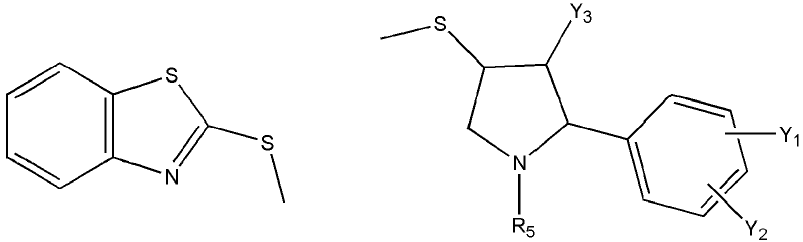 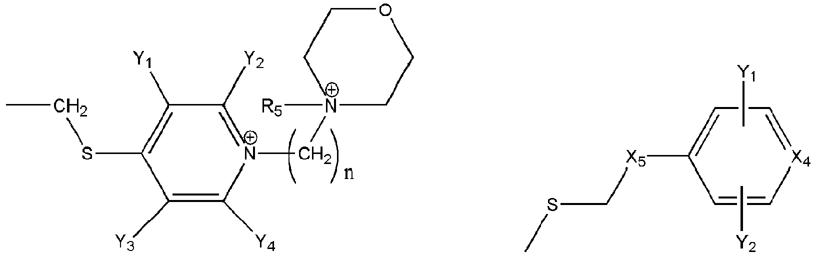 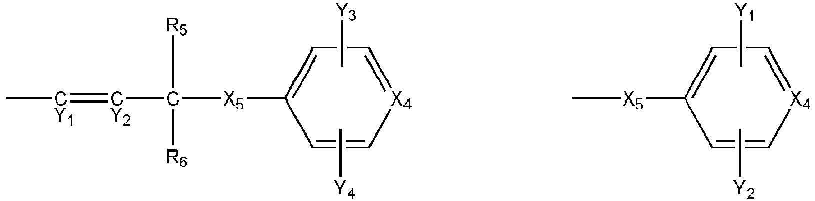 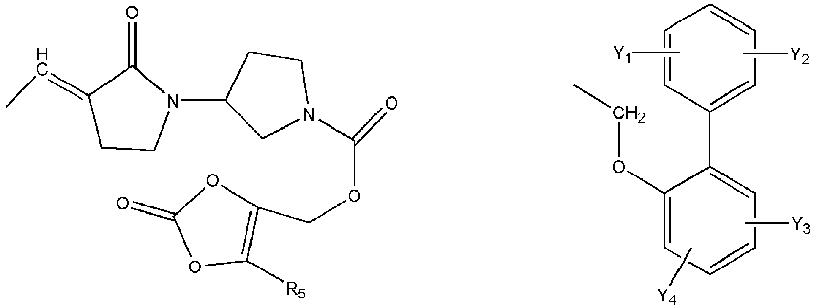 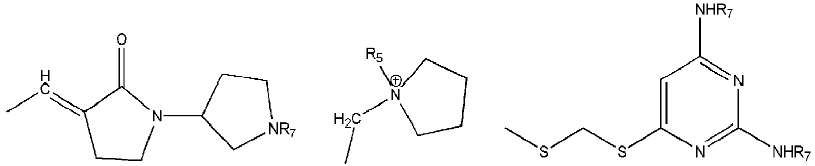 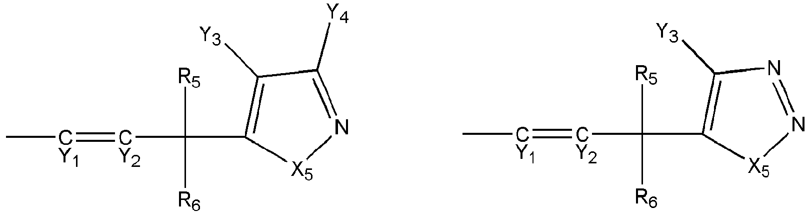 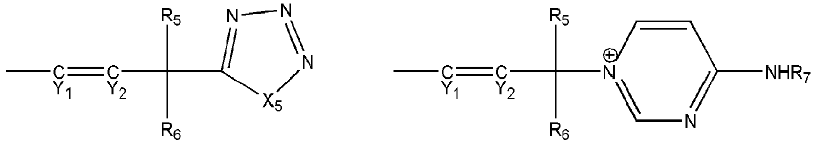 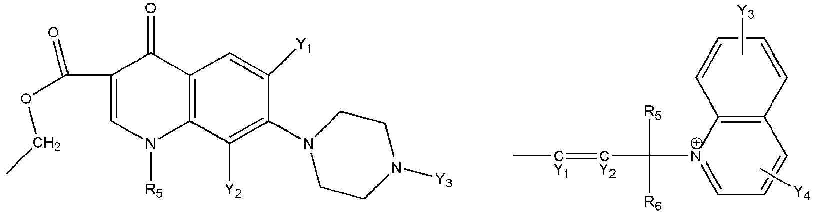 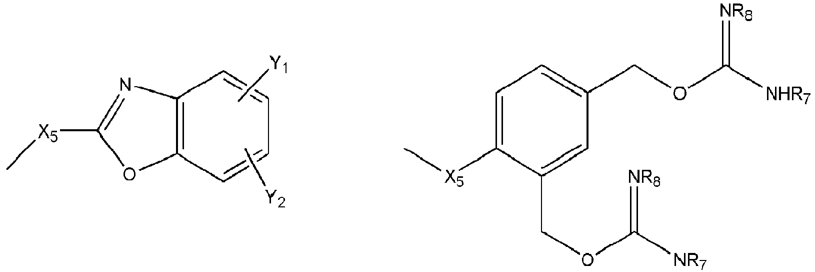 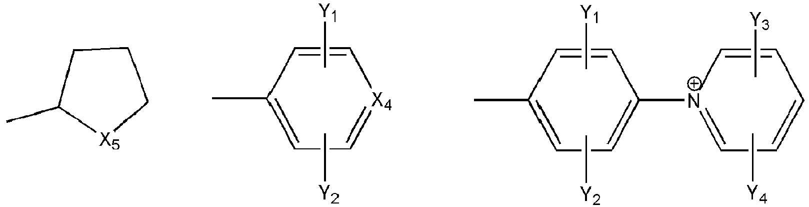 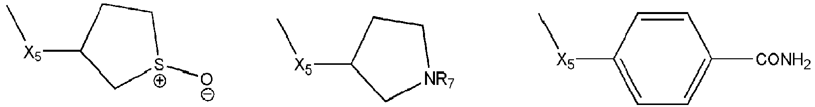 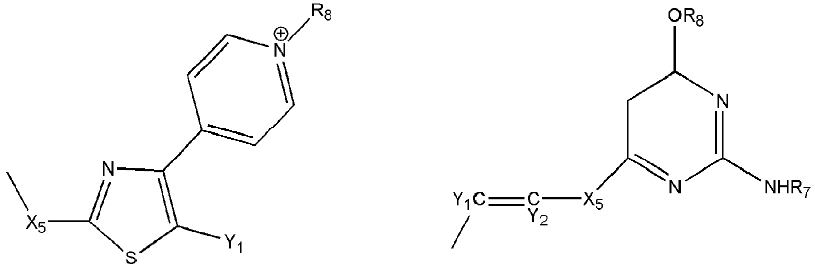 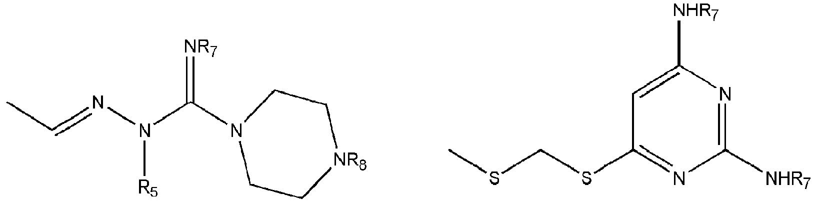 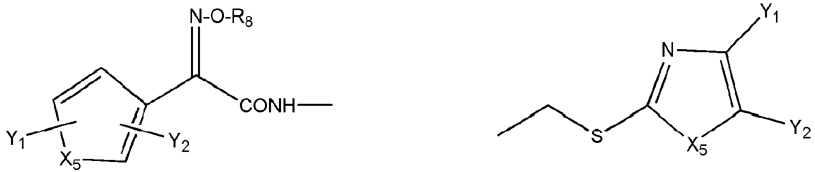 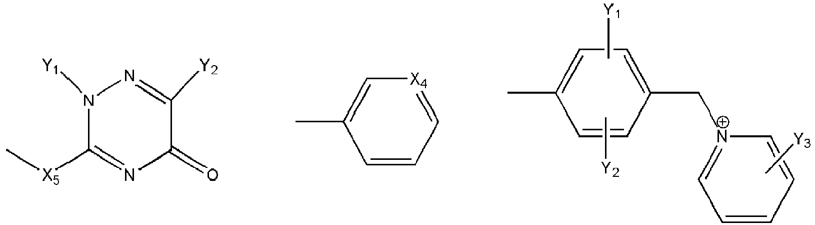 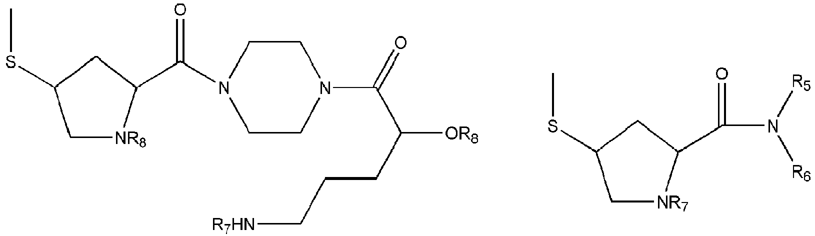 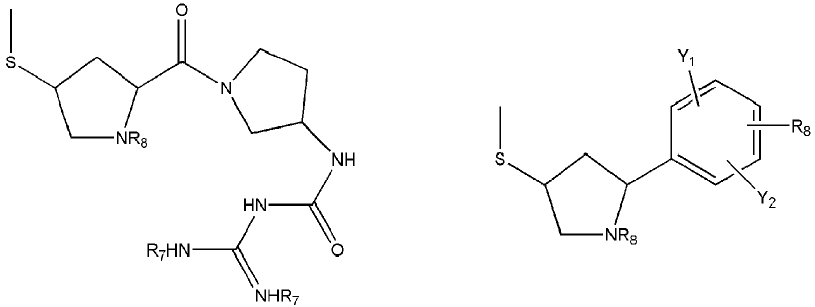 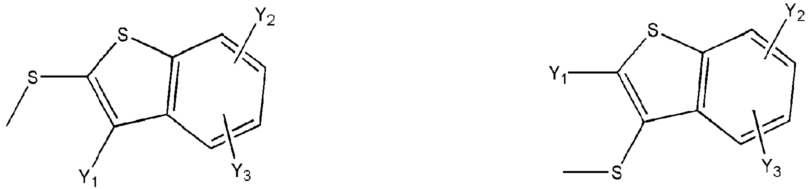 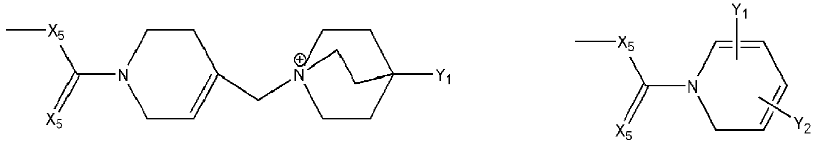 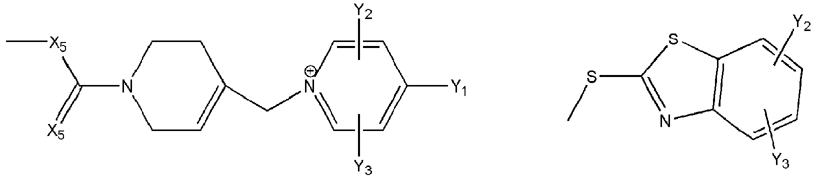 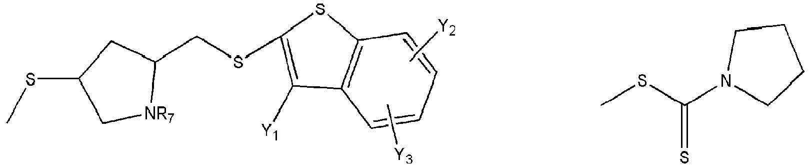 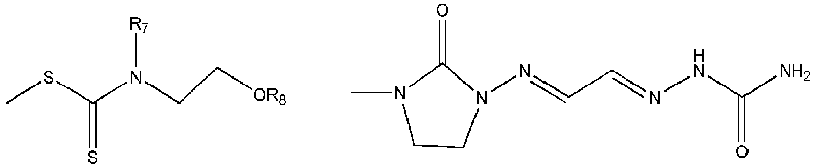 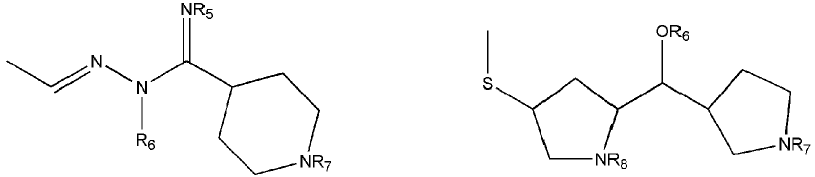 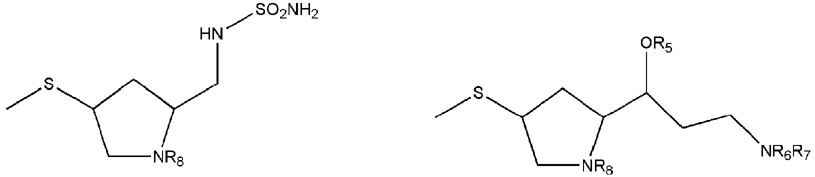 wherein , Z represents S, O, SO, SO₂, NH, CH₂, CHCH₃, or CHCH₂CH₃; R represents H, CONH₂, CH₂CH₂OR₆, CH₂CH₂N(CH₃)₂, CH₂CH₂N(CH₂CH₃)₂, Cl, F, Br, I, one of any alkyl, alkyloxyl, alkenyl or alkynyl residues having 1 to 12 carbon atoms, aryl or heteroaryl residues , CO-W, or W; R₆ represents H, F, Cl, Br, I, Na⁺, K⁺, COR₅, one of any alkyl, alkyloxyl, alkenyl or alkynyl residues having 1 to 12 carbon atoms, aryl or heteroaryl residues , CO-W, or W; R₇ represents H, CH₃, C₂H₅, CF₃, COCH₃, C₃H₇, C₄H₉, C₅H₁₁, CONH₂, COR₆, COR₅, CO-W, or W; R₈ represents H, CH₃, C₂H₅, CF₃, CH₂CH₂F, CH₂CH₂Cl, CH₂CH₂ Br, CH₂CH₂I, CH₂CHF₂, CH₂CF₃, CH₂F, CH₂Cl, CH₂Br, CH₂I, CH₂NR₆R₇, CH(NHR₇)CH₂CONH₂, C₃H₇, C₄H₉, C₅H₁₁, CONH₂, CH₂ CONH₂, CH₂OCONH₂, C(CH₃)₂COOR₆, CH(CH₃)COOR₆, CH₂COOR₆, COR₆, COR₅, CO-W, or W; X represents O, S, NH, or CH₂; X₂ represents H, CH₃, CH₂(CH₂)ₙ OR₈, Cl, F, Br, I, NO₂, CN, CF₃, OCF₃, NH₂, CH₃, C₂H₅, CONH₂, CH₂OCONH₂, CH₂COOR₅, CH₂ (CH)₂ₙN(CH₃)₂, CH₂(CH₂)ₙSO₃R₅, C₁₋₈ alkyl, C₁₋₈ alkylthiol, C₁₋₈ alkylamine, or C alkyloxy; X₃ represents H, CH₃, CH₂(CH₂)ₙOR₈, Cl, F, Br, I, NO₂, CN, CF₃, OCF₃, NH₂, CH₃, C₂H₅, CONH₂, CH₂OCONH₂, CH₂COOR₅, CH₂(CH₂)ₙN(CH₃)₂, CH₂(CH₂)ₙSO₃R₅, C₁₋₈ alkyl, C₁₋₈ alkylthiol, C₁₋₈ alkylamine, or C₁₋₈ alkyloxy; X₄ represents N, CH , or CY₁; X₅ represents CH₂, CHY₁, S, O or NH; Y₁ represents H, OH, OW, OCOW, OCOCH₃, CH₃, C₂H₅, C₃H₇, C₄H₉, CH₂COOR₈, OCH₃, OC₂ H₅, CH₃SO₃, NO₃, CN, CF₃, OCF₃, CH₂(CH₂)ₙNR₅R₆, CH₂(CH₂)ₙOR₆, CH(CONH₂)NHR₆, CH₂CONH₂, F, Br, I, or Cl; Y₂ represents H, OH, OW, OCOW, CH₃, C₂H₅, C₃H₇, C₄H₉, CH₂COOR₈, OCOCH₃, OCH₃, OC₂H₅, CH₃SO₃, NO₂, CN, CF₃, OCF₃, CH₂(CH₂)ₙNR₅R₆, CH₂(CH₂)ₙOR₆, CH(CONH₂)NHR₆, CH ₂CONH₂, F, Br, I, or Cl; Y₃ represents H, OH, OW, OCOW, OCOCH₃, CH₃, C₂H C₃H₇, C₄H₉, CH₂COOR₈, OCH₃, OC₂H₅, CH₃SO₃, NO₂, CN, CF₃, OCF₃, CH₃ (CH₂)ₙNR₅R₆, CH₂(CH₂)ₙOR₆, CH(CONH₂)NHR₆, CH₂CONH₂, F, Br, I, or Cl; Y represents H, OH, OW, OCOW, OCOCH₃, CH₃, C₂H₅, C₃H₇, C₄H₉, CH₂COOR₈, OCH₃, OC₂H₅, CH₃SO₃, NO₂, CN, CF₃, OCF₃, CH₂(CH₂)ₙNR₅R₆, CH₂(CH₂)ₙOR₆, CH(CONH₂)NHR₆, CH₂CONH₂, F, Br, I, or Cl; AA represents any amino acids; n=0, 1, 2, 3, 4, 5, 6,...; Rₛ - represents: 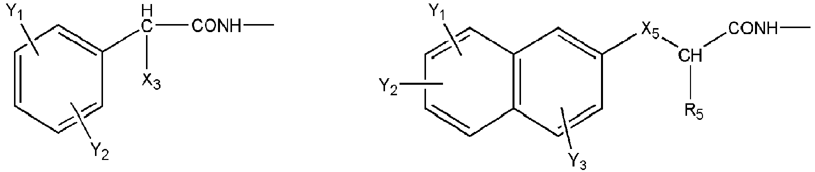 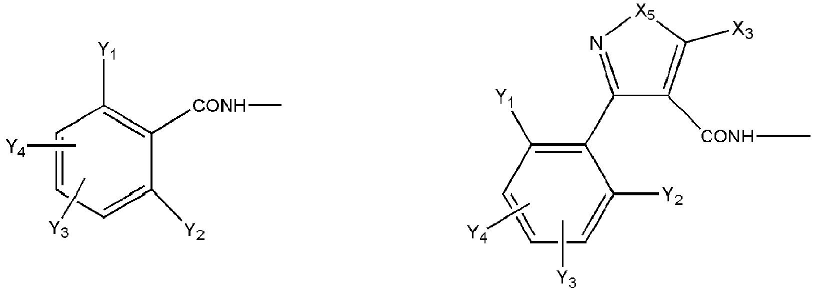 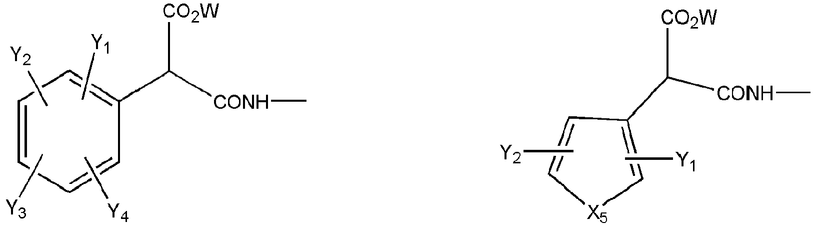 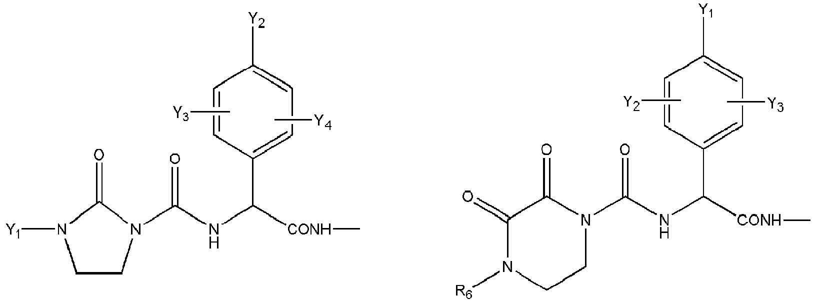 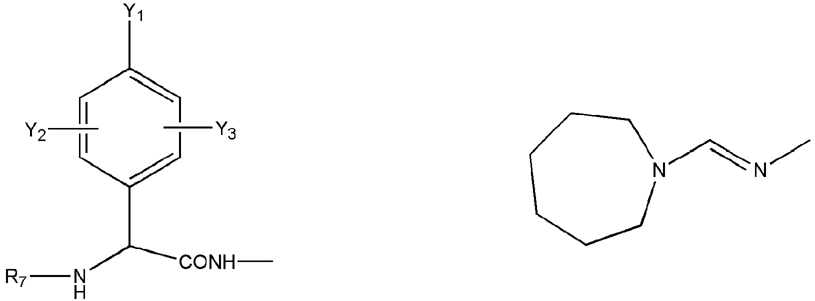 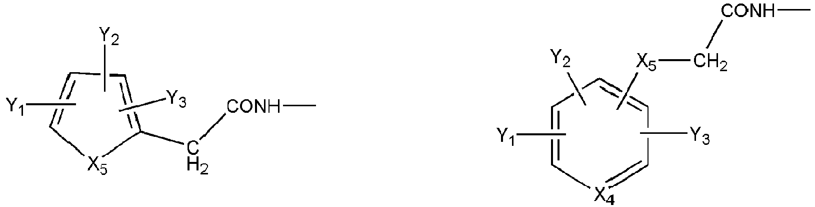 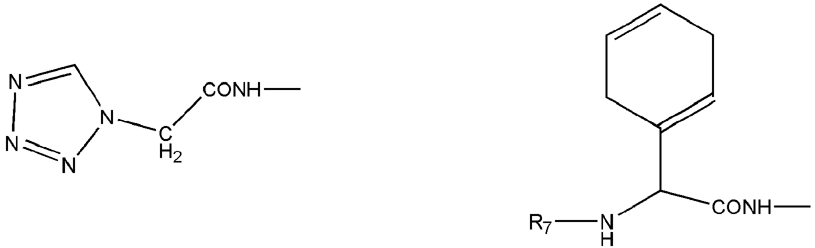 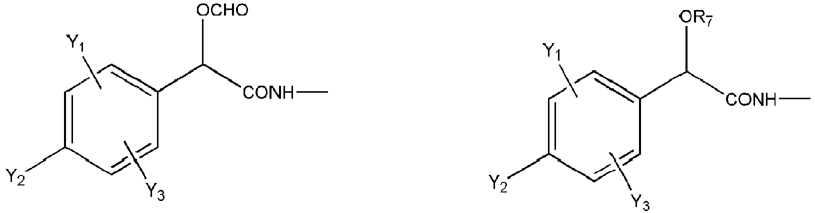 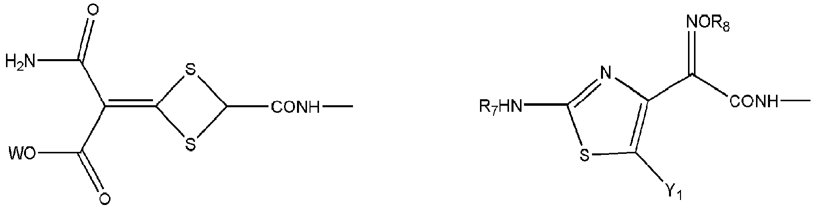 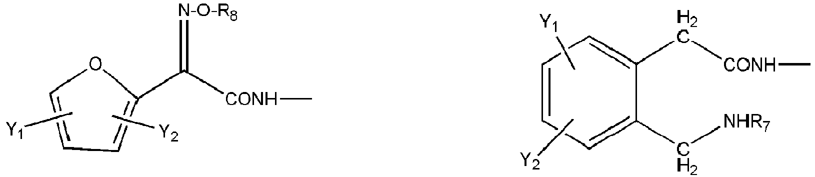 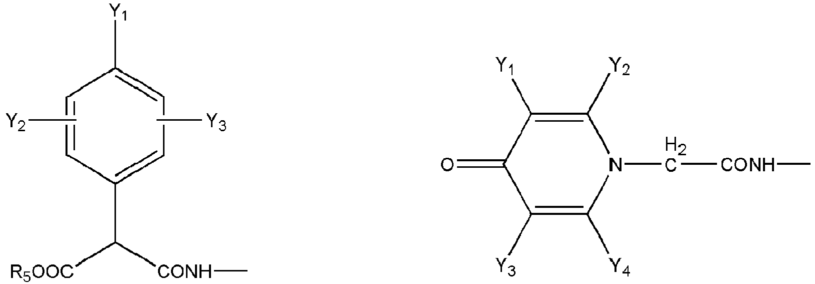 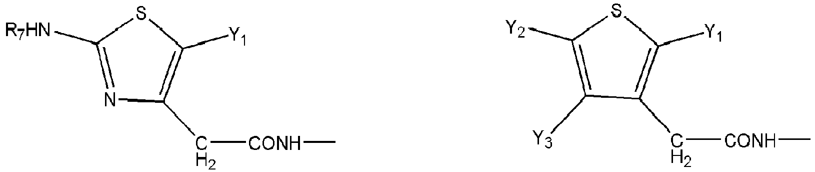 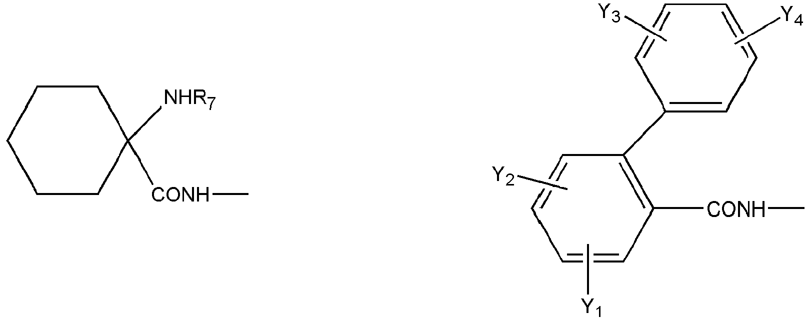 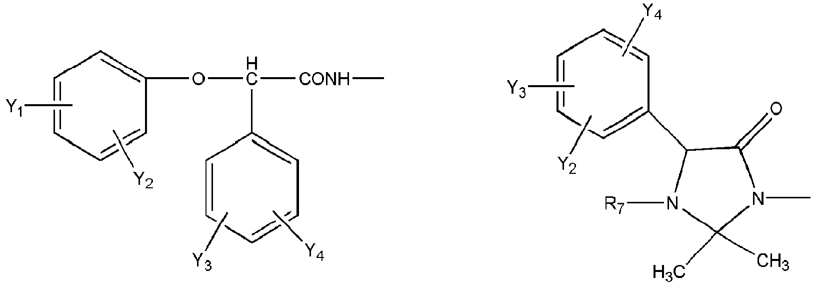 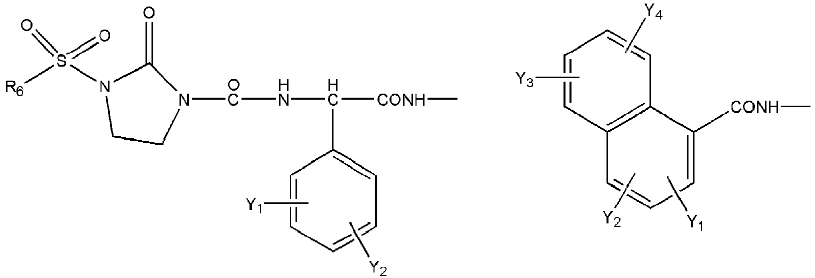 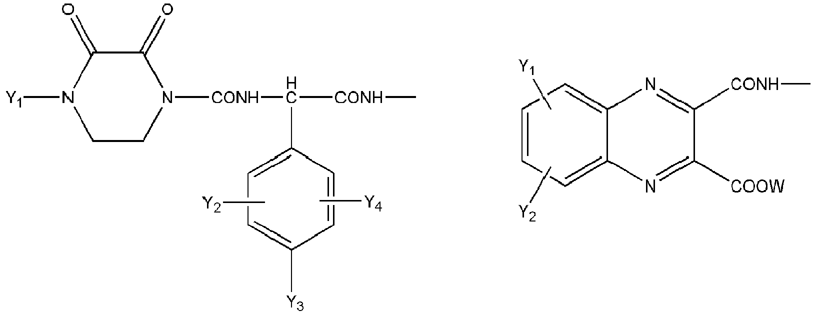 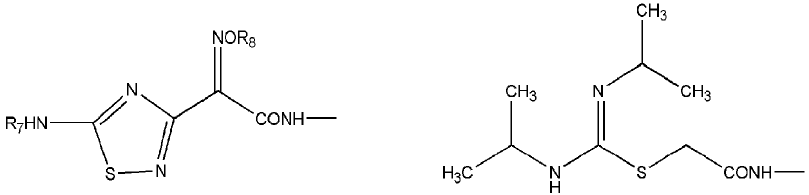 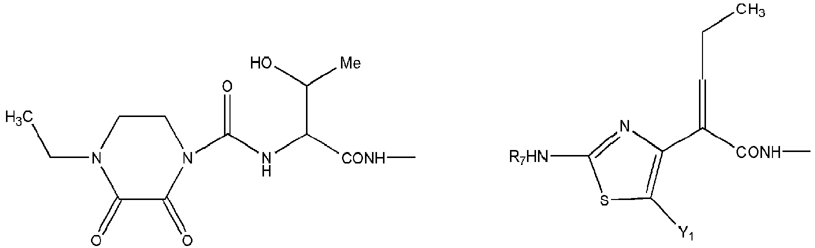 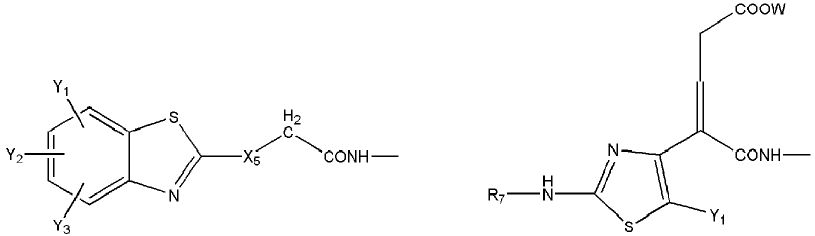 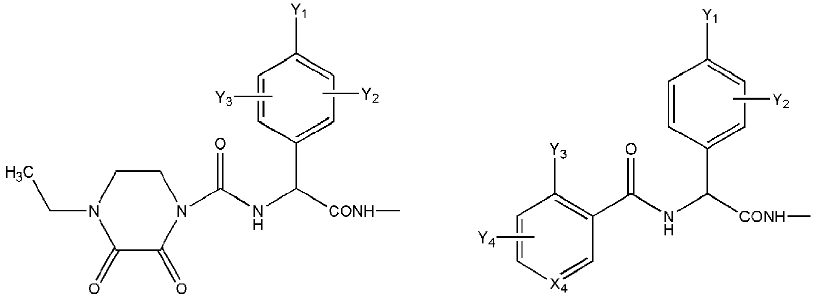 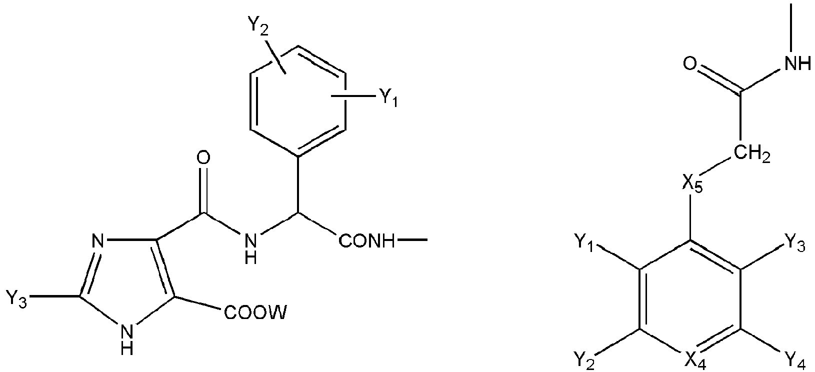 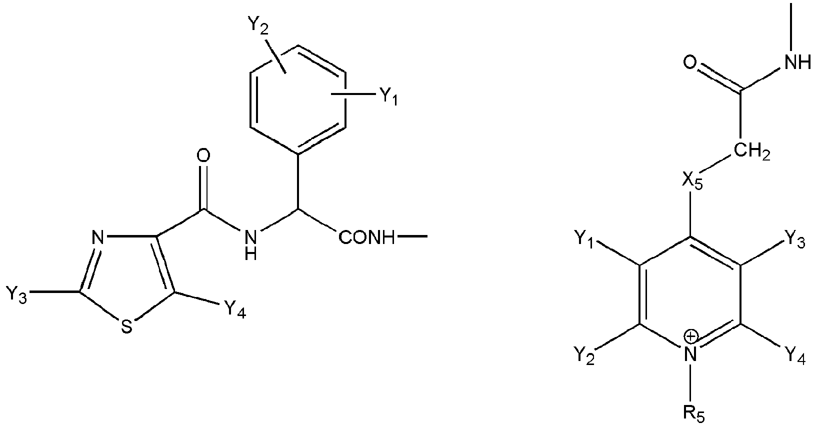 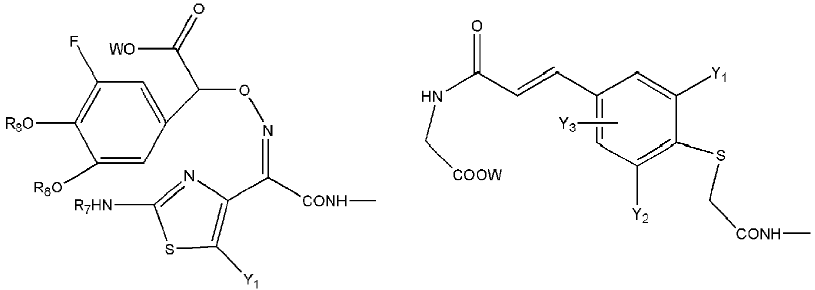 R₆OOCCH(NHR₇)(CH₂)ₙCONH-, R₆OOCCH(NHR₇)(CH₂)ₙSCONH- , CF₃SCH₂ CONH-, CF₃CH₂CONH- , CHF₂SCH₂CONH- , CH₂FSCH₂CONH- , NH₂ COCHFSCH₂CONH-, R₇NHCH(COOW)CH₂SCH₂CONH-, CNCH₂SCH₂ CONH-, CH₃(CH₂)ₙCONH-, R₇N=C(NR₇)CH₂CH₂S-, R₇N=C(NHR₇)NHCO-, CH₃C(Cl)=CHCH₂SCH₂CONH-, (CH₃)₂C(OR₆)-, CNCH₂CONH-, CNCH₂CH₂S-, R₇N=C(NR₇)CH₂CH₂S-, CH₂=CHCH₂SCH₂CONH-, CH₃CH(OH)-, CH₃CH(OR₈ )-, CH CH(Y₁)-, (CH₃)₂ CH-, CH₃CH₂-, or CH₃(CH₂)ₙCH=CH(CH₂)ₘCONH-. Wherein, n or m= 0, 1, 2, 3, 4, 5, 6, ...; X₃ represents H, N₃, SO₃, F, Cl, Br, I, OH, OCH₃, OC₂H₅, CH₃, C₂H₅, COOW, OW, or W; X₄ represents N, CH , or CY ₁; X₅ represents CH₂, S, O or NH; R₅ represents H, -CONH, CH₂CH₂N(CH₃)_{2'} CH₂CH₂N(CH₂CH₃)₂, CH₂CH₂OR₆, Cl, F, Br, I, one of any alkyl, alkyloxyl, alkenyl or alkynyl residues having 1 to 12 carbon atoms, aryl or heteroaryl residue, CO-W, or W; R₆ represents H, F, Cl, Br, I, 2-oxo-1-imidazolidinyl, one of any alkyl, alkyloxyl, alkenyl or alkynyl residues having 1 to 12 carbon atoms, aryl (phenyl, 5-indanyl, 2,3-dihydro-1H-inden-5-yl, et al.) or heteroaryl (4-hydroxy-1,5-naphthyridin-3-yl, et al.) residues; R₇ represents H, F, Cl, Br, I , CH₃NHCOCH₂CH(NHR₈)CO, R₅N=C(NHR₆)NHCO-, COCH₃, COR₆, PO(OR₅ )OR₆, one of any alkyl, alkyloxyl, alkenyl or alkynyl residues having 1 to 12 carbon atoms, aryl or heteroaryl residues , CO-W, or W; R₈ represents H, F, Cl, Br, I, CH₃, C₂H₅, CF₃, CH₂CH₂F, CH₂CH₂Cl, CH₂CH₂Br, CH₂CH₂I, CH₂CHF₂, CH₂CF₃, CH₂F, CH₂Cl, CH₂Br, CH₂I, C₃H₇, C₄H₉, C₅H₁₁, COR₆, CONH₂, CH₂ OCONH₂, PO(OR₅)OR₆, C(CH₃)₂COOR₆, CH(CH₃)COOR₆, CH₂COOR₆, CO-W, or W; Y₁ represents H, OH, OW, OCOW, OCOCH₃, CH₃, C₂H₅, C₃H₇, C₄H₉, SO_{3 6}, CH₂COOR₈, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR₆, CH(CONH₂)NHR ₆, COOR₅, F, Br, I, or Cl; Y₂ represents H, OH, OW, OCOW, CH₃, C₂H₅, C₃H₇, C ₄H₉, CH₂COOR₈, OCOCH₃, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR₆, CH(CONH₂)NHR ₆, SO₃R₆, COOR₅, F, Br, I, or Cl; Y₃ represents H, OH, OW, OCOW, OCOCH₃, CH₃, C₂H₅, C₃H₇, C₄H₉, CH₂COOR₈, OCH₃, OC₂H₅, CH₃SO₃, NO₂, CN, CF₃,OCF ₃, CH=CHCONHCH₂COOW, CH₂(CH₂)ₙNR₅R₆, CH₂(CH₂)ₙOR₆, CH(CONH₂ )NHR₆, SO₃R₆, COOR₅, F, Br, I, or Cl; Y₄ represents H, OH, OW, OCOW, OCOCH₃, CH₃, C₂H₅, C₃H₇, C₄H₉, CH₂COOR₈, OCH₃, OC₂H₅, CH₃SO₃, NO₂, NR₈ COR₅, SO₂NR₅R₈, COR₅, SR₅, CN, CF₃, OCF₃, CH=CHCONHCH₂COOW, CH₂ (CH₂)ₙNR₅R₆, CH₂(CH₂)ₙOR₆, CH(CONH₂)NHR₆, SO₃R₆, COOR₅, F, Br, I, or Cl; -W represents -H, one of any alkyl, alkyloxy, alkenyl or alkynyl residues having 1 to 12 carbon atoms, aryl or heteroaryl residues, or one of the following residues: 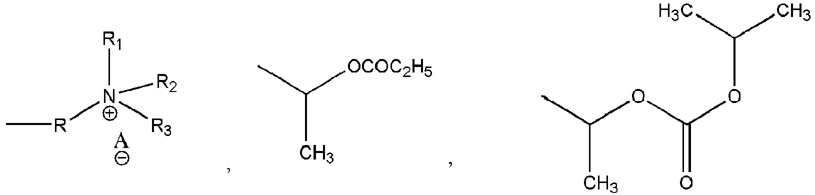 wherein, R represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, or aryl and heteroaryl residues; R represents a branched or straight chain, -(CH₂)ₙ-, aryl residues or heteroaryl residues; n = 0, 1, 2, 3, 4, 5, 6, 7, 8, ...; R and Y taken together are R₆OCH₂C(R₅ )=; A⁻ represents Cl⁻, Br⁻, F⁻, I⁻, AcO⁻, citrate, or any negative ions. All double bond can be cis or trans. All R, R₁, R₂, R₃, R₄, R₅, R₆, R₇, R₈, -(CH₂)ₙ-, or -(CH₂)ₘ - groups are branched or straight chains and can include C, H, O, S, or N atoms and may have single, double, and triple bonds. Any CH₂ groups can be replaced with O, S, or NH.
[2] The design of transdermal delivery systems of beta-lactam antibiotics. The principles for designing the prodrugs of beta-lactam antibiotics are: (1) The prodrug must have a lipophilic portion and a primary, secondary, or tertiary amine group, a quanidino, or monoprotected quanidino group that exists in the protonated form (hydrophilic portion) at physiological pH. (2) Every prodrug of beta-lactam antibiotics should have only one or two (preferably one) primary, secondary, or tertiary amine group, a quanidino, or monoprotected quanidino group that exists in the protonated form (hydrophilic portion) at physiological pH. (3) The primary, secondary, or tertiary amine group, quanidino, or monoprotected quanidino groups can be on any part of the compound. The design in which one end of the compound is the positive charge and other end is the lipophilic portion is preferable. (4) Carboxyl groups, amino groups, guanidine groups or other hydrophilic groups can be protected with an alkyl, aryl, or heteroaryl ester or amide group to make beta-lactam antibiotics more lipophilic.
[3] Processes for the preparation of compounds of the general formula (1) 'Structure 1' according to embodiment 1, wherein the compounds can be prepared from betalactam antibiotics (free acids), by reaction with compounds of the general formula (2) 'Structure 2' by using coupling reagents, such as N,N'-Dicyclohexylcarbodiimide, N, N'-Diisopropylcarbodiimide, 0-(Benzotriazol-1-yl)-N,N,N',N'-tetramethyluronium tetrafluoroborate, 0-(Benzotriazol-1-yl)-N,N,N',N'-tetramethyluronium hexafluorophosphate, Benzotriazol-1-yl-oxy-tris (dimethylamino)phosphonium hexafluorophosphate, et al. 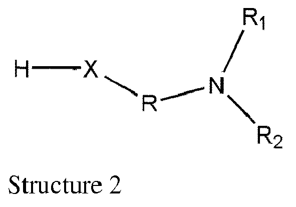 Wherein, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues; R represents a branched or straight chain, -(CH₂)ₙ -,aryl residues or heteroaryl residues; n = 0, 1, 2, 3, 4, 5, 6, 7, 8, .... ; X represents O, S, or NH.
[4] Processes for the preparation of compounds of the general formula (1) 'Structure 1' according to embodiment 1, wherein the compounds can be prepared from metal salts, organic base salts, or immobilized base salts of beta-lactam antibiotics, by reaction with compounds of the general formula (3) 'Structure 3'. 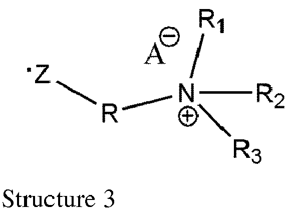 Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; Z represents F, Cl, Br, I, methylsulphonyl or p-toluenesulphonyl; A represents Cf, Br-, F, r, AcU, citrate, or any negative ions; and n=0,1,2,3,4,5 ... ; R represents a branched or straight chain, -(CHF₂)ₙ-, aryl or heteroaryl residues; n =0,1,2,3,4,5,6,7,8,....
[5] A compound or compounds (including penethamate hydriodide) of the general formula (1)'Structure 1' or a composition comprising of at least one compound (including penethamate hydriodide) of the general formula (1) 'Structure 1', as an active ingredient, according to embodiment 1, can be administered orally, transdermally, or by injection for treating any beta-lactam antibiotics-treatable conditions in humans or animals. The beta-lactam antibiotics-treatable conditions include, but are not limited to infections of the upper and lower respiratory tract, acute respiratory distress, pneumonia, meningitis, septic shock, septicemia, otitis media and sinusitis, meningicoccal meningitis and brain abscess, meningococcal and pneumococcal septicemia, streptococcal endocarditis, pelvic inflammatory disease, gonorrhea, syphilis, lyme disease, gas gangrene, tetanus, skin and related soft tissue, gastrointestinal tract, urinary tract, bones and joints, as well as septicemias and endocarditis and intra-abdominal and bile tract infections.
[6] Methods for treating any beta-lactam antibiotics-treatable conditions in humans or animals by administering transdermally to any part of body (in the form of a solution, spray, lotion, ointment, emulsion or gel) to deliver therapeutically effective plasma levels of a compound or compounds (including penethamate hydriodide) of the general formula (1) 'Structure 1' or a composition comprising of at least one compound (including penethamate hydriodide) of the general formula (1) 'Structure 1', as an active ingredient, according to embodiment 1.
[7] Methods for topically treating skin, brain, breast, prostate gland, the upper and lower respiratory tract, urinary tract, bones and joints, genital area, and other infections in humans or animals by administering transdermally to the inflamed area or the nearby area a therapeutically effective amount of a compound or compounds (including penethamate hydriodide) of the general formula (1) 'Structure 1' or a composition comprising at least one compound (including penethamate hydriodide) of the general formula (1) 'Structure 1', as an active ingredient, according to embodiment 1.
[8] Transdermal therapeutic application systems of a compound or compounds (including penethamate hydriodide) of the general formula (1) 'Structure 1' or a composition comprising of at least one compound (including penethamate hydriodide) of the general formula (2) 'Structure 2', as an active ingredient, according to embodiment 1, for treating any beta-lactam antibiotics-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables any beta-lactam antibiotics to reach constantly optimal therapeutic blood levels to increase effectiveness and reduce the side effects of any beta-lactam antibiotics.

## Claims

1. The compounds of the general formula (1) 'Structure 1' except penethamate hydriodide (3,3-dimethyl-7-oxo-6-[(phenylacetyl)amino] - 4-thia-1-azabicyclo[3.2.0]heptane-2-carboxylic acid 2-(diethylamino)ethyl ester monohydriodide) which is a known compound (the high penetration rate through skin, blood-brain, and blood-milk barriers of this compound have never been mentioned). 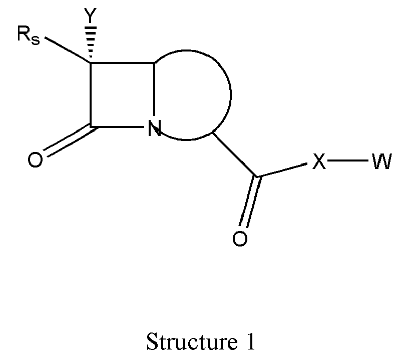 Wherein, X represents O, S, or NH; Y represents H, OH, OR₅, NHCHO, CH₂CH₂ NHC(=NR₇)NHR₇, OCOCH₃, OCH₃, OC₂H₅, CH₃SO₃, NO₂, CN, CF₃, OCF₃, F, Br, I, or Cl; 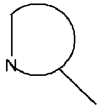 represents 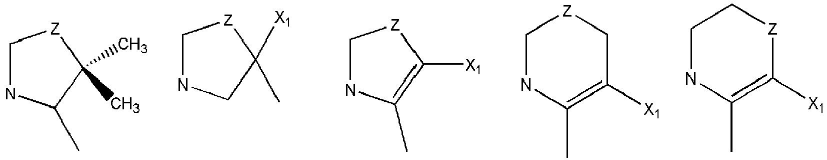 wherein, Z represents S, O, SO, SO₂, NH, CHCH₃, CHCH₂CH₃, or CH₂; -X₁ represents: -H, -OH, -OCH₃, -OC₂H₅, OC₃H₇, -CONH₂, -CH₂OCONH₂, -CH₂ OCOCH_{3,} -OCOCH₃, -CH₂OCOCH₃, -CH₂OCH₃, -CH₃, -C₂H₅, -C₃H₇, -C₄H₉, C₅H ₁₁, C₆H₁₃, -Cl, -F, -Br, -I, -HC=CHCH₃, -HC=CH₂, -CH₂OCH₃, -S(CH₂)ₙ-NHR₇, 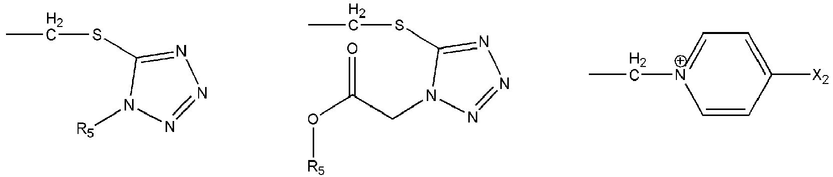 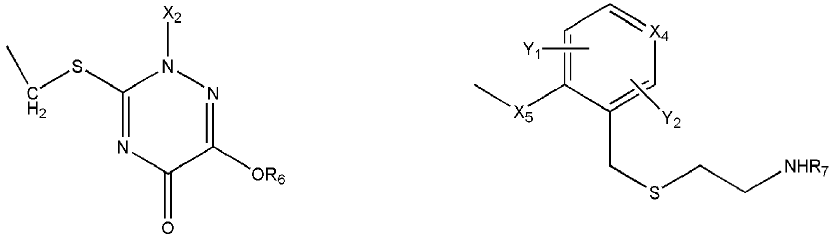 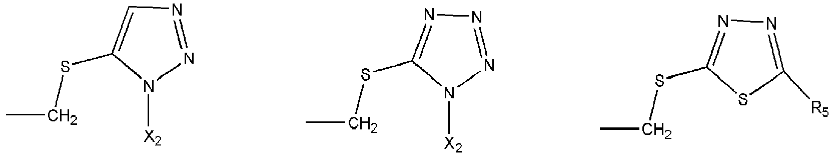 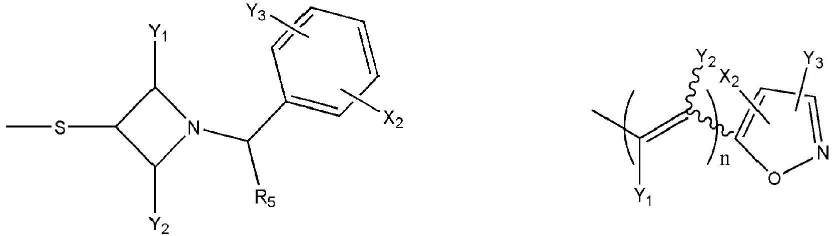 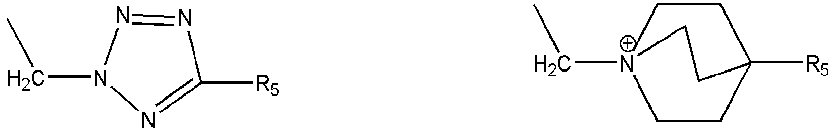 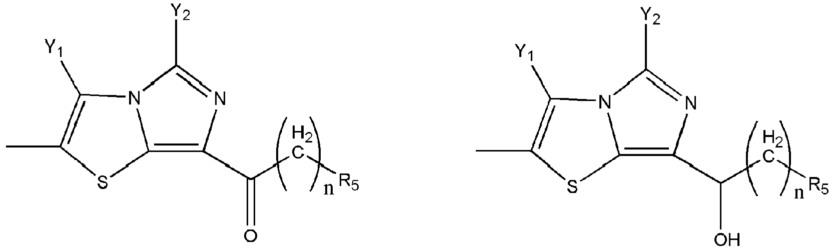 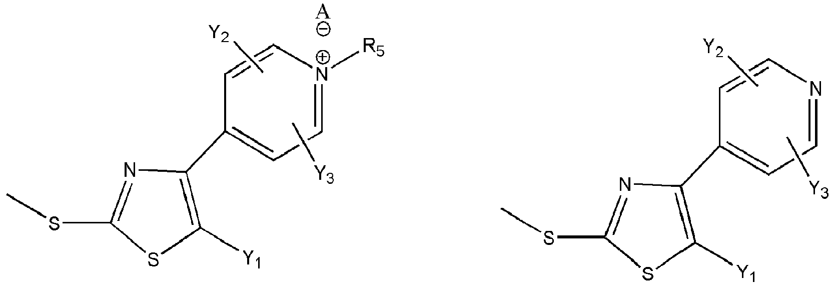 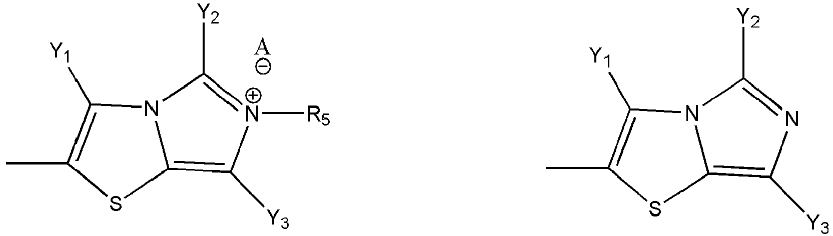 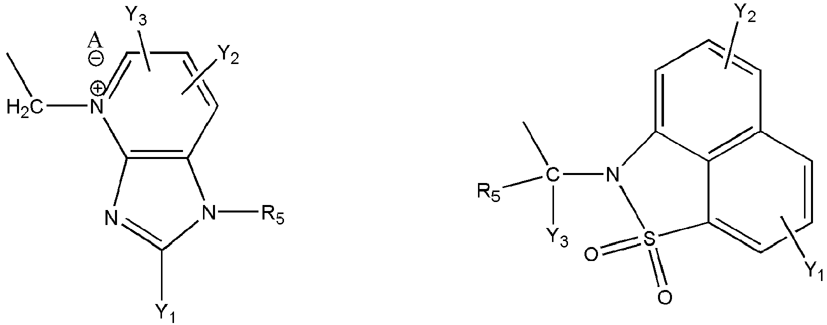 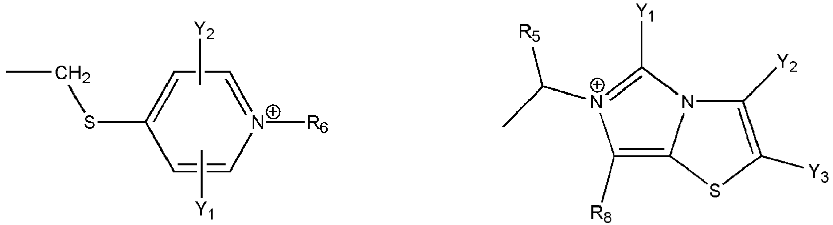 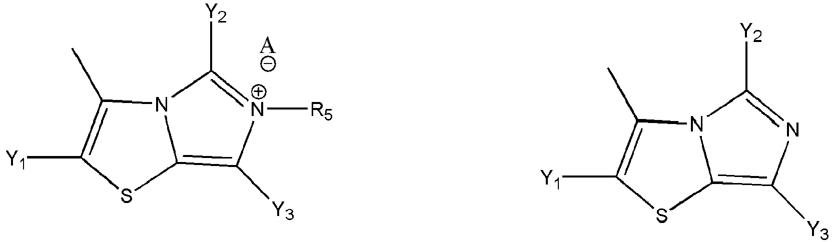 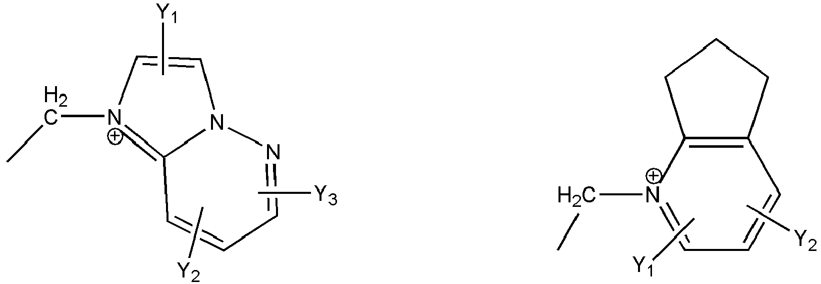 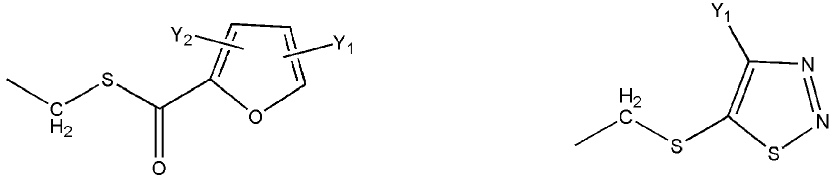 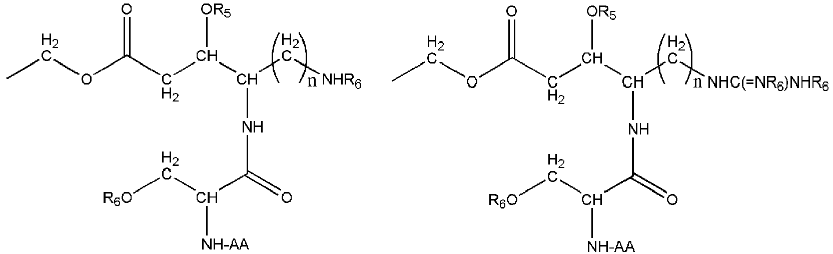 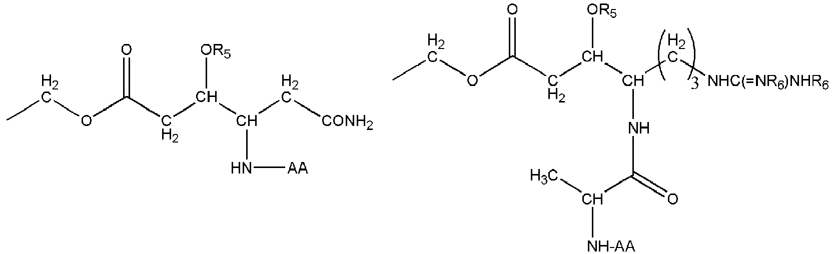 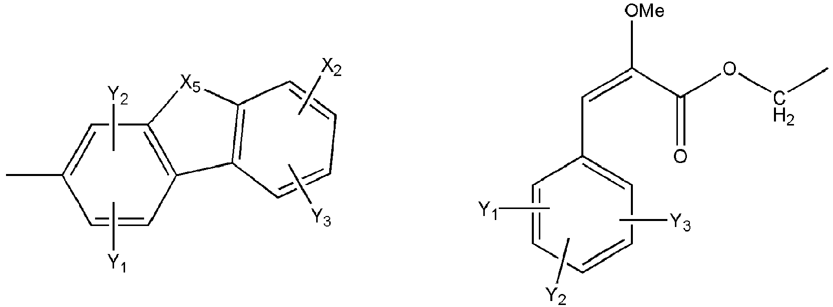 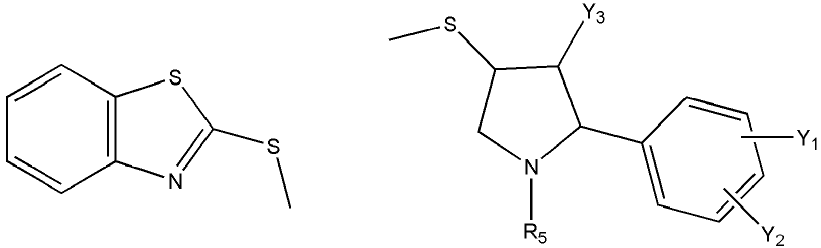 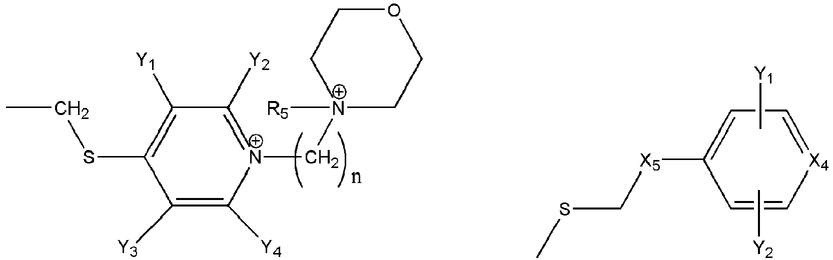 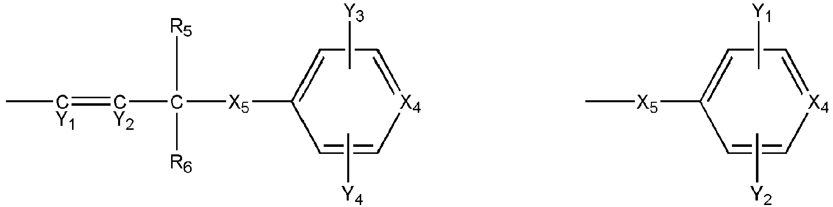 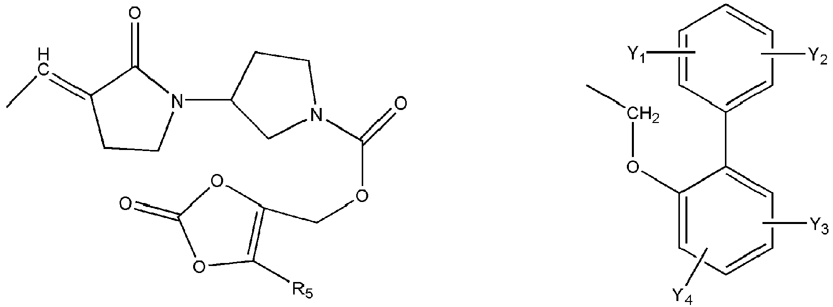 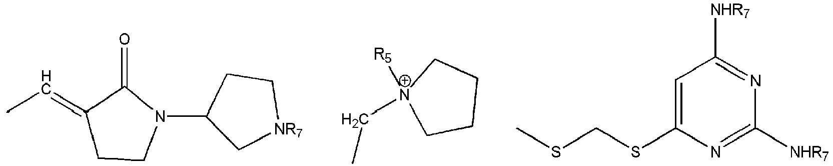 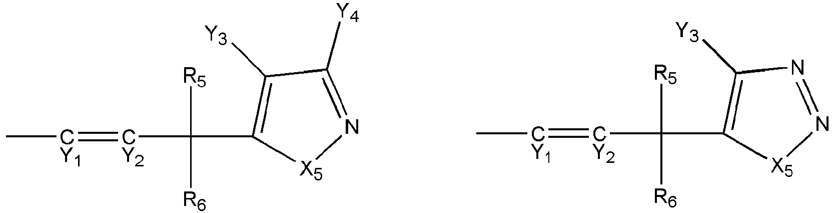 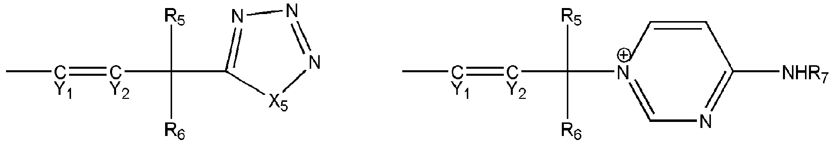 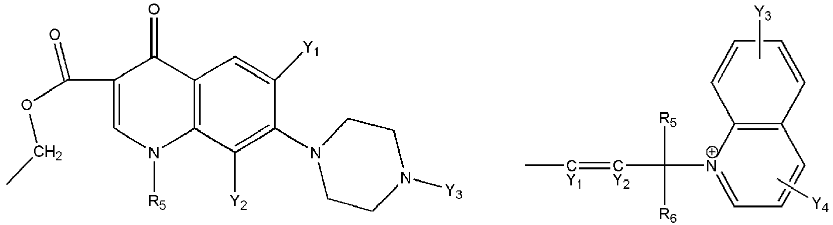 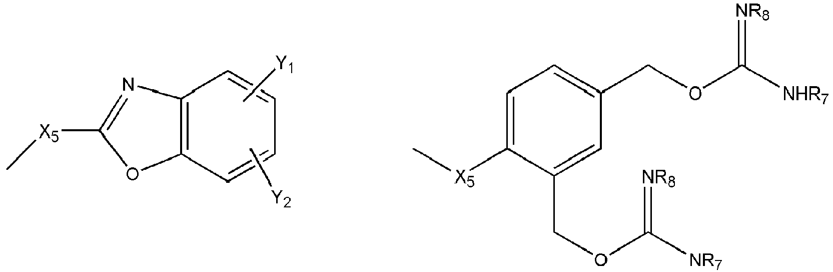 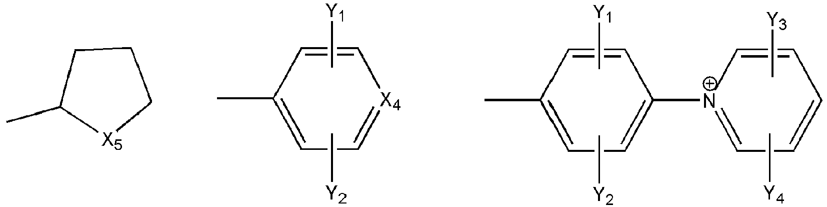 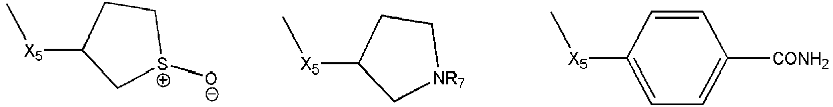 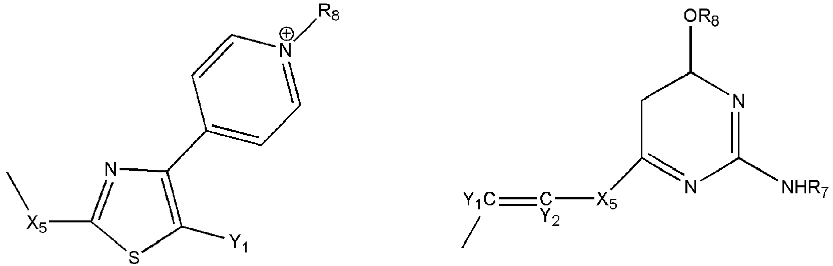 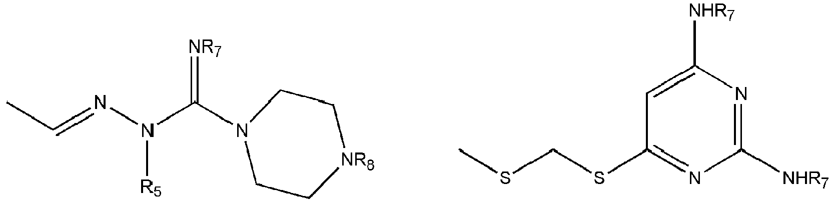 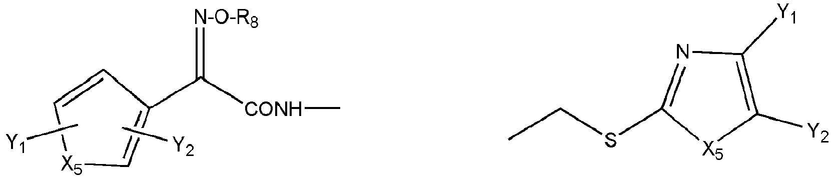 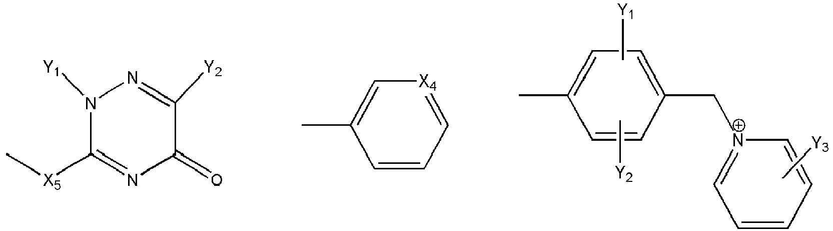 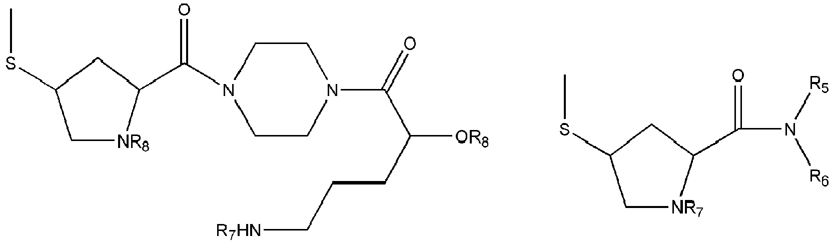 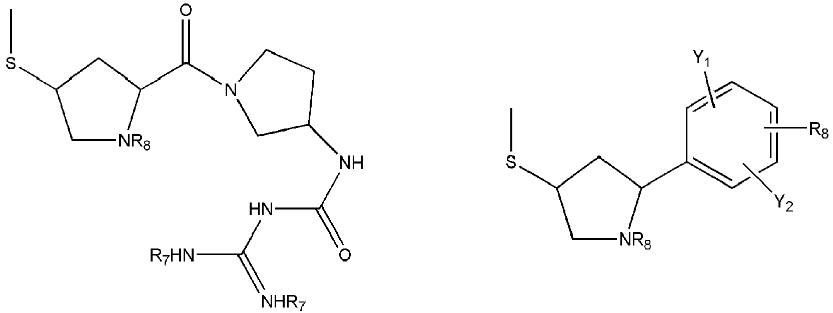 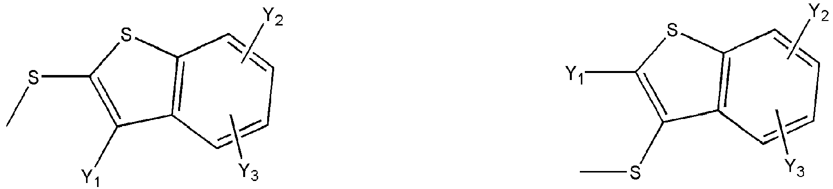 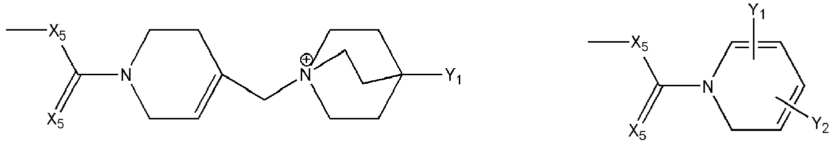 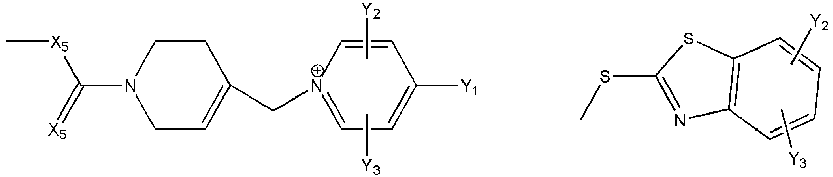 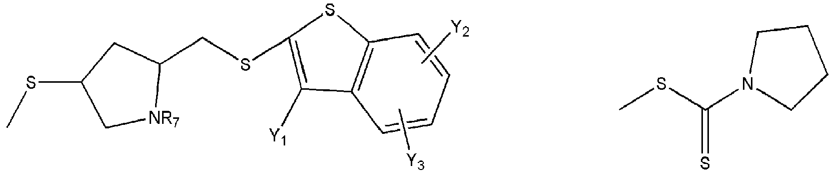 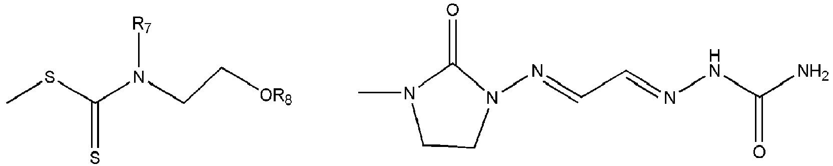 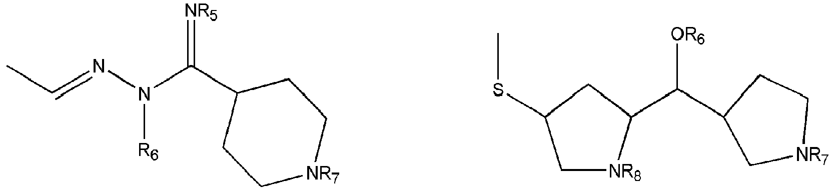 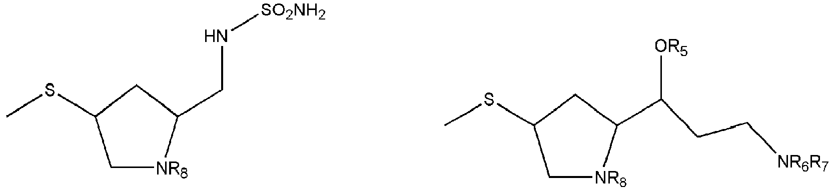 wherein , Z represents S, O, SO, SO₂, NH, CH₂, CHCH₃, or CHCH₂CH₃; R₅ represents H, CONH₂, CH₂CH₂OR₆, CH₂CH₂N(CH₃)₂, CH₂CH₂N(CH₂CH₃)₂, Cl, F, Br, I, one of any alkyl, alkyloxyl, alkenyl or alkynyl residues having 1 to 12 carbon atoms, aryl or heteroaryl residues , CO-W, or W; R₆ represents H, F, Cl, Br, I, Na⁺, K⁺, COR₅, one of any alkyl, alkyloxyl, alkenyl or alkynyl residues having 1 to 12 carbon atoms, aryl or heteroaryl residues , CO-W, or W; R₇ represents H, CH₃, C₂H₅, CF₃, COCH₃, C₃H₇, C₄H₉, C₅H₁₁, CONH₂, COR₆, COR₅, CO-W, or W; R₈ represents H, CH₃, C₂H₅, CF₃, CH₂CH₂F, CH₂CH₂Cl, CH₂CH₂ Br, CH₂CH₂I, CH₂CHF₂, CH₂CF₃, CH₂F CH₂Cl, CH₂Br, CH₂I, CH₂NR₆R₇, CH(NHR₇)CH₂CONH₂, C₃H₇, C₄H₉, C₅H₁₁, CONH₂, CH₂ CONH_{2,} CH₂ OCONH₂, C(CH₃)₂COOR₆, CH(CH₃)COOR₆, CH₂COOR₆, COR₆, COR₅, CO-W, or W; X represents O, S, NH, or CH₂; X₂ represents H, CH₃, CH₂(CH₂)ₙOR₈, Cl, F, Br, I, NO₂, CN, CF₃, OCF₃, NH₂, CH₃, C₂H₅, CONH₂, CH₂CONH₂, CH₂COOR₅, CH₂ (CH)₂)ₙN(CH)₃)₂, CH₂(CH)₂)ₙSO₃R₅, C₁₋₈ alkyl, C₁₋₈ alkylthiol, C₁₋₈ alkylamine, or C alkyloxy; X₂ represents H, CH₃, CH₂(CH₂)ₙ OR₈, Cl, F, Br, I, NO₂, CN, CF₃, OCF₃, NH₂, CH₃, C₂H₅, CONH₂, CH₂OCONH₂, CH₂COOR₅, CH₂(CH₂)ₙN(CH₃)₂, CH₂(CH₂)ₙSO₃R₅, C₁₋₈ alkyl, C₁₋₈ alkylthiol, C₁₋₈ alkylamine, or C₁₋₈ alkyloxy; X₄ represents N, CH , or CY₁ ; X₂ represents CH₂, CHY₁, S, O or NH; Y₁ represents H, OH, OW, OCOW, OCOCH₃, CH₃, C₂H_{5,} C₃H_{7,} C₄H_{9,} CH₂COOR₈, OCH₃, OC₂ H₅, CH₃SO₃, NO₂, CN, CF₃, OCF₃, CH₃(CH₂)ₙNR₅R₆, CH₂(CH₂)ₙOR₆, CH(CONH₂)NHR₆, CH₂CONH₂, F, Br, I, or Cl; Y₂ represents H, OH, OW, OCOW, CH₃, C₂H₅, C₃H₇, C₄H₉, CH₂COOR₈, OCOCH₃, OCH₃, OC₂H₅, CH₃SO₃, NO₂, CN, CF₃, OCF₃, CH₂(CH₂)ₙR₅R₆, CH₂(CH₂)OR₆, CH(CONH₂)NHR₆, CH ₂CONH₂, F, Br, I, or Cl; Y₃ represents H, OH, OW, OCOW, OCOCH₃, CH₃, C₂H ₅, C₃H₇, C₄H₉, CH₂COOR₈, OCH₃, OC₂H₅, CH₃SO₃, NO₂, CN, CF₃, OCF₃, CH₂ (CH₂)ₙNR₅R₆, CH₂(CH₂)ₙOR₆, CH(CONH₂)NHR₆, CH₂CONH₂, F, Br, I, or Cl; Y represents H, OH, OW, OCOW, OCOCH₃, CH₃, C₂H₅, C₃H₇, C₄H₉, CH₂COOR₈, OCH₃, OC₂H₅, CH₃SO₃, NO₂, CN, CF₃, OCF₃, CH₂(CH₂)ₙNR₅R₆, CH₂(CH₂)ₙOR₆, CH(CONH₂)NHR₆, CH₂CONH_{2,} F, Br, I, or Cl; AA represents any amino acids; n=0, 1, 2, 3, 4, 5, 6,...; R₈ - represents: 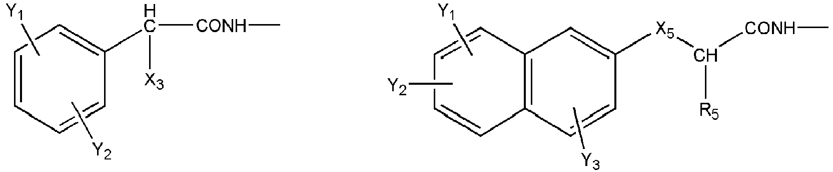 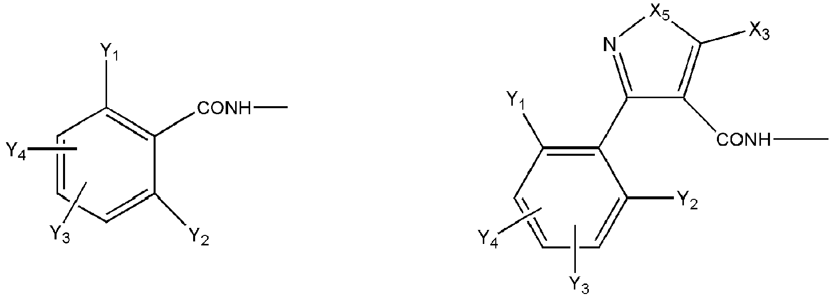 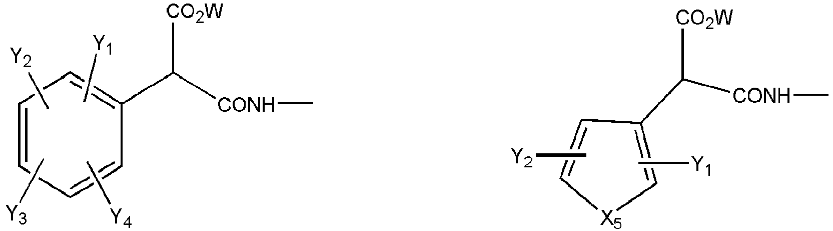 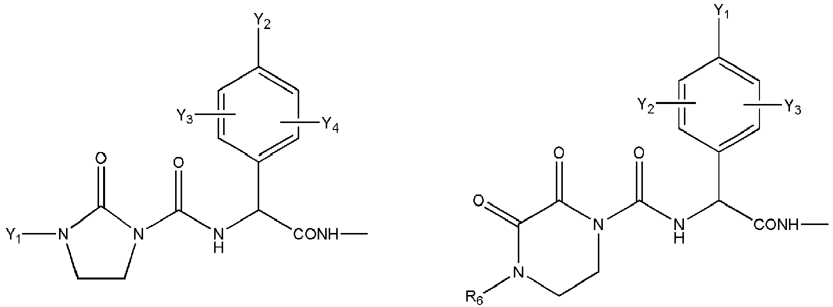 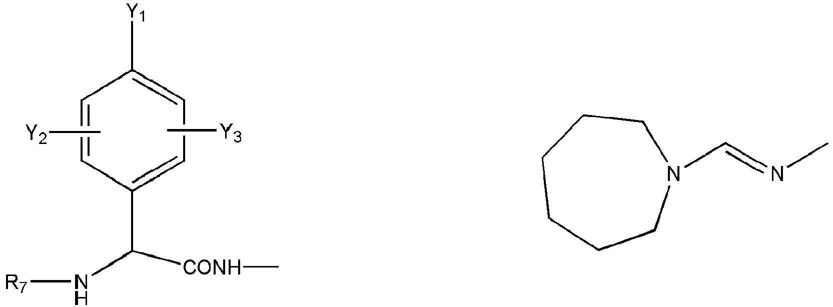 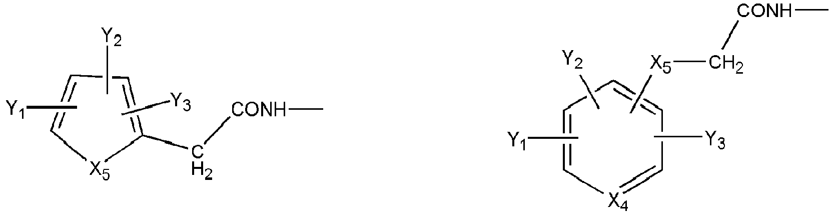 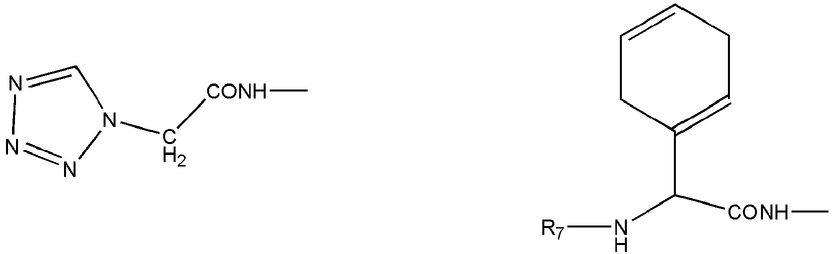 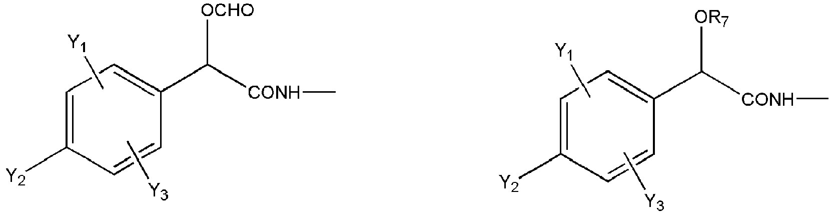 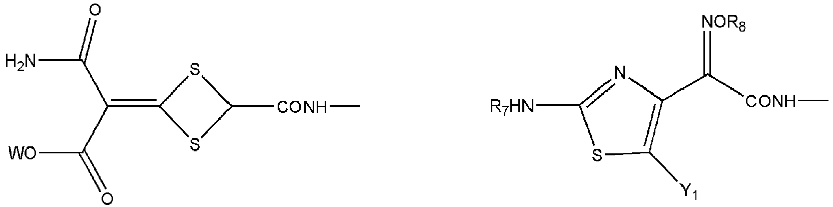 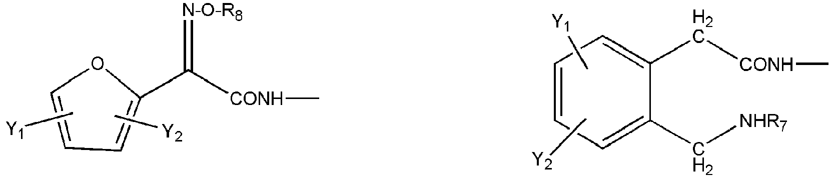 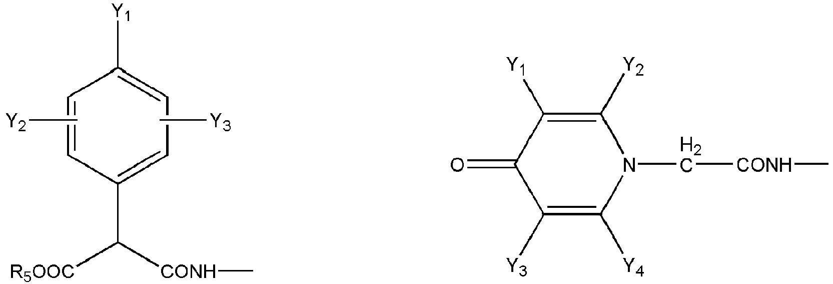 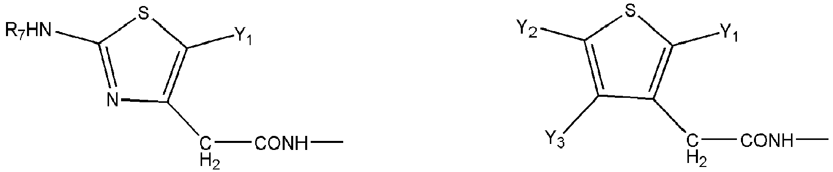 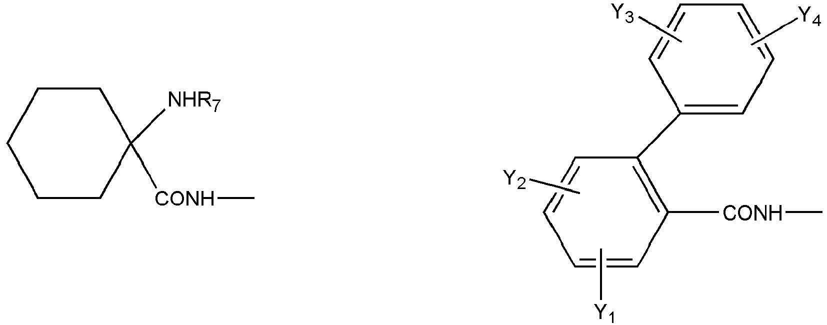 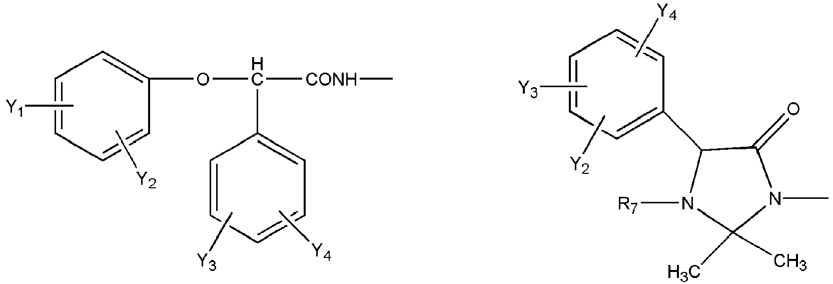 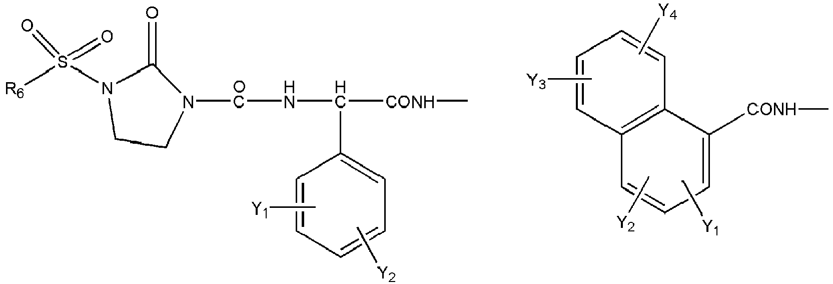 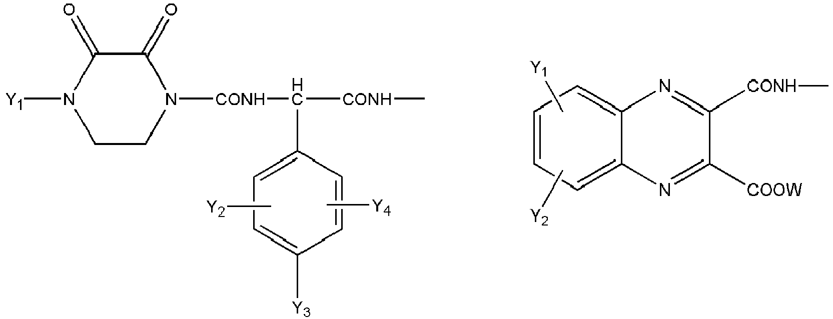 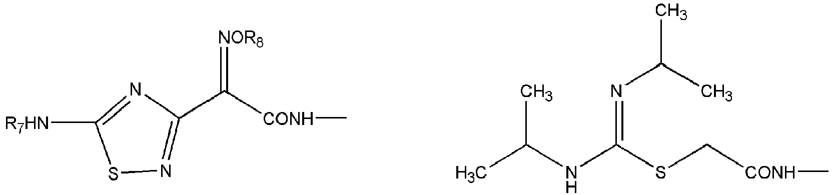 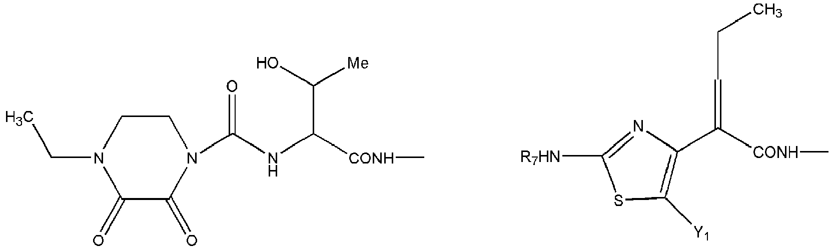 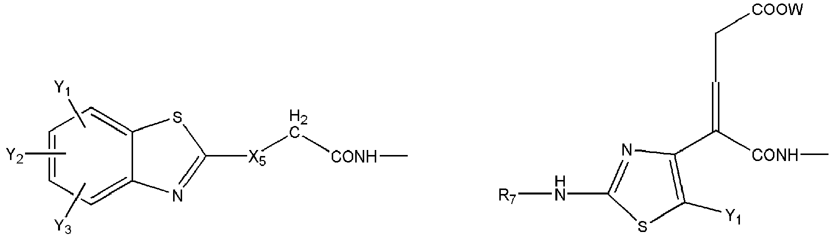 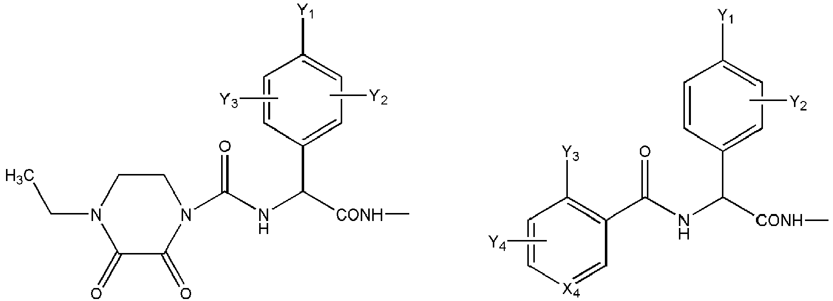 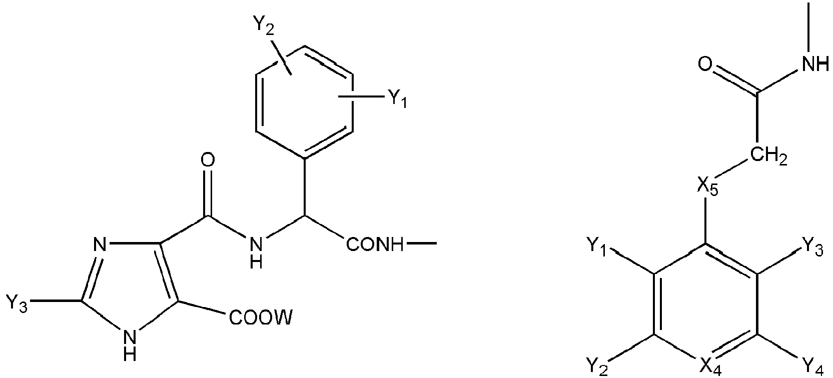 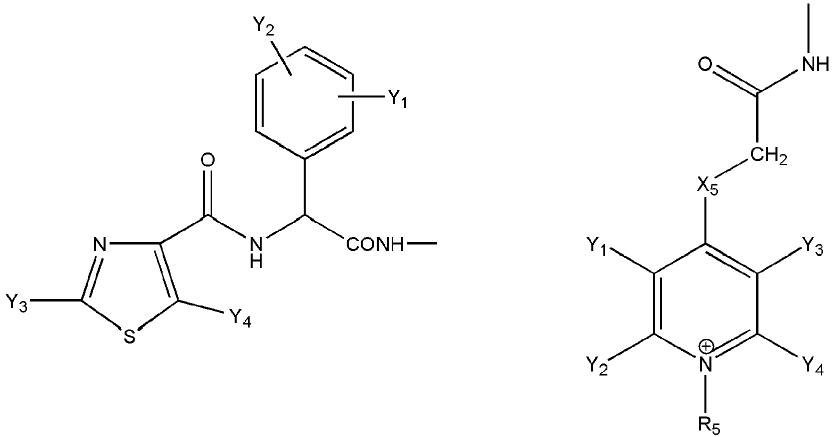 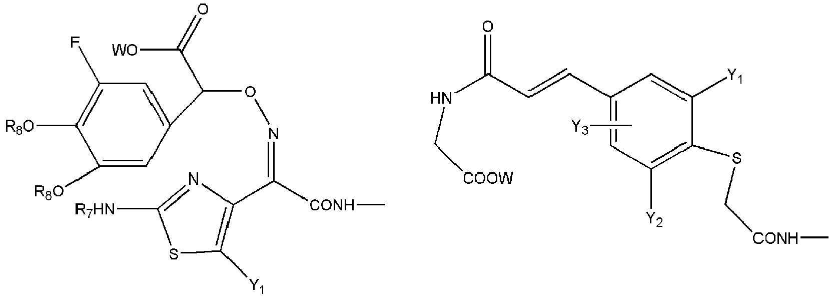 R₆OOCCH(NHR₇)(CH₂)ₙCONH-, R₆OOCCH(NHR₇)(CH₂)ₙSCONH-, CF₃SCH₂ CONH-, CF₃ CH₂ CONH- , CHF₂SCH₂CONH- , CH₂FSCH₂CONH- , NH₂ COCHFSCH₂CONH-, R₇NHCH(COOW)CH₂SCH₂CONH-, CNCH₂SCH₂ CONH-, CH₃(CH₂)ₙCONH-, R₇N=C(NR₇)CH₂CH₂S-, R₇N=C(NHR₇)NHCO-, CH₃C(Cl)=CHCH₂SCH₂CONH-, (CH₃)₂C(OR₆)-, CNCH₂CONH-, CNCH₂CH₂S-, R₇N=C(NR₇)CH₂CH₂S-, CH₂=CHCH₂SCH₂CONH-, CH₃CH(OH)-, CH₃CH(OR₈ )-, CH₃CH(Y₁)-, (CH₃)₂ CH-, CH₃CH₂-, or CH₃(CH₂)ₙCH=CH(CH₂)ₘCONH-. Wherein, n or m= 0, 1, 2, 3, 4, 5, 6, ...; X₃ represents H, N₃, SO₃W, F, Br, I, OH, OCH₃, OC₂H₅, CH₃, C₂H₅, COOW, OW, or W; X₄ represents N, CH , or CY ₁; X₅ represents CH₂, S, O or NH; R₅ represents H, -CONH₂, CH₂CH₂N(CH₃)₂, CH₂CH₂N(CH₂CH₃)₂, CH₂CH₂OR₆, F, Br, I, one of any alkyl, alkyloxyl, alkenyl or alkynyl residues having 1 to 12 carbon atoms, aryl or heteroaryl residue, CO-W, or W; R₆ represents H, F, Br, I, 2-oxo-1-imidazolidinyl, one of any alkyl, alkyloxyl, alkenyl or alkynyl residues having 1 to 12 carbon atoms, aryl (phenyl, 5-indanyl, 2,3-dihydro-1H-inden-5-yl, et al.) or heteroaryl (4-hydroxy-1,5-naphthyridin-3-yl, et al.) residues; R₇ represents H, F, Br, I, CH₃NHCOCH₂CH(NHR₈)CO, R₅N=C(NHR₆)NHCO-, COCH₃, COR₆, PO(OR₅ )OR₆, one of any alkyl, alkyloxyl, alkenyl or alkynyl residues having 1 to 12 carbon atoms, aryl or heteroaryl residues , CO-W, or W; R₈ represents H, F, Br, I, CH₃, C₂H₅, CF₃, CH₂CH₂F, CH₂CH₂Cl, CH₂CH₂Br, CH₂CH₂I, CH₂CHF₂, CH₂CF₃, CH₃F, CH₂Cl, CH₂Br, CH₂I, C₃H₇, C₄H₉, C₅H₁₁, COR₆, CONH₂, CH₂ OCONH₂, PO(OR₅)OR₆, C(CH₃)₂COOR₆, CH(CH₃)COOR₆, CH₂COOR₆, CO-W, or W; Y₁ represents H, OH, OW, OCOW, OCOCH₃, CH₃, C₂H₅, C₃H₇, C₄H₉, SO₃ R₆, CH₂COOR₈, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR₆, CH(CONH₂)NHR 6, COOR₅, F, Br, I, or Cl; Y₂ represents H, OH, OW, OCOW, CH₃, C₂H₅, C₃H₇, C ₄H₉ CH₂COOR₈, OCOCH₃, OCH₃, OC₂H₅, CH₃SO₃, NO₂, CN, CF₃, OCF₃, CH=CHCONHCH₂COOW, CH₂(CH₂)ₙNR₅R₆, CH₂(CH₂)ₙOR₆, CH(CONH₂)NHR ₆, SO₃R₆, COOR₅, F, Br, I, or Cl; Y₃ represents H, OH, OW, OCOW, OCOCH₃, CH₃,C₂H₅, C₃H₇, C₄H₉ CH₂COOR₈, OCH₃, OC₂H₅, CH₃SO₃, NO₂, CN, CF₃, OCF ₃, CH=CHCONHCH₂COOW, CH₂(CH₂)ₙNR₅R₆, CH₂(CH₂)ₙOR₆, CH(CONH₂ )NHR₆, SO₃R₆, COOR₅, F, Br, I, or Cl; Y represents H, OH, OW, OCOW, OCOCH₃, CH₃, C₂H₅, C₃H₇, C₄H₉, CH₂COOR₈, OCH₃, OC₂H₅, CH₃SO₃, NO₂, NR₈ COR₅, SO₂NR₅R₈, COR₅, SR₅, CN, CF₃, OCF₃, CH=CHCONHCH₂COOW, CH₂ (CH₂)ₙNR₅R₆, CH₂(CH₂)ₙOR₆, CH(CONH₂)NHR₆, SO₃R₆, COOR₅, F, Br, I, or Cl; -W represents -H, one of any alkyl, alkyloxy, alkenyl or alkynyl residues having 1 to 12 carbon atoms, aryl or heteroaryl residues, or one of the following residues: 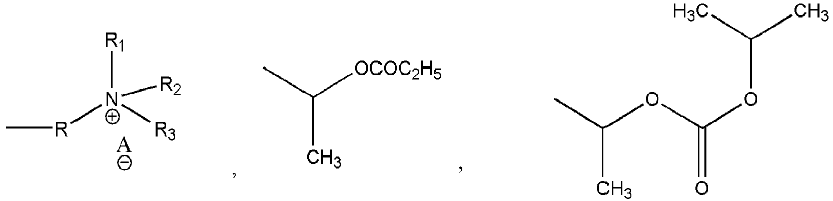 wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, or aryl and heteroaryl residues; R represents a branched or straight chain, -(CH₂)ₙ-, aryl residues or heteroaryl residues; n = 0, 1, 2, 3, 4, 5, 6, 7, 8, ...; Rₛ and Y taken together are R₆OCH₂C(R₅ )=; A⁻ represents Cl⁻, Br⁻, F⁻, I⁻, AcO⁻, citrate, or any negative ions. All double bond can be cis or trans. All R, R₁, R₂, R₃, R₄, R₅, R₆, R₇, R₈, -(CH₂)ₙ-, or -(CH₂)ₘ - groups are branched or straight chains and can include C, H, O, S, or N atoms and may have single, double, and triple bonds. Any CH₂ groups can be replaced with O, S, or NH.

2. The design of transdermal delivery systems of beta-lactam antibiotics. The principles for designing the prodrugs of beta-lactam antibiotics are: (1) The prodrug must have a lipophilic portion and a primary, secondary, or tertiary amine group, a quanidino, or monoprotected quanidino group that exists in the protonated form (hydrophilic portion) at physiological pH. (2) Every prodrug of beta-lactam antibiotics should have only one or two (preferably one) primary, secondary, or tertiary amine group, a quanidino, or monoprotected quanidino group that exists in the protonated form (hydrophilic portion) at physiological pH. (3) The primary, secondary, or tertiary amine group, quanidino, or monoprotected quanidino groups can be on any part of the compound. The design in which one end of the compound is the positive charge and other end is the lipophilic portion is preferable. (4) Carboxyl groups, amino groups, guanidine groups or other hydrophilic groups can be protected with an alkyl, aryl, or heteroaryl ester or amide group to make beta-lactam antibiotics more lipophilic.

3. Processes for the preparation of compounds of the general formula (1) 'Structure 1' according to Claim 1, wherein the compounds can be prepared from betalactam antibiotics (free acids), by reaction with compounds of the general formula (2) 'Structure 2' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al. 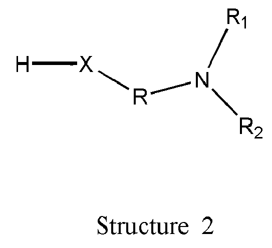 Wherein, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues
having 1 to 12 carbon atoms, or aryl residues; R represents a branched or straight chain, -(CH₂)ₙ-, aryl residues or heteroaryl residues; n = 0, 1, 2, 3, 4, 5, 6, 7, 8, ....; X represents O, S, or NH.

4. Processes for the preparation of compounds of the general formula (1) 'Structure 1' according to Claim 1, wherein the compounds can be prepared from metal salts, organic base salts, or immobilized base salts of beta-lactam antibiotics, by reaction with compounds of the general formula (3) 'Structure 3'. 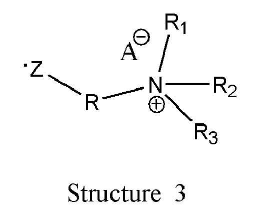 Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; Z represents F, Cl, Br, I, methylsulphonyl or p-toluenesulphonyl; A⁻ represents Cl⁻, Br⁻, F⁻, I⁻, AcO⁻, citrate, or any negative ions; and n=0,1,2,3,4,5...; R represents a branched or straight chain, -(CH₂)ₙ-, aryl or heteroaryl residues; n = 0, 1, 2, 3, 4, 5, 6, 7, 8, ....

5. A compound or compounds (including penethamate hydriodide) of the general formula (1)'Structure 1' or a composition comprising of at least one compound (including penethamate hydriodide) of the general formula (1) 'Structure 1', as an active ingredient, according to Claim 1, can be administered orally, transdermally, or by injection for treating any beta-lactam antibiotics-treatable conditions in humans or animals. The beta-lactam antibiotics-treatable conditions include, but are not limited to infections of the upper and lower respiratory tract, acute respiratory distress, pneumonia, meningitis, septic shock, septicemia, otitis media and sinusitis, meningicoccal meningitis and brain abscess, meningococcal and pneumococcal septicemia, streptococcal endocarditis, pelvic inflammatory disease, gonorrhea, syphilis, lyme disease, gas gangrene, tetanus, skin and related soft tissue, gastrointestinal tract, urinary tract, bones and joints, as well as septicemias and endocarditis and intra-abdominal and bile tract infections.

6. Methods for treating any beta-lactam antibiotics-treatable conditions in humans or animals by administering transdermally to any part of body (in the form of a solution, spray, lotion, ointment, emulsion or gel) to deliver therapeutically effective plasma levels of a compound or compounds (including penethamate hydriodide) of the general formula (1) 'Structure 1' or a composition comprising of at least one compound (including penethamate hydriodide) of the general formula (1) 'Structure 1', as an active ingredient, according to Claim 1.

7. Methods for topically treating skin, brain, breast, prostate gland, the upper and lower respiratory tract, urinary tract, bones and joints, genital area, and other infections in humans or animals by administering transdermally to the inflamed area or the nearby area a therapeutically effective amount of a compound or compounds (including penethamate hydriodide) of the general formula (1) 'Structure 1' or a composition comprising at least one compound (including penethamate hydriodide) of the general formula (1) 'Structure 1', as an active ingredient, according to Claim 1.

8. Transdermal therapeutic application systems of a compound or compounds (including penethamate hydriodide) of the general formula (1) 'Structure 1' or a composition comprising of at least one compound (including penethamate hydriodide) of the general formula (2) 'Structure 2', as an active ingredient, according to Claim 1, for treating any beta-lactam antibiotics-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables any beta-lactam antibiotics to reach constantly optimal therapeutic blood levels to increase effectiveness and reduce the side effects of any beta-lactam antibiotics.
